(19) **Europäisches Patentamt**
**European Patent Office**
**Office européen des brevets**

(11) **EP 4 206 321 A1**

(12) **EUROPEAN PATENT APPLICATION**
published in accordance with Art. 153(4) EPC

(43) Date of publication:
**05.07.2023 Bulletin 2023/27**

(21) Application number: **21866882.0**

(22) Date of filing: **10.09.2021**

(51) International Patent Classification (IPC):
*C12N 5/0793* (2010.01)    *C12N 5/0797* (2010.01)
*A61L 27/22* (2006.01)    *A61L 27/36* (2006.01)
*A61L 27/38* (2006.01)    *A61L 27/44* (2006.01)
*A61L 27/52* (2006.01)    *A61L 27/58* (2006.01)

(52) Cooperative Patent Classification (CPC):
**A61L 27/22; A61L 27/36; A61L 27/38; A61L 27/44;**
**A61L 27/52; A61L 27/58; C12N 5/06**

(86) International application number:
**PCT/JP2021/033387**

(87) International publication number:
**WO 2022/054925 (17.03.2022 Gazette 2022/11)**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority: **11.09.2020  JP 2020153257**

(71) Applicants:
• **RIKEN**
  **Wako-shi, Saitama 351-0198 (JP)**

• **Sumitomo Pharma Co., Ltd.**
  **Osaka 541-0045 (JP)**

(72) Inventors:
• **MANDAI, Michiko**
  **Wako-shi, Saitama 351-0198 (JP)**
• **TAKAHASHI, Masayo**
  **Wako-shi, Saitama 351-0198 (JP)**
• **YAMASAKI, Suguru**
  **Kobe-shi, Hyogo 650-0047 (JP)**

(74) Representative: **Hoffmann Eitle**
**Patent- und Rechtsanwälte PartmbB**
**Arabellastraße 30**
**81925 München (DE)**

(54) **COMPLEX CONTAINING NEURAL RETINA-CONTAINING CELL AGGREGATES AND MATRIX, AND METHOD FOR MANUFACTURING SAME**

(57)    An object of the present invention is to provide a complex containing two or more neural retina-containing cell aggregates, and a method for manufacturing the same. The complex of the present invention is a complex comprising: two or more cell aggregates each containing a neural retina derived from a pluripotent stem cell; and a matrix, the two or more cell aggregates being arranged in the matrix. The method for manufacturing the complex of the present invention is a method for manufacturing a complex in which two or more neural retina-containing cell aggregates are arranged in a matrix, comprising: (1) a first step of preparing two or more cell aggregates of neural retinas from a pluripotent stem cell; and (2) a second step of contacting the two or more cell aggregates in a predetermined arrangement with the matrix or a precursor of the matrix, followed by the gelation of the matrix.

*Fig.1*

## Description

### Technical Field

The present invention relates to a complex containing neural retina-containing cell aggregates and a matrix, and a method for manufacturing the same.

### Background Art

Research on methods for producing neural retinas from pluripotent stem cells has been actively made in relation to transplantation therapies with retinas for diseases caused by the damage of retinal tissue such as retinitis pigmentosa. For example, methods of obtaining a neural retina by suspension-culturing an aggregate of pluripotent stem cells in a medium containing a BMP signaling pathway agonist (Patent Literatures 1 and 2 and Non Patent Literature 1) are known as the methods for producing neural retinas from pluripotent stem cells.

Meanwhile, techniques using gelatin have been studied so far for transplantation. Examples thereof include a method of solidifying a retinal tissue *in vivo* in gelatin in order to improve the ease of dissection in dissecting a photoreceptor layer (Non Patent Literature 2), a method of embedding a retinal tissue *in vivo* in temperature-sensitive gelatin (Non Patent Literature 3), and a method of embedding human fetus-derived retinal tissue in gelatin in order to protect fragile fetal tissue (Non Patent Literature 4).

Fibrin gel is administered to humans for the purpose of tissue repair by the adhesion and sealing of tissue. One example thereof includes Bolheal(R) for tissue adhesion. Also, a laminate of fibrin gel and sheet-shaped cell cultures is disclosed in which the fibrin gel is used for enhancing the strength of transplant tissue (Patent Literature 3). Furthermore, a report on the transplantation of retinal progenitor cells embedded in fibrin gel has been made (Non Patent Literature 5).

However, use of a matrix such as gelatin or fibrin gel for the purpose of adhesion of two or more neural retinas has not yet been known.

### Citation List

### Patent Literature

Patent Literature 1: WO2015/025967
Patent Literature 2: WO2016/063986
Patent Literature 3: Japanese Unexamined Patent Publication No. 2016-52271

### Non Patent Literature

Non Patent Literature 1: Atsushi Kuwahara et al., "Generation of a ciliary margin-like stem cell niche from self-organizing human retinal tissue", Nature Communications, 6, Article number: 6286 (2015)
Non Patent Literature 2: Taylor et al., IOVS 1989 Aug; Volume 30, Issue 8
Non Patent Literature 3: M' Barek et al., Sci. Transl. Med. 2017 Dec; Volume 9, Issue 421
Non Patent Literature 4: Seiler et al., Prog Retin Eye Res. 2012 Nov; 31 (6): 661-687
Non Patent Literature 5: Tamer A.E. Ahmed et al., Frontiersin Bioengineering and Biotechnology, Biomaterials, February 2015, Volume 2, Article 85, 1-11

### Summary of Invention

### Technical Problem

Methods of conveniently causing adhesion of a plurality of tissues have been desired as methods for formulating transplant tissue for application to regenerative medicine. Accordingly, an object of the present invention is to provide a complex containing two or more neural retina-containing cell aggregates.

**Solution to Problem**

The present inventors have conducted studies on a method of forming a complex by causing adhesion or embedding of two or more neural retina-containing cell aggregates using some "glue". The "glue", which is foreign matter, may hinder the post-transplant engraftment of the complex and become foreign matter *in vivo*. Hence, the present inventors have thought that a matrix which is a substance that adheres strongly in a small amount and is degraded *in vivo* is preferable as the "glue".

As a result of conducting diligent studies, the present inventors have successfully produced a complex of two or more neural retina-containing cell aggregates and a matrix by embedding the two or more neural retina-containing cell aggregates using fibrin gel or gelatin as a "glue". In this method, two or more neural retina-containing cell aggregates are embedded in a matrix and thereby permit suction into and ejection from an injector without disrupting the matrix gel.

Specifically, the present invention relates to each of the following aspects.

[1] A complex comprising:

two or more cell aggregates each containing a neural retina derived from a pluripotent stem cell; and
a matrix,
the two or more cell aggregates being arranged in the matrix.

[2] The complex according to [1], wherein the neural retina is a transplant neural retina sheet having the following features (1) to (10):

(1) being derived from a pluripotent stem cell,
(2) having a three-dimensional structure,
(3) comprising a neural retinal layer having a plurality of layer structures including a photoreceptor layer and an inner layer,
(4) the photoreceptor layer comprising one or more cells selected from the group consisting of a photoreceptor progenitor cell and a photoreceptor cell,
(5) the inner layer comprising one or more cells selected from the group consisting of a retinal progenitor cell, a ganglion cell, an amacrine cell, a bipolar cell, a horizontal cell, and a Muller glial cell,
(6) the surface of the neural retinal layer having an apical surface,
(7) the inner layer being present inside the photoreceptor layer present along the apical surface,
(8) the area of the neural retinal layer being 50% or more with respect to the total area of the surface of the transplant neural retina sheet,
(9) the area of a continuous epithelium structure being 80% or more with respect to the total area of the apical surface of the neural retinal layer, and
(10) the expression of neural retina-related cell-related gene being found and the expression of non-neural retina-related cell-related gene being not found in the transplant neural retina sheet, wherein the non-neural retina-related cell-related gene comprising one or more genes selected from the group consisting of brain and spinal cord tissue marker gene and eyeball-related tissue marker gene.

[3] The complex according to [1] or [2], wherein the cell aggregates have a major axis of 600 $\mu$m to 2500 $\mu$m, and the two or more cell aggregates are arranged in a row in the matrix.
[4] The complex according to any of [1] to [3], wherein the matrix is fibrin gel or gelatin.
[5] A method for manufacturing a complex in which two or more neural retina-containing cell aggregates are arranged in a matrix, comprising:

(1) a first step of preparing two or more cell aggregates of neural retinas from pluripotent stem cells; and
(2) a second step of contacting the two or more cell aggregates in a predetermined arrangement with the matrix or a precursor of the matrix, followed by the gelation of the matrix.

[6] The manufacturing method according to [5], comprising, in the second step, arranging 2 to 20 cell aggregates in a row.
[7] The manufacturing method according to [5] or [6], wherein the matrix is fibrin gel, and in the second step, the gelation is performed by arranging the two or more cell aggregates in a row, and contacting the two or more cell aggregates arranged with any one of a fibrinogen solution and a thrombin solution and further with the other solution of the fibrinogen solution and the thrombin solution so that the fibrinogen and the thrombin are reacted.
[8] The manufacturing method according to [5] or [6], wherein the matrix is gelatin.

[9] A pharmaceutical composition comprising the complex according to any of [1] to [3].

[10] A method for treating a disease caused by the damage of a retinal cell or retinal tissue or the injury of retinal tissue, comprising transplanting the complex according to any of [1] to [3] to a subject in need of transplantation.

**Advantageous Effects of Invention**

According to the present invention, a complex comprising two or more neural retina-containing cell aggregates and a matrix can be provided, and the simultaneous transplantation of a plurality of neural retina (NR)-containing cell aggregates can be easily carried out.

**Brief Description of Drawings**

[Figure 1] Figure 1 is bright field stereo microscope images, fluorescence stereo microscope images and bright field + fluorescence stereo microscope images in which the process of encapsulating a plurality of (8 to 13) transplant neural retinas (Caps) in fibrin gel (complex) and aspiration and discharge using Surflo were studied in Example 1.

[Figure 2] Figure 2 is bright field stereo microscope images, fluorescence stereo microscope images and bright field + fluorescence stereo microscope images in which the process of encapsulating a plurality of (8 to 10) transplant neural retinas (Caps) in gelatin (complex) and aspiration and discharge using a 1 ml syringe with a catheter tip were studied in Example 2.

[Figure 3] Figure 3 is fluorescence microscope images showing results of performing immunostaining on cell aggregates containing a transplant neural retina with Crx and Chx10 in Reference Example 1.

[Figure 4] Figure 4 is fluorescence microscope images showing results of performing immunostaining on cell aggregates containing a transplant neural retina with Rx and Recoverin in Reference Example 1.

[Figure 5] Figure 5 is a conceptual view of preparing a Cap and a Ring from a typical cell aggregate.

[Figure 6] Figure 6 is a conceptual view of preparing a Cap and a Ring from cell aggregates having various shapes. Portions indicated in black color and gray color mean non-target tissue.

[Figure 7] Figure 7 shows images of typical grafts and a schematic view of a graft as well as the heights, major axes and minor axes of grafts in Reference Example 3.

[Figure 8] Figure 8 is confocal fluorescence microscope images showing results of performing immunostaining on grafts with Crx, Chx10, Rx and Recoverin in Reference Example 4.

[Figure 9A] Figure 9A shows results of analyzing gene expression for RNA extracted from a Cap and a Ring by quantitative PCR in Reference Example 5.

[Figure 9B] Figure 9B shows results of analyzing gene expression for RNA extracted from a Cap and a Ring by quantitative PCR in Reference Example 5.

[Figure 10] Figure 10 is images showing results of analyzing RNA extracted from a Ring by quantitative PCR, then subretinally transplanting a graft (cap) to a rat, and observing an image of post-transplant engraftment under a fluorescence microscope in Reference Example 6.

[Figure 11] Figure 11 is fluorescence microscope images showing results of performing immunostaining on a Cap and a Ring prepared from one cell aggregate in Reference Example 7.

[Figure 12] Figure 12 is fluorescence microscope images showing results of performing immunostaining on a cap prepared from one cell aggregate in Reference Example 8.

**Description of Embodiments**

[Definition]

The "stem cells" refer to undifferentiated cells having differentiation potency and proliferation potency (particularly, self-renewal ability). In the stem cells, subgroups of pluripotent stem cells, multipotent stem cells and unipotent stem cells, are included according to the differentiation potency. The pluripotent stem cells refer to stem cells that can be cultured *in vitro* and has an ability (pluripotency) to be able to differentiate into three germ layers (ectoderm, mesoderm, endoderm) and/or all cell lineages belonging to the extraembryonic tissue. The multipotent stem cells refer to stem cells having an ability to differentiate into a plurality of tissues or cells, although the definition is not applied to all of them. The unipotent stem cells refer to stem cells having an ability to be able to differentiate into a predetermined tissue or cells.

The "pluripotent stem cells" can be induced from, e.g., a fertilized egg, a cloned embryo, germline stem cells, tissue stem cells and somatic cells. Examples of the pluripotent stem cells can include embryonic stem cells (ES cells), embryonic germ cells (EG cells) and induced pluripotent stem cells (iPS cells). Muse cells (Multi-lineage differentiating stress enduring cells) obtained from the mesenchymal stem cells (MSC) and GS cells prepared from germ cells (for example, testis) are included in the pluripotent stem cells.

Human embryonic stem cells were established in 1998 and have been used also for regenerative medicine. The embryonic stem cells can be produced by culturing inner cell aggregate on feeder cells or a culture medium containing bFGF. The method for producing embryonic stem cells is described, for example, in WO96/22362, WO02/101057, US5,843,780, US6,200,806, US6,280,718. The embryonic stem cells are available from a predetermined institution and also, commercially available. For example, human embryonic stem cells such as KhES-1, KhES-2 and KhES-3 are available from the Institute for Frontier Life and Medical Sciences, Kyoto University. Human embryonic stem cells such as human ES cells genetically engineered so as to have Rx:: Venus, Rx::AcGFP and Crx:: Venus reporter genes (derived from KhES-1, Non Patent Literature 1) are available from RIKEN.

The "induced pluripotent stem cells" refers to cells having pluripotency, which is induced by reprogramming somatic cells by a method known in the art.

The induced pluripotent stem cells were established in mouse cells by Yamanaka et al., in 2006 (Cell, 2006, 126 (4), pp. 663-676). The induced pluripotent stem cells were also established in human fibroblasts in 2007. The induced pluripotent stem cells have pluripotency and self-renewal ability similarly to embryonic stem cells (Cell, 2007, 131 (5), pp. 861-872; Science, 2007, 318 (5858), pp. 1917-1920; Nat. Biotechnol., 2008, 26 (1), pp. 101-106).

The induced pluripotent stem cells more specifically refer to cells which are induced to be pluripotent by reprogramming somatic cells differentiated into, for example, fibroblasts and peripheral blood mononuclear cells, by allowing any one of sets of a plurality of genes selected from a reprogramming gene group containing Oct3/4, Sox2, Klf4, Myc (c-Myc, N-Myc, L-Myc), Glis1, Nanog, Sall4, lin28 and Esrrb to express. Examples of a preferable set of reprogramming factors may include (1) Oct3/4, Sox2, Klf4, and Myc (c-Myc or L-Myc) and (2) Oct3/4, Sox2, Klf4, Lin28 and L-Myc (Stem Cells, 2013; 31: 458-466).

Other than producing induced pluripotent stem cells through direct reprogramming by gene expression, the pluripotent stem cells can be artificially induced from somatic cells, for example, by adding a chemical compound (Science, 2013, 341, pp. 651-654).

Alternatively, an induced pluripotent stem cell strain is available. For example, human induced pluripotent cell strains established by Kyoto University, such as 201B7 cell, 201B7-Ff cell, 253G1 cell, 253G4 cell, 1201C1 cell, 1205D1 cell, 1210B2 cell and 1231A3 cell, are available form Kyoto University and iPS Academia Japan, Inc. As the induced pluripotent stem cells, for example, Ff-I01 cell, Ff-I14 cell and QHJI01s04 cell established by Kyoto University, are available from Kyoto University.

In the specification, the pluripotent stem cells are preferably embryonic stem cells or induced pluripotent stem cells, more preferably induced pluripotent stem cells.

In the specification, the pluripotent stem cells are human pluripotent stem cells, preferably human induced pluripotent stem cells (iPS cells) or human embryonic stem cells (ES cells) (e.g., embryonic stem cell established from the embryo within 14 days after fertilization).

Pluripotent stem cells such as human iPS cells can be subjected to maintenance culture and expansion culture performed by methods known to those skilled in the art.

The "retinal tissue" means a tissue in which a single type or a plurality of types of retinal cells constituting each retinal layer in a retina *in vivo* are present according to a predetermined order. The "neural retina" is a retinal tissue and means a tissue containing an inside neural retinal layer that does not contain a retinal pigment epithelial layer among retinal layers mentioned later.

The "retinal cells" mean cells constituting each retinal layer in a retina *in vivo* or progenitor cells thereof. In the retinal cells, cells such as photoreceptor cells (rod photoreceptor cell, cone photoreceptor cell), horizontal cells, amacrine cells, intermediate neuronal cells, retinal ganglion cells (ganglion cell), bipolar cells (rod bipolar cell, cone bipolar cell), Muller glial cells, retinal pigment epithelial (RPE) cells, ciliary body, their progenitor cells (e.g., photoreceptor progenitor cell, bipolar progenitor cell), and retinal progenitor cells are included, though not limited thereto. Among the retinal cells, examples of cells constituting a neural retinal layer (also referred to as neural retina cells or neural retina-related cells) specifically include cells such as photoreceptor cells (rod photoreceptor cell, cone photoreceptor cell), horizontal cells, amacrine cells, intermediate neuronal cells, retinal ganglion cells (ganglion cell), bipolar cells (rod bipolar cell, cone bipolar cell), Muller glial cells, and their progenitor cells (e.g., photoreceptor progenitor cell, bipolar progenitor cell). In other words, in the neural retina-related cells, neither retinal pigment epithelial cells nor ciliary body cells are included.

The "matured retinal cells" mean cells that may be contained in the retinal tissue of a human adult, and specifically mean differentiated cells such as photoreceptor cells (rod photoreceptor cell, cone photoreceptor cell), horizontal cells, amacrine cells, intermediate neuronal cells, retinal ganglion cells (ganglion cell), bipolar cells (rod bipolar cell, cone bipolar cell), Muller glial cells, retinal pigment epithelial (RPE) cells, and ciliary body cells. The "immature retinal cells" mean progenitor cells (e.g., photoreceptor progenitor cell, bipolar progenitor cell, retinal progenitor cell) destined for differentiation into matured retinal cells.

The photoreceptor progenitor cells, the horizontal progenitor cells, the bipolar progenitor cells, the amacrine progenitor cells, the retinal ganglion progenitor cells, the Muller glial progenitor cells, and the retinal pigment epithelial progenitor cells refer to progenitor cells destined for differentiation into photoreceptor cells, horizontal cells, bipolar cells, amacrine cells, retinal ganglion cells, Muller glial cells, and retinal pigment epithelial cells, respectively.

The "retinal progenitor cells" are progenitor cells capable of differentiating into any one of the immature retinal cells such as photoreceptor progenitor cells, horizontal progenitor cells, bipolar progenitor cells, amacrine progenitor cells, retinal ganglion progenitor cells, Muller glial cells, and retinal pigment epithelial progenitor cells, and refer to progenitor cells also capable of eventually differentiating into any one of the matured retinal cells such as photoreceptor cells, rod photoreceptor cells, cone photoreceptor cells, horizontal cells, bipolar cells, amacrine cells, retinal ganglion cells, and retinal pigment epithelial cells.

The "photoreceptor cells" are present in the photoreceptor layer of a retina *in vivo* and plays a role in absorbing light stimuli and converting them to electrical signals. The photoreceptor cells have two types, cones which function in the light and rods which function in the dark (referred to as cone photoreceptor cells and rod photoreceptor cells, respectively). Examples of the cone photoreceptor cells can include S cone photoreceptor cells which express S-opsin and receive blue light, L cone photoreceptor cells which express L-opsin and receive red light, and M cone photoreceptor cells which express M-opsin and receive green light. The photoreceptor cells are matured after differentiation from photoreceptor progenitor cells. Whether or not cells are photoreceptor cells or photoreceptor progenitor cells can be readily confirmed by those skilled in the art, for example, through the expression of cell markers (Crx and Blimp1 expressed in photoreceptor progenitor cells, recoverin expressed in photoreceptor cells, rhodopsin, S-opsin and M/L-opsin expressed in mature photoreceptor cells, etc.) mentioned later or the formation of an outer segment structure. In an embodiment, the photoreceptor progenitor cells are Crx-positive cells, and the photoreceptor cells are rhodopsin-, S-opsin- and M/L-opsin-positive cells. In an embodiment, the rod photoreceptor cells are NRL- and rhodopsin-positive cells. In an embodiment, the S cone photoreceptor cells are S-opsin-positive cells, the L cone photoreceptor cells are L-opsin-positive cells, and the M cone photoreceptor cells are M-opsin-positive cells. Specifically, in the specification, the photoreceptor cells conceptually include photoreceptor progenitor cells and matured photoreceptor cells.

The neural retina contains preferably 10% or more, more preferably 20% or more, of a photoreceptor cell or a photoreceptor progenitor cell.

The neural retina contains preferably 10% or more, more preferably 20% or more, of a neural retinal progenitor cell. Also, the neural retina contains preferably 10% or more of a photoreceptor progenitor cell. In an embodiment, the neural retina contains 3% or more of a matured photoreceptor cell.

The presence of neural retina-related cells can be confirmed from the presence or absence of expression of a neural retina-related cell-related gene (hereinafter, also referred to as "neural retina-related cell marker" or "neural retina marker"). The presence or absence of expression of the neural retina-related cell marker, or the ratio of neural retina-related cell marker-positive cells in a cell population or a tissue can be readily confirmed by those skilled in the art. Examples thereof include an approach using an antibody, an approach using nucleic acid primers, and an approach using sequencing reaction. As the approach using an antibody, the expression of a protein of the neural retina-related cell marker can be confirmed, for example, by dividing the number of predetermined neural retina-related cell marker-positive cells by the total number of cells in accordance with an approach such as flow cytometry or immunostaining using a commercially available antibody. As the approach using nucleic acid primers, the expression of RNA of the neural retina-related cell marker can be confirmed by, for example, PCR, semiquantitative PCR, or quantitative PCR (e.g., real-time PCR). As the approach using sequencing reaction, the expression of RNA of the neural retina-related cell marker can be confirmed using, for example, a nucleic acid sequencer (e.g., next-generation sequencer).

Examples of the neural retina-related cell marker include Rx (also referred to as Rax) and PAX6 expressed in retinal progenitor cells, Rx, PAX6 and Chx10 (also referred to as Vsx2) expressed in neural retinal progenitor cells, and Crx and Blimp1 expressed in photoreceptor progenitor cells. Examples thereof also include Chx10 strongly expressed in bipolar cells, PKCα, Goα, VSX1 and L7 expressed in bipolar cells, TuJ1 and Brn3 expressed in retinal ganglion cells, calretinin and HPC-1 expressed in amacrine cells, calbindin expressed in horizontal cells, recoverin expressed in photoreceptor cells and photoreceptor progenitor cells, rhodopsin expressed in rod cells, Nrl expressed in rod photoreceptor cells and rod photoreceptor progenitor cells, S-opsin and LM-opsin expressed in cone photoreceptor cells, RXR-γ expressed in cone cells, cone photoreceptor progenitor cells and ganglion cells, TRβ2, OTX2 and OC2 expressed in cone photoreceptor cells that appear at the early phase of differentiation among cone photoreceptor cells, or progenitor cells thereof, and Pax6 commonly expressed in horizontal cells, amacrine cells and ganglion cells.

The "positive cells" mean cells expressing a predetermined marker on the cell surfaces or within the cells. For example, the "Chx10-positive cells" mean cells expressing Chx10 protein.

The "retinal pigment epithelial cells" mean epithelial cells present outside the neural retina in a retina *in vivo.* Whether or not cells are retinal pigment epithelial cells can be readily confirmed by those skilled in the art, for example, through the expression of cell markers (RPE65, MITF, CRALBP, MERTK, BEST1, TTR, etc.), the presence of melanin granules (brown-black), intercellular tight junctions, or polygonal/flagstone-like characteristic cell morphology. Whether or not cells have a function of retinal pigment epithelial cells can be readily confirmed from the ability to secrete cytokines such as VEGF and PEDF. In an embodiment, the retinal pigment epithelial cells are RPE65-positive cells, MITF-positive cells, or RPE65-positive and MITF-positive cells.

The "retinal layer" means individual layers constituting the retina, and examples thereof can specifically include retinal pigment epithelial layer, photoreceptor layer, outer limiting membrane, outer nuclear layer, outer plexiform layer, inner nuclear layer, inner plexiform layer, ganglion cell layer, nerve fiber layer and inner limiting membrane.

The "neural retinal layer" means individual layers constituting the neural retina, and examples thereof can specifically include photoreceptor layer, outer limiting membrane, outer nuclear layer, outer plexiform layer, inner nuclear layer, inner plexiform layer, ganglion cell layer, nerve fiber layer and inner limiting membrane. The "photoreceptor layer" means a retinal layer that is formed in the outermost of the neural retina and is rich in one or more cells selected from the group consisting of a photoreceptor cell (rod photoreceptor cell, cone photoreceptor cell), a photoreceptor progenitor cell and a retinal progenitor cell. Each layer other than the photoreceptor layer is referred to as an inner layer. Which retinal layer the individual cells constitute can be confirmed by a known method, for example, by determining the presence or absence of expression or expression level of a cell marker.

In the case of retinal tissue at a stage where the appearance ratio of photoreceptor cells or photoreceptor progenitor cells is low, a layer containing proliferating neural retinal progenitor cells is referred to as "neuroblastic layer" and includes inner neuroblastic layer and outer neuroblastic layer. Those skilled in the art can make a judgment from the shade of color (the outer neuroblastic layer is light, and the inner neuroblastic layer is dark) by a known method, for example, under a bright field microscope.

The "ciliary body" includes "ciliary body" and "ciliary marginal zone" in the process of development and of an adult. Examples of a marker of the "ciliary body" include Zic1, MAL, HNF1beta, FoxQ1, CLDN2, CLDN1, GPR177, AQP1 and AQP4. Examples of the "ciliary marginal zone (CMZ)" can include a tissue that is present in a boundary region between the neural retina and the retinal pigment epithelium in a retina *in vivo,* and is a region containing tissue stem cells of the retina (retinal stem cells). The ciliary marginal zone is also called ciliary margin or retinal margin, and the ciliary marginal zone, the ciliary margin and the retinal margin are equivalent tissues. The ciliary marginal zone is known to play an important role in the supply of retinal progenitor cells or differentiated cells to retinal tissue, the maintenance of a retinal tissue structure, etc. Examples of a marker gene of the ciliary marginal zone can include *Rdh10* gene (positive), *Otx1* gene (positive) and *Zic1* (positive). The "ciliary marginal zone-like structure" is a structure similar to the ciliary marginal zone.

The "cell aggregate" is not particularly limited as long as a plurality of cells mutually adhere to form a three-dimensional structure, and refers to, for example, a mass formed by the aggregation of cells dispersed in a vehicle such as a culture medium, or a mass of cells formed through cell division. In the cell aggregate, the case of forming a predetermined tissue is also included.

The "sphere-like cell aggregate" means a cell aggregate having a stereoscopic shape close to a spherical shape. The stereoscopic shape close to a spherical shape is a shape having a three-dimensional structure, and examples thereof include a spherical shape that exhibits a circle or an ellipse when projected onto a two-dimensional surface, and a shape formed by fusing a plurality of spherical shapes (e.g., which exhibits a shape formed by 2 to 4 circles or ellipses overlapping when two-dimensionally projected). In an embodiment, the core part of the aggregate has a vesicular lamellar structure and is characterized in that the central part is observed to be dark and the outer edge portion is observed to be bright under a bright field microscope.

The "epithelial tissue" is a tissue formed by covering the body surface or the surface of a lumen (digestive tract, etc.), body cavity (pericardial cavity, etc.) or the like with cells without any space. The cells forming the epithelial tissue are referred to as epithelial cells. The epithelial cells have a polarity in the apical-basal direction. The epithelial cells can mutually and firmly join via adherence junction and/or tight junction to form a layer of the cells. A tissue formed from a single layer or dozen layers overlapping of this layer of the cells is the epithelial tissue. In a tissue capable of forming the epithelial tissue, retinal tissue, brain and spinal cord tissue, eyeball tissue, neural tissue or the like of a fetal stage and/or an adult is also included. In the specification, the neural retina is also the epithelial tissue. The "epithelial structure" means a structure characteristic of the epithelial tissue, such as apical surface or basal membrane.

In the specification, an epithelial structure containing neural tissue is referred to as neural epithelium. Particularly, an epithelial structure containing a neural retina is referred to as neural retinal epithelium.

The "continuous epithelial tissue" is a tissue having a continuous epithelium structure. The continuous epithelium structure is a structure where the epithelial tissue is continuously formed. The epithelium tissue continuously formed is a state in which 10 cells to $10^7$ cells, for example, in the tangent direction of the epithelial tissue, preferably 30 cells to $10^7$ cells, further preferably $10^2$ cells to $10^7$ cells, in the tangent direction, are aligned. The continuous epithelial structure does not have a structure where an apical surface is divided, as found in a rosette-like structure. In an embodiment, the number of cells per area of the cross section of retinal tissue having the continuous epithelial structure, for example, the number of cells per area of the cross section of retinal tissue having the continuous epithelial structure in an embodiment of cells in a frozen section having a thickness on the order of 10 $\mu$m, is 10 cells to 900 cells, preferably 30 cells to 300 cells, more preferably 50 cells to 250 cells, still more preferably 75 cells to 160 cells, per 100 $\mu$m$^2$, for example, in the case of evaluating the number of nuclei of cells in the frozen section having a thickness on the order of 10 μm.

For example, in the continuous epithelium formed in retinal tissue, the retinal tissue has an apical surface intrinsic to the epithelial tissue. The apical surface is formed almost in parallel to, for example, at least photoreceptor layer (outer nuclear layer) among the layers forming a neural retinal layer and continuously on the surface of the retinal tissue. For example, in the case of a cell aggregate containing retinal tissue prepared from pluripotent stem cells, the apical surface is formed on the surface of the aggregate, and continuous neural epithelium is formed in which 10 cells or more, preferably 30 cells or more, more preferably 100 cells or more, further preferably 400 cells or more of photoreceptor cells or photoreceptor progenitor cells are regularly and continuously arranged in a row in the tangent direction of the surface. A neural retina containing such continuous neural epithelium is neural retinal epithelium containing continuous epithelium.

In an embodiment, epithelial tissue is polarized so that "apical surface" and "basal membrane" are formed. The "basal membrane" refers to a basal side layer (basal membrane) produced by epithelial cells, is rich in laminin and IV-type collagen, and has a thickness of 50 to 100 nm. The "apical surface" refers to the surface (upper surface layer) formed on the opposite side to the "basal membrane". In an embodiment, in the retinal tissue developed to the extent that photoreceptor cells or photoreceptor progenitor cells are observed, the "apical surface" refers to a surface in contact with photoreceptor layer (outer nuclear layer) in which outer limiting membrane is formed and photoreceptor cells and photoreceptor progenitor cells are present. Such an apical surface can be identified by, for example, immunostaining (known to those skilled in the art) using an antibody against an apical surface marker (e.g., atypical PKC (hereinafter, abbreviated to "aPKC"), E-cadherin, N-cadherin).

Whether retinal tissue contains continuous epithelium can be confirmed from the continuity (i.e., undivided form) of the apical surface of retinal tissue. The continuity of the apical surface can be determined, for example, by immunostaining a marker of the apical surface (e.g., aPKC, E-cadherin, N-cadherin) and a marker of photoreceptor cells or photoreceptor progenitor cells positioned on the apical surface side (e.g., Crx or recoverin), and analyzing obtained images, etc. for the positional relationship of the apical surface to a photoreceptor layer and each retinal layer. A retinal layer other than the apical surface or the photoreceptor layer (outer nuclear layer) can be identified by, for example, DAPI staining which stains the nuclei of cells, PI staining, Hoechst staining, or immunostaining with a marker protein (Rx, Chx10, Ki67, Crx, etc.) or the like localized in the nuclei of cells.

Specifically, the continuity of the apical surface can be identified from the continuous presence of cells co-expressing a marker of cells present on the apical surface side, i.e., a photoreceptor cell marker or a photoreceptor progenitor cell marker, and a marker capable of staining the nuclei of cells.

[Complex]

The complex of the present invention comprises: two or more cell aggregates each containing a neural retina derived from a pluripotent stem cell; and a matrix, the two or more cell aggregates being arranged in the matrix. Hereinafter, the neural retina-containing cell aggregates, the matrix, and the complex comprising them will be described in detail.

(1) Neural retina-containing cell aggregate

In an embodiment, the neural retina-containing cell aggregates are cell aggregates mentioned later (sphere-like cell aggregates). In an embodiment, the neural retina-containing cell aggregates are sheet-shaped retinal tissues dissected from sphere-like cell aggregates. The sheet-shaped retinal tissues are preferably sheet-shaped neural retinas containing continuous neural retinal epithelium (hereinafter, also referred to as neural retina sheets). In an embodiment, the neural retina-containing cell aggregates are transplant neural retinas, preferably transplant neural retina sheets. The transplant neural retinas or the transplant neural retina sheets are human neural retinas suitable for transplantation in humans and more preferably consist of only the neural retinas.

In the neural retina, a neural retinal layer containing at least a photoreceptor layer is formed, and the photoreceptor layer contains at least one or more cells selected from the group consisting of a photoreceptor cell, a photoreceptor progenitor cell and a retinal progenitor cell. The photoreceptor layer is formed at least in the outmost of the cell aggregate. Also, photoreceptor cells or photoreceptor progenitor cells may be present inside the cell aggregate. Alternatively, the photoreceptor layer may be formed in the inside. Photoreceptor cells, etc. are present continuously, i.e., by mutual adhesion, in the tangent direction of the surface of the cell aggregate. The photoreceptor cells, etc. are present continuously in the tangent direction of the surface of the cell aggregate, thereby forming a photoreceptor layer containing the photoreceptor cells, etc. The tangent direction refers to a direction tangent to the surface of the cell aggregate, i.e., a direction along which the photoreceptor cells, etc. in the photoreceptor layer are arranged, and is the direction in parallel to the neural retina or the lateral direction.

The neural retina is derived from a pluripotent stem cell, and it is preferable to be derived from a human pluripotent stem cell. The human pluripotent stem cell is preferably a human embryonic stem cell or a human induced pluripotent stem cell, more preferably a human induced pluripotent stem cell. Also, it is preferable that the neural retina should be a transplant neural retina.

In the specification, one embodiment of the retinal tissue includes retinal tissue containing a surface (apical surface) and a back surface (basal surface), wherein the surface constitutes an apical surface containing a neural retinal layer which is epithelial tissue by forming the adherence junction between cells, and the back surface constitutes a basal surface adjacent to the inner layer of the neural retina. Such retinal tissue can be referred to as neural retinal epithelium. For such retinal tissue, it is preferable to be a neural retina sheet which is a sheet-shaped retinal tissue. The surface has a smooth shape with less change in curvature, and the back surface has an irregular shape with large change in curvature. In an embodiment, the change in the curvature of the surface of the retinal tissue may be, for example, close to change in curvature of an ellipse (e.g., a major axis of 1 to 10 with respect to a minor axis of 1) (also referred to as continuous change in curvature). In an embodiment, the change in the curvature of the back surface of the retinal tissue may be, for example, close to sharp change in curvature that goes back and force between positive values and negative values, as in "teeth of a saw" (also referred to as sharp change in curvature).

(Method for producing cell aggregate)

Each cell aggregate containing a neural retina is derived from a pluripotent stem cell and, specifically, can be obtained by differentiating pluripotent stem cells. Examples of a differentiation method for a neural retina include, but are not particularly limited to, methods disclosed in WO2011/055855, WO2013/077425, WO2015/025967, WO2016/063985, WO2016/063986, WO2017/183732, PLoS One. 2010 Jan 20; 5 (1): e8763, Stem Cells. 2011 Aug; 29 (8): 1206-18, Proc Natl Acad Sci USA. 2014 Jun 10; 111 (23): 8518-23, and Nat Commun. 2014 Jun 10; 5: 4047.

Examples of a method for producing retinal tissue can include methods described in Bryce T. McLelland et al., IOVS, May 2018, Vol. 59, No. 6, p. 2586. Other examples of the method for producing retinal tissue include methods described in the following literatures.

Nakano, T. et al. Cell Stem Cell 10, 771-785 (2012).
Kawahara A. et al. Nature Communications, 6, p. 6286 (2015).
Kuwahara A, Yamasaki S, et al. Sci Rep. 2019 Dec 12; 9 (1): 18936.
Lamba, D.A., Gust, J. & Reh, T.A. Cell Stem Cell 4, 73-79, (2009).
Zhu, J., Cifuentes, H., Reynolds, J. & Lamba, D.A. Cell Stem Cell 20, 374-384. e375 (2017)
Meyer, J.S. et al. Stem Cells 29, 1206-1218, (2011).
Zhong, X. et al. Nat Commun 5, doi: 10.1038/ncomms5047 (2014).
Boucherie, C., et al. Stem Cells 31, 408-414, doi: 10.1002/stem.1268 (2013).
Gonzalez-Cordero, A. et al. Nat Biotechnol31, 741-747, (2013).
Mellough, C.B. et al. Stem Cells 33, 2416-2430, (2015).
Hallam, D. et al. Stem Cells, doi: 10.1002/stem.2883 (2018).
Reichman, S. et al. PNAS 111, 8518-8523, (2014).
Gagliardi, G. et al. Stem Cell Reports 11, 665-680, (2018).
Tucker, B.A., et al. Stem Cells Transl Med2, 16-24, (2013).
Wahlin, K.J. et al. Sci Rep 7, 766, (2017).
DiStefano, T. et al. Stem Cell Reports 10, 300-313, (2018).

In a specific embodiment, the cell aggregate containing a neural retina can be prepared by a method comprising the following steps (A), (B), (C) and (D):

(A) culturing pluripotent stem cells in a culture medium for pluripotent stem cell culture in the absence of feeder cells;
(B) forming a cell aggregate by suspension-culturing the cells obtained in the step (A);
(C) further suspension-culturing the cell aggregate obtained in the step (B) in a culture medium containing a BMP signaling pathway agonist; and
(D) suspension-culturing and maturing the cell aggregate obtained in the step (C).

The step (A) may further involve a TGFβ family signaling pathway inhibitor and/or a sonic hedgehog signaling pathway agonist.

Also, the step (B) may involve a sonic hedgehog signaling pathway agonist and/or a Wnt signaling pathway inhibitor, as mentioned later.

This method is also disclosed in, for example, WO2015/025967, WO2016/063985, and WO2017/183732. For more details, see WO2015/025967, WO2016/063985, WO2017/183732, WO2019/017492, WO2019/054514, WO2019/054515, etc.

The "culture medium for pluripotent stem cell culture" that is used in the step (A) is a culture medium that allows pluripotent stem cells to be cultured under feeder-free conditions. Examples of the culture medium include culture media containing a factor for maintaining undifferentiated state.

In the specification, the factor for maintaining undifferentiated state is not particularly limited as long as it is a substance having an action of suppressing the differentiation of pluripotent stem cells. Examples of the factor for maintaining undifferentiated state that is generally used by those skilled in the art can include FGF signaling pathway agonists, TGFβ family signaling pathway agonists, and insulin. Examples of the FGF signaling pathway agonist specifically include fibroblast growth factors (e.g., bFGF, FGF4, FGF8). Examples of the TGFβ family signaling pathway agonist include TGFβ signaling pathway agonists and Nodal/activin signaling pathway agonists. Examples of the TGFβ signaling pathway agonist include TGFβ1 and TGFβ2. Examples of the Nodal/activin signaling pathway agonist include Nodal, activin A, and activin B. In the case of culturing human pluripotent stem cells (human ES cells, human iPS cells), the culture medium in the step (A) preferably contains bFGF as the factor for maintaining undifferentiated state.

The concentration of the factor for maintaining undifferentiated state in the culture medium that is used in the step (A) is a concentration capable of maintaining the undifferentiated state of the pluripotent stem cells to be cultured, and can be appropriately set by those skilled in the art. For example, specifically, in the case of using bFGF as the factor for maintaining undifferentiated state in the absence of feeder cells, its concentration is usually on the order of 4 ng to 500 ng/mL, preferably on the order of 10 ng to 200 ng/mL, more preferably on the order of 30 ng to 150 ng/mL.

Many synthetic media have been developed or are commercially available as culture media for pluripotent stem cell culture applicable under feeder-free conditions. Examples thereof include Essential 8 medium (manufactured by Life Technologies Corp.). The Essential 8 medium contains L-ascorbic acid-2-phosphate magnesium (64 mg/L), sodium selenium (14 $\mu$g/L), insulin (19.4 mg/L), NaHCOs (543 mg/L), transferrin (10.7 mg/L), bFGF (100 ng/mL), and the TGFβ family signaling pathway agonist (TGFβ1 (2 ng/mL) or Nodal (100 ng/mL)) as additives in DMEM/F12 medium (Nature Methods, 8, 424-429 (2011)). Examples of other commercially available feeder-free media include S-medium (manufactured by DS Pharma Biomedical Co., Ltd.), StemPro (manufactured by Life Technologies Corp.), hESF9 (Proc. Natl. Acad. Sci. USA. 2008 Sep 9; 105 (36): 13409-14), mTeSR1 (manufactured by STEMCELL Technologies Inc.), mTeSR2 (manufactured by STEMCELL Technologies Inc.), TeSR-E8 (manufactured by STEMCELL Technologies Inc.), and StemFit (manufactured by Ajinomoto Co., Inc.). In the step (A), the present invention can be conveniently carried out by using these. By using these culture media, it is possible to perform the culture of pluripotent stem cells under feeder-free conditions. The culture medium that is used in the step (A) is, as one example, a serum-free medium that is not supplemented with any of the BMP signaling pathway agonist, the Wnt signaling pathway agonist and the Wnt signaling pathway inhibitor.

The culture medium that is used in the preparation of the cell aggregate containing a neural retina, i.e., the culture medium that is used in the steps (B), (C) and (D), can employ a basal medium for cell proliferation (also referred to as a basal medium), unless otherwise specified. The basal medium for cell proliferation is not particularly limited as long as the culture of cells is possible. A basal medium commercially available as a culture medium for cell proliferation can be appropriately used. Specifically, examples thereof can include culture media that can be used in the culture of animal cells, such as BME medium, BGJb medium, CMRL 1066 medium, Glasgow MEM(GMEM) medium, Improved MEM Zinc Option medium, IMDM medium, Medium 199 medium, MEM medium, Eagle MEM medium, αMEM medium, DMEM medium, F-12 medium, DMEM/F12 medium, INMMIF12 medium, Ham's medium, RPMI 1640 medium, Fischer's medium, Leibovitz's L-15 medium and mixtures of these media. Alternatively, a culture medium supplemented with N2 medium which is an assisted culture medium may be used.

In the specification, the TGFβ family signaling pathway inhibitor refers to a substance inhibiting the TGFβ family signaling pathway, i.e., the signaling pathway transduced by the Smad family. Specifically, examples thereof can include TGFβ signaling pathway inhibitors (e.g., SB431542, LY-364947, SB505124, A-83-01), Nodal/activin signaling pathway inhibitors (e.g., SB431542, A-83-01) and BMP signaling pathway inhibitors (e.g., LDN193189, dorsomorphin). These substances are commercially available and can be obtained.

In the specification, the sonic hedgehog (hereinafter, also referred to as "Shh") signaling pathway agonist is a substance capable of enhancing signal transduction mediated by Shh. Examples of the Shh signaling pathway agonist include SHH, partial peptides of SHH (e.g., sonic hedgehog N-terminus (Shh-N), recombinant human sonic hedgehog (C24II) N-terminus (SHH-C24II), recombinant mouse sonic hedgehog (C25II) N-terminus (SHH-C25II)), hedgehog family proteins other than Shh (e.g., Hh, IHH, DHH, EHH, TwHH), PMA (purmorphamine), and SAG (smoothened agonist).

In the step (A), the concentrations of the TGFβ family signaling pathway inhibitor and the sonic hedgehog signaling pathway agonist can be concentrations capable of inducting differentiation into retinal cells. For example, SB431542 is used at a concentration of usually 0.1 to 200 $\mu$M, preferably 2 to 50 $\mu$M. A-83-01 is used at a concentration of usually 0.05 to 50 $\mu$M, preferably 0.5 to 5 $\mu$M. LDN193189 is used at a concentration of usually 1 to 2000 nM, preferably 10 to 300 nM. SAG is used at a concentration of usually 1 to 2000 nM, preferably 10 to 700 nM. PMA is used at a concentration of usually 0.002 to 20 $\mu$M, preferably 0.02 to 2 $\mu$M.

In the culture of pluripotent stem cells under feeder-free conditions in the step (A), a suitable matrix may be used as a scaffold in order to provide a scaffold as a replacement for feeder cells to the pluripotent stem cells. Examples of the matrix that can be used as a scaffold include laminin (Nat Biotechnol 28, 611-615, (2010)), laminin fragments (Nat Commun 3, 1236, (2012)), basal membrane preparations (Nat Biotechnol 19, 971-974, (2001)), gelatin, collagen, heparan sulfate proteoglycan, entactin, and vitronectin.

The culture time of the pluripotent stem cells in the step (A) is not particularly limited within a range in which an effect of improving the quality of the cell aggregate to be formed in the step (B) can be achieved in the case of culture in the presence of the TGFβ family signaling pathway inhibitor and/or the sonic hedgehog signaling pathway agonist (e.g., from 100 nM to 700 nM), and is usually from 0.5 to 144 hours. In an embodiment, it is preferably from 2 to 96 hours, more preferably from 6 to 48 hours, further preferably from 12 to 48 hours, still further preferably from 18 to 28 hours (e.g., 24 hours).

The culture medium that is used in the step (B) may be a serum-containing medium or a serum-free medium. A serum-free medium is suitably used from the viewpoint of circumventing contamination with chemically undetermined components. In order to circumvent the complication of preparation, examples thereof include serum-free media supplemented with an appropriate amount of a serum replacement such as commercially available KSR. The amount of KSR added to the serum-free medium is usually from about 1% to about 30%, preferably from about 2% to about 20%.

For the formation of the aggregate, first, dispersed cells are prepared by the dispersion operation of the cells obtained in the step (A). The "dispersed cells" obtained by dispersion operation include a state in which 70% (preferably 80% or more) or more are single cells and 30% or less (preferably 20% or less) of 2- to 50-cell masses are present. The dispersed cells include a state in which the mutual adhesion (e.g., surface adhesion) of cells has been mostly lost.

A suspension of the dispersed cells is seeded into an incubator, and the dispersed cells are cultured under conditions of non-adhesive to the incubator, thereby causing the aggregation of a plurality of cells to form an aggregate. In an embodiment, when a predetermined number of dispersed stem cells is placed in each well of a multi-well plate (U-bottom, V-bottom) such as a 96-well plate and this is statically cultured, the cells aggregate rapidly, thereby forming one aggregate in each well (SFEBq). In the case of suspension-culturing cells using a 96-well plate, a liquid prepared so as to attain about $1 \times 10^3$ to about $1 \times 10^5$ cells (preferably about $3 \times 10^3$ to about $5 \times 10^4$ cells or about $4 \times 10^3$ to about $2 \times 10^4$ cells) per well is added to the wells, and the plate is left standing to form aggregates.

In an embodiment, the culture medium that is used in the step (B) contains a sonic hedgehog signaling pathway agonist.

In other words, in a specific embodiment, the cell aggregate containing a neural retina can be prepared by a method comprising the following steps (A), (B) and (C):

(A) culturing pluripotent stem cells in a culture medium containing a factor for maintaining undifferentiated state and optionally containing a TGFβ family signaling pathway inhibitor and/or a sonic hedgehog signaling pathway agonist in the absence of feeder cells;
(B) forming a cell aggregate by suspension-culturing the cells obtained in the step (A) in a culture medium containing a sonic hedgehog signaling pathway agonist; and
(C) further suspension-culturing the cell aggregate obtained in the step (B) in a culture medium containing a BMP signaling pathway agonist.

As the sonic hedgehog signaling pathway agonist in the step (B), the one mentioned above can be used at the concentration mentioned above (e.g., from 10 nM to 300 nM). The sonic hedgehog signaling pathway agonist is preferably contained in the culture medium from the start of suspension culture. A ROCK inhibitor (e.g., Y-27632) may be added to the culture medium. The culture time is, for example, from 12 hours to 6 days. The culture medium that is used in the step (B) is, as one example, a culture medium that is not supplemented with one or more (preferably all) selected from the group consisting of a BMP signaling pathway agonist, a Wnt signaling pathway agonist, a TGFβ family signaling pathway inhibitor and a TGFβ family signaling pathway agonist.

In an embodiment, the cell aggregate in the step (B) is at a stage of differentiation where a pluripotency marker is expressed. Specifically, it is a state of differentiation where one or more markers selected from Oct3/4, Sox2, Klf4, Nanog, Sall4, lin28, Esrrb and Esrrb are detectable.

In the specification, the BMP signaling pathway agonist is a substance capable of enhancing the signaling pathway mediated by BMP. Examples of the BMP signaling pathway agonist include BMP protein such as BMP2, BMP4 and BMP7, GDF protein such as GDF7, anti-BMP receptor antibodies, and BMP partial peptides. The BMP2 protein, the BMP4 protein and the BMP7 protein are available from, for example, R&D Systems, Inc., and the GDF7 protein is available from, for example, Wako Pure Chemical Industries, Ltd.

Examples of the culture medium that is used in the step (C) include serum-free media and serum media (preferably serum-free media) supplemented with a BMP signaling pathway agonist. The serum-free medium and the serum medium can be provided as mentioned above. The culture medium that is used in the step (C) is, as one example, a culture medium that is not supplemented with one or more (preferably all) selected from the group consisting of a Wnt signaling pathway agonist, a TGFβ family signaling pathway inhibitor and a TGFβ family signaling pathway agonist. Alternatively, the culture medium that is used in the step (C) is, as one example, a culture medium that is not supplemented with a sonic hedgehog signaling pathway agonist. Alternatively, the culture medium that is used in the step (C) is a culture medium that may be supplemented with a Wnt signaling pathway agonist.

The concentration of the BMP signaling pathway agonist can be a concentration capable of inducing differentiation into retinal cells. For example, human BMP4 protein is added to the culture medium so as to attain a concentration of about 0.01 nM to about 1 μM, preferably about 0.1 nM to about 100 nM, more preferably about 1 nM to about 10 nM, further preferably about 1.5 nM (55 ng/mL).

The BMP signaling pathway agonist can be added about 24 hours or later after the start of suspension culture in the step (A), and may be added to the culture medium within several days (e.g., within 15 days) after the start of suspension culture. Preferably, the BMP signaling pathway agonist is added to the culture medium between Day 1 and Day 15, more preferably between Day 1 and Day 9, most preferably on Day 3, after the start of suspension culture.

In a specific embodiment, a part or the whole of the culture medium is exchanged with a culture medium containing BMP4, for example, on Days 1 to 9, preferably Days 1 to 3, after the start of suspension culture in the step (B), and the medium is prepared such that the final concentration of BMP4 becomes about 1 to 10 nM. Culture can be performed for, for example, 1 to 12 days, preferably 2 to 9 days, further preferably 2 to 5 days, in the presence of BMP4. In this context, in order to maintain the concentration of BMP4 at the same concentration, a part or the whole of the culture medium can be exchanged with a culture medium containing BMP4 once or about twice. Alternatively, the concentration of BMP4 may be decreased in stages. For example, the concentration of the BMP signaling pathway agonist (BMP4) is maintained from Days 2 to 10 after the start of suspension culture in the step (B), and then, the concentration of the BMP signaling pathway agonist (BMP4) may be decreased in stages from Days 6 to 20 after the start of suspension culture in the step (B).

Culture conditions such as culture temperature and $CO_2$ concentration in the step (A) to the step (C) can be appropriately set. The culture temperature is, for example, from about 30°C to about 40°C, preferably about 37°C. The $CO_2$ concentration is, for example, from about 1% to about 10%, preferably about 5%.

Retinal cells at various stages of differentiation can be produced as retinal cells contained in the cell aggregate by varying the culture period in the step (C). In other words, retinal cells in the cell aggregate containing immature retinal cells (e.g., retinal progenitor cell, photoreceptor progenitor cell) and matured retinal cells (e.g., photoreceptor cell) at various ratios can be produced. The ratio of matured retinal cells can be increased by extending the culture period in the step (C).

The step (B) and/or the step (C) may employ a method disclosed in WO2017/183732. Specifically, in the step (B) and/or the step (C), the cell aggregate can be formed by suspension culture in a culture medium further containing a Wnt signaling pathway inhibitor.

The Wnt signaling pathway inhibitor that is used in the step (B) and/or the step (C) is not particularly limited as long as it is capable of suppressing signal transduction mediated by Wnt, and may be any of a protein, a nucleic acid, a low-molecular compound, and the like. Signals mediated by Wnt are transduced via Wnt receptor present as a heterodimer of frizzled (Fz) and LRP5/6 (low-density lipoprotein receptor-related protein 5/6). Examples of the Wnt signaling pathway inhibitor include, but are not limited to, substances acting directly on Wnt or Wnt receptor (anti-Wnt neutralizing antibody, anti-Wnt receptor neutralizing antibody, etc.), substances suppressing the expression of a gene encoding Wnt or Wnt receptor (e.g., antisense oligonucleotide, siRNA), substances inhibiting the binding of Wnt to Wnt receptor (soluble Wnt receptor, dominant negative Wnt receptor, etc., Wnt antagonist, Dkk1, Cerberus protein, etc.), and substances inhibiting bioactivity caused by signal transduction ascribable to Wnt receptor [e.g., low-molecular compounds such as CKI-7 (N-(2-aminoethyl)-5-chloroisoquinoline-8-sulfonamide), D4476 (4-[4-(2,3-dihydro-1,4-benzodioxin-6-yl)-5-(2-pyridinyl)-1H-imidazol-2 -yl]benzamide), IWR-1-endo (IWR1e) (4-[(3aR,4S,7R,7aS)-1,3,3a,4,7,7a-hexahydro-1,3-dioxo-4,7-methano-2 H-isoindol-2-yl]-N-8-quinolinyl-benzamide), and IWP-2 (N-(6-methyl-2-benzothiazolyl)-2-[(3,4,6,7-tetrahydro-4-oxo-3-phenylt hieno[3,2-d]pyrimidin-2-yl)thio]acetamide)]. One or two or more of these may be contained as the Wnt signaling pathway inhibitor. CKI-7, D4476, IWR-1-endo (IWR1e), IWP-2, and the like are known Wnt signaling pathway inhibitors, and commercially available products, etc. can be appropriately obtained. IWR1e is preferably used as the Wnt signaling pathway inhibitor.

The concentration of the Wnt signaling pathway inhibitor in the step (B) can be a concentration capable of inducing the favorable formation of the cell aggregate. For example, IWR-1-endo is added to the culture medium so as to attain a concentration of about 0.1 μM to about 100 μM, preferably about 0.3 μM to about 30 μM, more preferably about 1 μM to about 10 μM, further preferably about 3 μM. In the case of using a Wnt signaling pathway inhibitor other than IWR-1-endo, it is desirable to be used at a concentration that exhibits Wnt signaling pathway inhibitory activity equivalent to the concentration of IWR-1-endo.

In the step (B), the timing of adding the Wnt signaling pathway inhibitor to the culture medium is preferably earlier. The Wnt signaling pathway inhibitor is added to the culture medium usually within 6 days, preferably within 3 days, more preferably within 1 day, more preferably within 12 hours, from the start of suspension culture in the step (B), further preferably at the start of suspension culture in the step (B). Specifically, for example, the addition of a basal medium supplemented with the Wnt signaling pathway inhibitor, or the exchange of a part or the whole of the culture medium with the basal medium can be performed. Although a period for which the Wnt signaling pathway inhibitor is allowed to act on the cells obtained in the step (A) in the step (B) is not particularly limited, preferably, it is added to the culture medium at the start of suspension culture in the step (B) and then allowed to act until the completion of the step (B) (immediately before addition of a BMP signaling pathway agonist). Further preferably, as mentioned later, exposure to the Wnt signaling pathway inhibitor is continued even after the completion of the step (B) (i.e., during the period of the step (C)). In an embodiment, as mentioned later, the action of the Wnt signaling pathway inhibitor is continued even after the completion of the step (B) (i.e., during the period of the step (C)), and the action may be performed until retinal tissue is formed.

In the step (C), as the Wnt signaling pathway inhibitor, any of the Wnt signaling pathway inhibitors mentioned above can be used. Preferably, the same type as the Wnt signaling pathway inhibitor used in the step (B) is used in the step (C).

The concentration of the Wnt signaling pathway inhibitor in the step (C) can be a concentration capable of inducing retinal progenitor cells and retinal tissue. For example, IWR-1-endo is added to the culture medium so as to attain a concentration of about 0.1 $\mu$M to about 100 $\mu$M, preferably about 0.3 $\mu$M to about 30 $\mu$M, more preferably about 1 $\mu$M to about 10 $\mu$M, further preferably about 3 $\mu$M. In the case of using a Wnt signaling pathway inhibitor other than IWR-1-endo, it is desirable to be used at a concentration that exhibits Wnt signaling pathway inhibitory activity equivalent to the concentration of IWR-1-endo. The concentration of the Wnt signaling pathway inhibitor in the culture medium in the step (C) is preferably 50 to 150, more preferably 80 to 120, further preferably 90 to 110, when the concentration of the Wnt signaling pathway inhibitor in the culture medium in the step (B) is defined as 100. It is more preferable to be equivalent to the concentration of the Wnt signaling pathway inhibitor in the culture medium in the step (B).

The timing of addition of the Wnt signaling pathway inhibitor to the culture medium is not particularly limited within a range that can achieve the formation of an aggregate containing retinal cells or retinal tissue, and is preferably earlier. Preferably, the Wnt signaling pathway inhibitor is added to the culture medium at the start of the step (C). More preferably, the Wnt signaling pathway inhibitor is added in the step (B) and then also continuously (i.e., from the start of the step (B)) contained in the culture medium in the step (C). Further preferably, the Wnt signaling pathway inhibitor is added at the start of suspension culture in the step (B) and then also continuously contained in the culture medium in the step (C). For example, a BMP signaling pathway agonist (e.g., BMP4) can be added to the cultures (suspension of aggregates in a culture medium containing a Wnt signaling pathway inhibitor) obtained in the step (B).

A period for which the Wnt signaling pathway inhibitor is allowed to act is not particularly limited, but is preferably from 2 days to 30 days, more preferably from 6 days to 20 days, from 8 days to 18 days, from 10 days to 18 days, or from 10 days to 17 days (e.g., 10 days), with the start of suspension culture in the step (B) as a commencement when the Wnt signaling pathway inhibitor is added at the start of suspension culture in the step (B). In another embodiment, the period for which the Wnt signaling pathway inhibitor is allowed to act is preferably from 3 days to 15 days (e.g., 5 days, 6 days, 7 days), more preferably from 6 days to 10 days (e.g., 6 days), with the start of suspension culture in the step (B) as a commencement when the Wnt signaling pathway inhibitor is added at the start of suspension culture in the step (B).

A neural retina having a ciliary marginal zone-like structure can also be produced by culturing the cell aggregate obtained by the method mentioned above in a serum-free medium or a serum medium containing a Wnt signaling pathway agonist and/or a FGF signaling pathway inhibitor for a period on the order of 2 days to 4 days (step (D)), followed by culture in a serum-free medium or a serum medium containing neither a Wnt signaling pathway agonist nor a FGF signaling pathway inhibitor for about 30 days to about 200 days (from 30 days to 150 days, from 50 days to 120 days, from 60 days to 90 days) (step (E)).

In an embodiment, a neural retina having a ciliary marginal zone-like structure can be produced by the step (D) and the step (E) from the cell aggregate obtained in the steps (A) to (C), the cell aggregate being of Days 6 to 30 or Days 10 to 20 (Day 10, Day 11, Day 12, Day 13, Day 14, Day 15, Day 16, Day 17, Day 18, Day 19 or Day 20) after the start of suspension culture in the step (B).

The Wnt signaling pathway agonist is not particularly limited as long as it is capable of enhancing signal transduction mediated by Wnt. Examples of a specific Wnt signaling pathway agonist can include GSK3$\beta$ inhibitors (e.g., 6-bromoindirubin-3'-oxime (BIO), CHIR99021, kenpaullone). For example, in the case of CHIR99021, the range of about 0.1 $\mu$M to about 100 $\mu$M, preferably about 1 $\mu$M to about 30 $\mu$M, can be included.

The FGF signaling pathway inhibitor is not particularly limited as long as it can inhibit signal transduction mediated by FGF. Examples of the FGF signaling pathway inhibitor include SU-5402, AZD4547, and BGJ398. For example, SU-5402 is added at a concentration of about 0.1 $\mu$M to about 100 $\mu$M, preferably about 1 $\mu$M to about 30 $\mu$M, more preferably about 5 $\mu$M.

The culture medium that is used in the step (D) is, as one example, a culture medium that is not supplemented with one or more (preferably all) selected from the group consisting of a BMP signaling pathway agonist, a Wnt signaling pathway inhibitor, a SHH signaling pathway agonist, a TGFβ family signaling pathway inhibitor and a TGFβ family signaling pathway agonist.

A part of the step (E) or the whole step can perform culture using a culture medium for continuous epithelial tissue maintenance disclosed in WO2019/017492. Specifically, the continuous epithelium structure of the neural retina can be maintained by culture using a culture medium for continuous epithelial tissue maintenance. One example of the culture medium for continuous epithelial tissue maintenance can include a medium in which Neurobasal medium (e.g., manufactured by Thermo Fisher Scientific Inc., 21103049) is blended with B27 supplement (e.g., Thermo Fisher Scientific Inc., 12587010).

For the culture in the step (E), exchange with the culture medium for continuous epithelial tissue maintenance in stages is preferable for achieving both the differentiation and/or maturation of retinal cells (particularly, photoreceptor cell) and the maintenance of the continuous epithelium structure. For example, culture can be performed using a basal medium for cell proliferation (e.g., a culture medium in which DMEM/F12 medium is supplemented with 10% fetal bovine serum, 1% N2 supplement, and 100 μM taurine) for first 10 days to 30 days, a mixture of a basal medium for cell proliferation and a culture medium for continuous epithelial tissue maintenance (culture medium in which a medium in which DMEM/F12 medium is supplemented with 10% fetal bovine serum, 1% N2 supplement, and 100 μM taurine, and a medium in which Neurobasal medium is supplemented with 10% fetal bovine serum, 2% B27 supplement, 2 mM glutamine, and 100 μM taurine, are mixed at a ratio of 1:3) for next 10 days to 40 days, and a culture medium for continuous epithelial tissue maintenance (e.g., a culture medium in which Neurobasal medium is supplemented with 10% fetal bovine serum, 2% B27 supplement, 2 mM glutamine, and 100 μM taurine) for next 20 days to 140 days.

In a part of the step (E) or the whole step, in the case of using any medium of the basal medium for cell proliferation, the culture medium for continuous epithelial tissue maintenance or a mixture of these media, a thyroid hormone signaling pathway agonist may be further contained. For the details of a culture method using a culture medium containing a thyroid hormone signaling pathway agonist, see WO2019/054514. By culture in a culture medium containing a thyroid hormone signaling pathway agonist, the production of a cell aggregate containing a neural retina becomes possible in which the ratio of bipolar cells, amacrine cells, ganglion cells or horizontal cells, etc. contained in the neural retina is low and the ratio of photoreceptor progenitor cells has been increased.

In the specification, the thyroid hormone signaling pathway agonist is a substance capable of enhancing signal transduction mediated by thyroid hormone, and is not particularly limited as long as it is capable of enhancing the thyroid hormone signaling pathway. Examples of the thyroid hormone signaling pathway agonist include triiodothyronine (hereinafter, also abbreviated to T3), thyroxin (hereinafter, also abbreviated to T4), and thyroid hormone receptor (preferably TRβ receptor) agonists.

Examples of the thyroid hormone receptor agonist known to those skilled in the art can include compounds such as diphenylmethane derivatives, diaryl ether derivatives, pyridazine derivatives, pyridine derivatives and indole derivatives described in International Publication No. WO 97/21993, International Publication No. WO 2004/066929, International Publication No. WO 2004/093799, International Publication No. WO 2000/039077, International Publication No. WO 2001/098256, International Publication No. WO 2003/018515, International Publication No. WO 2003/084915, International Publication No. WO 2002/094319, International Publication No. WO 2003/064369, Japanese Unexamined Patent Publication No. 2002-053564, Japanese Unexamined Patent Publication No. 2002-370978, Japanese Unexamined Patent Publication No. 2000-256190, International Publication No. WO 2007/132475, International Publication No. WO 2007/009913, International Publication No. WO 2003/094845, International Publication No. WO 2002/051805 or International Publication No. WO 2010/122980.

In the case of using T3 as the thyroid hormone signaling pathway agonist, it can be added to the culture medium so as to attain, for example, the range of 0.1 to 1000 nM. Preferably, examples thereof include concentrations having thyroid hormone signaling enhancing activity that corresponds to T3 with a concentration of 1 to 500 nM; more preferably 10 to 100 nM; further preferably 30 to 90 nM; still more preferably around 60 nM. In the case of using T4 as the thyroid hormone signaling pathway agonist, it can be added to the culture medium so as to attain, for example, the range of 1 nM to 500 μM. Preferably, it is the range of 50 nM to 50 μM; more preferably 500 nM to 5 μM. In the case of using other thyroid hormone receptor agonists, the concentration can exhibit activity equivalent to the agonist activity exhibited by T3 or T4 with the concentration mentioned above.

The culture medium that is used in the step (E) may appropriately contain L-glutamine, taurine, serum, or the like. The culture medium that is used in the step (E) is, as one example, a culture medium that is not supplemented with one or more (preferably all) selected from the group consisting of a BMP signaling pathway agonist, a FGF signaling pathway inhibitor, a Wnt signaling pathway agonist, a Wnt signaling pathway inhibitor, a SHH signaling pathway agonist, a TGFβ family signaling pathway inhibitor and a TGFβ family signaling pathway agonist.

In a specific embodiment, the cell aggregate containing a neural retina can be prepared by a method comprising the following steps (A) to (E):

(A) culturing pluripotent stem cells in a culture medium containing a factor for maintaining undifferentiated state and optionally containing a TGFβ family signaling pathway inhibitor and/or a sonic hedgehog signaling pathway agonist in the absence of feeder cells;

(B) forming a cell aggregate by suspension-culturing the cells obtained in the step (A) in a culture medium optionally containing a Wnt signaling pathway inhibitor and/or a sonic hedgehog signaling pathway agonist;

(C) further suspension-culturing the cell aggregate obtained in the step (B) in a culture medium containing a BMP signaling pathway agonist;

(D) culturing the cell aggregate obtained in the step (C) in a serum-free medium or a serum medium containing a Wnt signaling pathway agonist and/or a FGF signaling pathway inhibitor for a period on the order of 2 days to 4 days; and

(E) culturing the cell aggregate obtained in the step (D) in a serum-free medium or a serum medium containing neither a Wnt signaling pathway agonist nor a FGF signaling pathway inhibitor and optionally containing a thyroid hormone signaling pathway agonist for about 30 days to about 200 days.

In a specific embodiment, the cell aggregate containing a neural retina can be prepared by a method comprising the following steps (A) to (E):

(A) culturing pluripotent stem cells in a culture medium containing a factor for maintaining undifferentiated state and containing a TGFβ family signaling pathway inhibitor and/or a sonic hedgehog signaling pathway agonist in the absence of feeder cells for 12 hours to 48 hours;

(B) forming a cell aggregate by suspension-culturing the cells obtained in the step (A) in a culture medium containing a Wnt signaling pathway inhibitor and/or a sonic hedgehog signaling pathway agonist for 12 hours to 72 days (24 hours to 48 hours);

(C) further suspension-culturing the cell aggregate obtained in the step (B) in a culture medium containing a BMP signaling pathway agonist for 8 days to 15 days (10 days to 13 days);

(D) culturing the cell aggregate obtained in the step (C) in a serum-free medium or a serum medium containing a Wnt signaling pathway agonist and/or a FGF signaling pathway inhibitor for 2 days to 4 days; and

(E) culturing the cell aggregate obtained in the step (D) in a serum-free medium or a serum medium containing neither a Wnt signaling pathway agonist nor a FGF signaling pathway inhibitor and optionally containing a thyroid hormone signaling pathway agonist for about 10 days to about 200 days.

In this context, the step (E) may comprise the step of performing culture in a basal medium for cell proliferation for 10 days to 30 days, subsequently performing culture in a mixture of a basal medium for cell proliferation and a culture medium for continuous epithelial tissue maintenance containing a thyroid hormone signaling pathway agonist for 10 days to 40 days, and further performing culture in a culture medium for continuous epithelial tissue maintenance containing a thyroid hormone signaling pathway agonist for 20 days to 140 days.

In an embodiment, the step (E) comprises performing culture in the presence of a thyroid hormone signaling pathway agonist for 20 days to 60 days (30 days to 50 days).

In an embodiment, the culture period from the step (B) to the step (E) is from 70 days to 100 days (from 80 days to 90 days).

The cell aggregate containing a neural retina can be produced by the method mentioned above, though not limited thereto. In an embodiment, the cell aggregate containing a neural retina can also be obtained as a mixture of cell aggregates. In another embodiment, for example, one cell aggregate may be produced per well of a 96-well plate, and cell aggregates containing a neural retina may be obtained one by one.

The neural retina according to the present invention comprises a neural retinal layer including a photoreceptor layer and an inner layer, contains, in the photoreceptor layer, one or more cells selected from the group consisting of a photoreceptor progenitor cell and a photoreceptor cell, has a structure of epithelial tissue, and has an apical surface and a basal surface. It is preferable that the structure of epithelial tissue should be a continuous epithelial structure.

In an embodiment, the neural retina according to the present invention can be obtained by dissecting it from a cell aggregate of a neural retina using tweezers, a knife, scissors, or the like. A neural retina sheet can be obtained by dissecting it from a cell aggregate of a sphere-like neural retina. The neural retina sheet means a stratified sheet-shaped structure that maintains the layer structure of the neural retinal layer mentioned above.

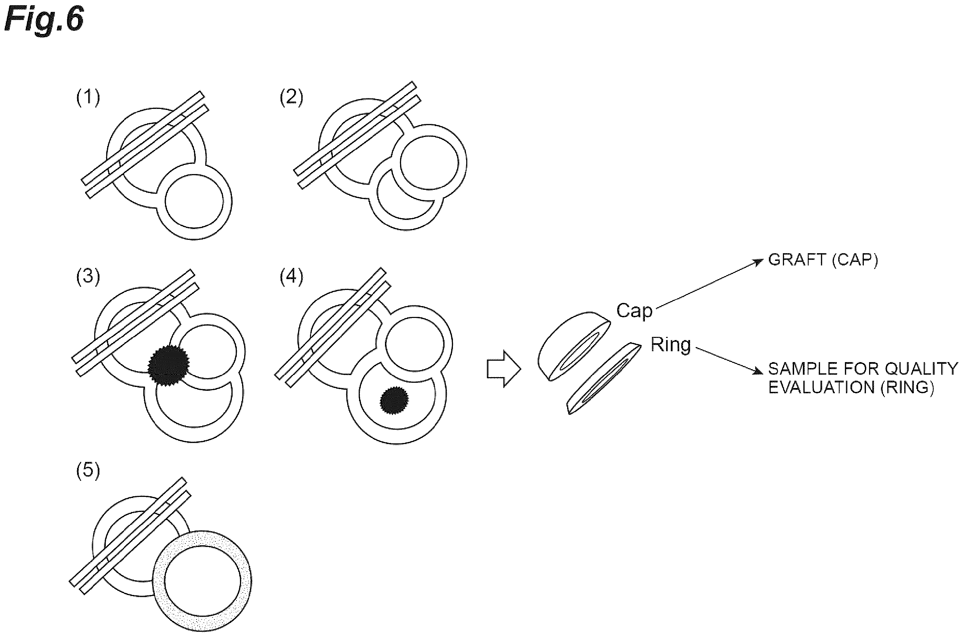

In an embodiment, in the cell aggregate containing a neural retina, a plurality of neural retinas may be present with an overlap (e.g., see conceptual views (1) and (2) in Figure 6). In an embodiment, the cell aggregate containing a neural retina contains at least first epithelial tissue and second epithelial tissue, wherein the first epithelial tissue contains a human neural retina, and the second epithelial tissue has the continuity of the slope of a tangent line to a surface different from the continuity of the slope of a tangent line to the surface of the first epithelial tissue, and may contain a cell other than a retinal cell and/or a retinal pigment epithelial cell. Examples of the cell other than a retinal cell contained in the second epithelial tissue include cells of eyeball-related tissue and brain and spinal cord tissue. Examples of the eyeball-related tissue include retinal pigment epithelial cells and ciliary marginal zone structures. The neural retina according to the present invention can be obtained by dissecting a neural retina from the aggregate so as not to contain the second epithelial tissue visually. The neural retina according to the present invention may contain a region on the first epithelial tissue most distant from the second epithelial tissue, particularly, a region of the center and/or its neighborhood of the first epithelial tissue, in the cell aggregates. In an embodiment, the neural retina according to the present invention may contain a region of the center and/or its neighborhood of continuous epithelial tissue.

In an embodiment, the cell aggregate containing a neural retina contains first epithelial tissue (target epithelial tissue) containing the transplant neural retina, and second epithelial tissue (non-target epithelial tissue) having the continuity of the slope of a tangent line to a surface different from the continuity of the slope of a tangent line to the surface of the first epithelial tissue, and containing a non-neural retina-related cell. In this context, the first epithelial tissue refers to epithelial tissue that does not substantially contain a non-neural retina-related cell (non-target cell) and allows the transplant neural retina to be dissected. On the other hand, the second epithelial tissue is epithelial tissue that may contain a neural retina, but is ineligible for dissecting the transplant neural retina because of containing non-target cells. In another embodiment, the cell aggregate containing a neural retina contains only the first epithelial tissue (target epithelial tissue) containing the transplant neural retina and does not contain non-target epithelial tissue.

The transplant neural retina contains at least a photoreceptor layer. The photoreceptor layer is formed at least in the outmost of the cell aggregate. Also, photoreceptor cells or photoreceptor progenitor cells may be present in the inside. Alternatively, the photoreceptor layer may be formed in the inside. Photoreceptor cells, etc. are present continuously, i.e., by mutual adhesion, in the tangent direction of the surface of the cell aggregate. The photoreceptor cells, etc. are present continuously in the tangent direction of the surface of the cell aggregate, thereby forming a photoreceptor layer containing the photoreceptor cells, etc. The tangent direction refers to a direction tangent to the surface of the cell aggregate, i.e., a direction along which the photoreceptor cells, etc. in the photoreceptor layer are arranged, and is the direction in parallel to the neural retina or the lateral direction. The slope of a tangent line to the surface of epithelial tissue refers to a direction along which cells are arranged when individual cells in the epithelial tissue are arranged in a predetermined direction, and refers to the direction in parallel to the epithelial tissue (or epithelial sheet) or the lateral direction.

The cell aggregate that is used for preparing the transplant neural retina may contain non-target tissue other than a neural retina. Examples of the non-target tissue include epithelial tissue other than a neural retina, i.e., second epithelial tissue containing non-target epithelial tissue. Examples of the second epithelial tissue include eyeball-related tissue and brain and spinal cord tissue. The eyeball-related tissue means a non-neural retinal tissue surrounding eyeball tissue, and examples thereof include retinal pigment epithelial cells, ciliary body (e.g., ciliary marginal zone), lens, and cornea. The brain and spinal cord tissue means neural tissue of the brain and the spinal cord, and examples thereof include the forebrain, the telencephalon, the cerebrum, the diencephalon, the hypothalamus, the midbrain, the hindbrain, the cerebellum, and the spinal cord. In an embodiment, the brain and spinal cord tissue may contain pituitary gland.

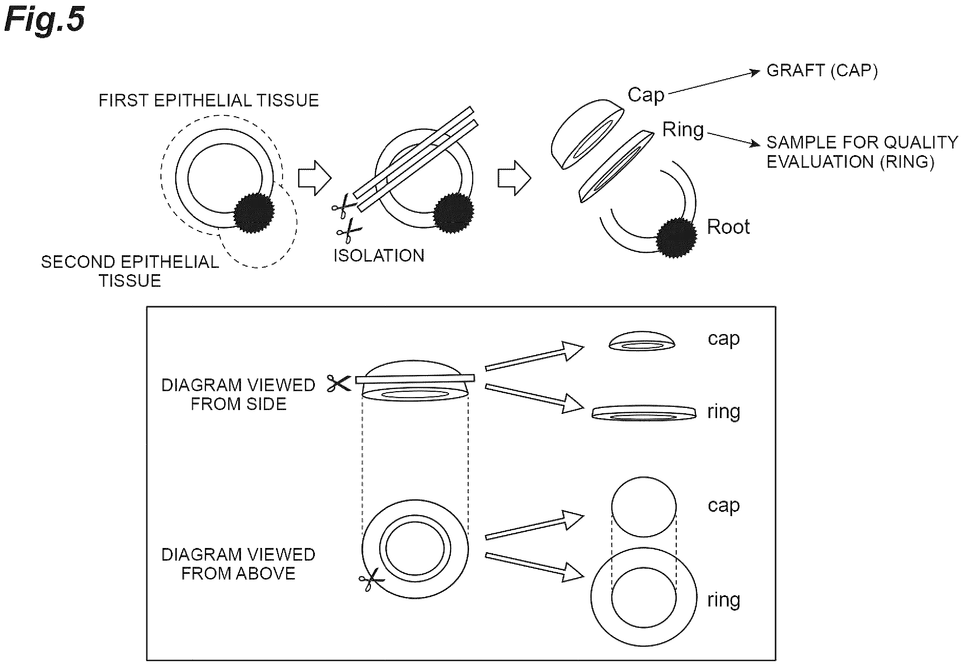

One example of the cell aggregate containing the first epithelial tissue and the second epithelial tissue includes cell aggregates shown in a conceptual view of Figure 5 and conceptual views (3) and (5) of Figure 6. The conceptual view of Figure 5 shows one example of a cell aggregate in which eyeball-related tissue (retinal pigment epithelial cells, ciliary body) (black portion of Figure 5) is present as the second epithelial tissue in a part of a neural retina which is the first epithelial tissue. The conceptual view (3) of Figure 6 shows one example of a cell aggregate in which eyeball-related tissue (retinal pigment epithelial cells, ciliary body) (black portion of the conceptual view (3) of Figure 6) is further present as the second epithelial tissue when a plurality of neural retinas are present with an overlap (e.g., conceptual views (1) and (2) of Figure 6). The conceptual view (5) of Figure 6 shows one example of a cell aggregate in which brain and spinal cord tissue (cerebrum, etc.) (gray portion of the conceptual view (5) of Figure 6) is present as the second epithelial tissue. As shown in the conceptual view (4) of Figure 6, non-target tissue may be contained inside the cell aggregate containing a transplant neural retina. This case does not apply to the definition "having the continuity of the slope of a tangent line to a surface different from the continuity of the slope of a tangent line to the surface of the first epithelial tissue", and therefore does not apply to the second epithelial tissue. It is preferable that the transplant neural retina and the sample for quality evaluation should be selected from a cell aggregate that does not contain non-target tissue in the inside.

<Sampling step>

In the case of dissecting a transplant neural retina from a cell aggregate, it is desirable to use containing a marker-positive cell of a photoreceptor cell or a photoreceptor progenitor cell as an index. In the case of dissecting a transplant neural retina from a cell aggregate, it is desirable to use containing a marker-positive cell of a retinal progenitor cell or a neural retinal progenitor cell as an index. It is also desirable to use not containing a marker-positive cell of a non-target cell as an index.

For obtaining a transplant neural retina that contains a marker-positive cell of a photoreceptor cell or a photoreceptor progenitor cell and contains a marker-positive cell of a non-target cell below a certain level (or is negative to a non-target cell marker), it is desirable to evaluate the quality of the transplant neural retina in advance.

The quality of the transplant neural retina can be evaluated, for example, by sampling a part or the whole of a cell aggregate containing a neural retina having an epithelial structure derived from a pluripotent stem cell as a sample for quality evaluation (hereinafter, referred to as "sampling step"), and detecting a marker expressed in the obtained sample by a method known to those skilled in the art. The sampling of a part of the cell aggregate as the sample for quality evaluation means selecting some (one or more) cell aggregates, or all cell aggregates from among a plurality of cell aggregates, and isolating (e.g., dissecting) a portion of the selected cell aggregates as the sample for evaluation using tweezers, scissors and/or a knife, etc. The sampling of the whole of the cell aggregate as the sample for quality evaluation means selecting some (one or more) cell aggregates from among a plurality of cell aggregates, and separately picking up the whole of the selected one or more cell aggregates as the sample for quality evaluation. In the case of selecting one or more cell aggregates from among a plurality of cell aggregate, random sampling is preferable. In the specification, the cell aggregate in the case of sampling a part of the cell aggregate as the sample for quality evaluation is referred to as "cell aggregate containing the sample for quality evaluation", and the cell aggregate in the case of sampling the whole of the cell aggregate as the sample for quality evaluation is referred to as "cell aggregate of the sample for quality evaluation".

In an embodiment, the sample for quality evaluation is a part of a cell aggregate containing a neural retina having an epithelial structure derived from a pluripotent stem cell. By sampling a part of the cell aggregate as the sample for quality evaluation, there is an advantage that a neural retina contained in the remaining portion can be used in transplantation without completely destroying the cell aggregate. Specifically, provided that the sample for quality evaluation which is a part of the cell aggregate is determined as being accepted by a determination step mentioned later, a neural retina having an epithelial structure in the cell aggregate containing the sample for quality evaluation is regarded as being applicable as the transplant neural retina and can be used in transplantation.

In the cell aggregate, it can be determined that a site having a continuous epithelium structure where an outer neuroblastic layer and an inner neuroblastic layer appear to be divided as two layers is the neural retina. On the other hand, eyeball-related tissue as the second epithelial tissue, particularly, retinal pigment epithelial cells, assume black color visually or under a microscope and therefore, can readily be distinguished from the neural retina by those skilled in the art. Also, brain and spinal cord tissue as the second epithelial tissue, visually or under a microscope, cannot be confirmed to have a continuous epithelium structure, which is a morphological feature, on the surface of the cell aggregate, cannot be confirmed to have morphological features intrinsic to the neural retina, and/or appears to have a dull color, and thus, can readily be distinguished from the neural retina by those skilled in the art by focusing thereon. Thus, those skilled in the art can isolate the transplant neural retina and the sample for quality evaluation from the first epithelial tissue containing the neural retina even in a cell aggregate containing the second epithelial tissue.

As mentioned above, in an embodiment, the sample for quality evaluation is set and sampled depending on a predetermined positional relationship with the transplant neural retina or a candidate of the transplant neural retina. In other words, a region to be dissected as the sample for quality evaluation can be fixed by the setting of the transplant neural retina or its candidate. In this context, in an embodiment, the transplant neural retina (also referred to as a graft or a cap) and its candidate can be defined by the position in the cell aggregate mentioned above (e.g., being the center and/or its neighborhood of the epithelial tissue (continuous epithelial tissue), and in the case of having the second epithelial tissue, being a region on the first epithelial tissue most distant from the second epithelial tissue), and a size described in (Transplant neural retina sheet) mentioned later, etc. Thus, those skilled in the art can set a neural retina having these features as the transplant neural retina or its candidate.

In the case of sampling the transplant neural retina and the sample for quality evaluation from the same cell aggregate, the sample for quality evaluation (also referred to as a ring) can be set as a region continuous or adjacent at least partially to the transplant neural retina set as mentioned above, and a region as narrow as possible within a range that permits quality evaluation, by those skilled in the art. In the case of sampling a part of one or more cell aggregates among the cell aggregates of the same lot as the sample for quality evaluation, the sample for quality evaluation can be sampled as the transplant neural retina mentioned above or its candidate portion in the cell aggregates by those skilled in the art. In this case, a size to be dissected as the sample for quality evaluation may be a size described in (Transplant neural retina sheet) mentioned later, or may be smaller. Thus, the sample for quality evaluation can be set and sampled depending on the positional relationship with the transplant neural retina or its candidate and the size.

<Detection step>

The method for evaluating the quality of a transplant neural retina according to the present invention comprises detecting the expression of a neural retina-related cell-related gene and a non-neural retina-related cell-related gene (non-target cell-related gene) in the sample for quality evaluation (detection step). It is preferable for the detection step to quantitatively detect the expression levels of the genes. The non-target cell-related gene comprises one or more genes selected from the group consisting of brain and spinal cord tissue marker gene and eyeball-related tissue marker gene.

(Neural retina-related cell-related gene)

The neural retina-related cell-related gene (target cell-related gene) means a gene expressed by neural retina-related cells. As the neural retina-related cell-related gene, a gene highly expressed in photoreceptor cells (rod photoreceptor cell, cone photoreceptor cell), horizontal cells, amacrine cells, intermediate neuronal cells, retinal ganglion cells (ganglion cell), bipolar cells (rod bipolar cell, cone bipolar cell), Muller glial cells, or progenitor cells of these cells, neural retinal progenitor cells, or the like as compared with non-target cells is preferable. Examples of the neural retina-related cell-related gene include the neural retina-related cell markers described above, and *RAX, Chx10, SIX3, SIX6, RCVPN, CRX, NRL* and *NESTIN* are preferable. GenBank IDs of the neural retina-related cell markers are shown in Table 1 below.

[Table 1]

| Gene name | GenBank ID |
|-----------|------------|
| *RAX* | **NM**_013435.2 |
| *Chx10* | **NM**_182894.2 |
| *SIX3* | **NM**_005413.4 |
| *SIX6* | **NM**_007374.2 |
| *RCVRN* | **NM**_002903.2 |
| *CRX* | **NM**_000554.6 |
| *NRL* | **NM**_006177.4 |
| *NESTIN* | **NM**_006617.2 |

The neural retina-related cell-related gene is preferably the gene described in Table 1, though not limited thereto. Other examples of the neural retina-related cell-related gene include *Rax2, Vsx1, Blimp1, RXRG, S-opsin, MIL-opsin, rhodopsin, Brn3, and L7.*

(Non-neural retina-related cell-related gene)

Identification can be performed by detecting a gene (hereinafter, referred to as non-neural retina-related cell-related gene or non-target cell-related gene) expressed in a non-target cell induced as a by-product in the process of producing the cell aggregate containing a neural retina as a medicine raw material, or a cell or tissue having the possibility that the non-target cell is produced as a by-product.

In an embodiment, examples of the non-neural retina-related cell-related gene (non-target cell-related gene) include brain and spinal cord tissue marker gene and eyeball-related tissue marker gene. In an embodiment, as the non-neural retina-related cell-related gene, undifferentiated iPS cell marker gene may be contained.

In an embodiment, the brain and spinal cord tissue marker gene may be one or more genes selected from the group consisting of telencephalon marker gene, diencephalon/midbrain marker gene and spinal cord marker gene. The diencephalon/midbrain marker gene may be one or more genes selected from the group consisting of diencephalon marker gene, midbrain marker gene, and hypothalamus marker gene regarding the hypothalamus which is a part of the diencephalon.

In an embodiment, the eyeball-related tissue marker gene may be one or more genes selected from the group consisting of optic stalk marker gene, ciliary body marker gene, lens marker gene and retinal pigment epithelium marker gene.

The telencephalon marker gene means a gene expressed in the telencephalon. The telencephalon marker gene may comprise one or more genes selected from the group consisting of *FoxG1* (also called *Bf1*), *Emx2, Dlx2, Dlx1* and *Dlx5.* GenBank IDs of the telencephalon marker genes are shown in Table 2 below.

[Table 2]

| Gene name | GenBank ID |
|-----------|------------|
| *FOXG1* | **NM**_005249.4 |

(continued)

| Gene name | GenBank ID |
|---|---|
| Emx2 | **NM**_004098.4<br>**NM**_001165924.1 |
| DLX2 | **NM**_004405.4 |
| DLX1 | **NM**_178120.5<br>**NM**_001038493.1 |
| DLX5 | NM_005221.6<br>**XM**_005250185.3<br>**XM**_017011803.1 |

The telencephalon marker gene is preferably the gene described in Table 2, though not limited thereto. Other examples of the telencephalon marker gene include *Emx1, LHX2, LHX6, LHX7,* and *Gsh2.*

The diencephalon/midbrain marker gene means a gene expressed in the diencephalon and/or the midbrain. The diencephalon/midbrain marker gene may comprise one or more genes selected from the group consisting of *OTX1, OTX2* and *DMBX1.* GenBank IDs of the diencephalon/midbrain marker genes are shown in Table 3 below. The diencephalon/midbrain marker gene may comprise a hypothalamus marker mentioned later regarding the hypothalamus which is a region of the diencephalon. In other words, the diencephalon/midbrain marker gene may comprise one or more genes selected from the group consisting of *OTX1, OTX2, OTX2, DMBX1, Rx, Nkx2.1, OTP, FGFR2, EFNA5* and *GAD1.*

[Table 3]

| Gene name | GenBank ID |
|---|---|
| OTX1 | **NM**_001199770.1<br>**NM**_014562.4 |
| OTX2 | **NM**_001270523.1<br>**NM**_001270524.1<br>**NM**_001270525.1<br>**NM**_021728.3<br>**NM**_172337.2 |
| DMBX1 | **NM**_172225.1<br>**NM**_147192.2<br>**XM**_011540668.2<br>**XM**_017000289.1 |

The hypothalamus marker gene means a gene expressed in the hypothalamus. The hypothalamus marker gene may comprise one or more genes selected from the group consisting of *Rx, Nkx2.1, Dmbx1, OTP, gad1, FGFR2* and *EFNAS.* GenBank IDs of the hypothalamus marker gene are shown in Table 4 below.

[Table 4]

| Gene name | GenBank ID |
|---|---|
| Rx | NM_013435.2 |
| Nkx2.1 | NM_003317.3, NM_001079668.2 |
| OTP | NM_032109.2 |
| gad1 | NM_000817.3, NM_013445.3<br>XM_005246444.3, XM_011510922.1<br>XM_017003756.1, XM_017003758.2<br>XM_017003757.2, XM_024452783.1 |

(continued)

| Gene name | GenBank ID |
|-----------|-----------|
| FGFR2 | NM_022970.3, NM_000141.4<br>NM_023029.2, NM_001144913.1<br>NM_001144914.1, NM_001144915.1<br>NM_001144916.1, NM_001144917.1<br>NM_001144918.1, NM_001144919.1<br>NM_001320654.1, NM_001320658.1<br>NR_073009.1, XM_006717708.3<br>XM_006717710.4, XM_017015920.2<br>XM_017015921.2, XM_017015924.2<br>XM_017015925.2, XM_024447888.1<br>XM_024447887.1, XM_024447890.1<br>XM_024447889.1, XM_024447892.1<br>XM_024447891.1 |
| EFNA5 | NM_001962.3, XM_006714565.3<br>XM_011543250.3, XM_011543251.2<br>XM_017009205.1 |

The spinal cord marker gene means a gene expressed in the spinal cord. The spinal cord marker gene may comprise one or more genes selected from the group consisting of *HoxB2, HoxA5, HOXC5, HOXD1, HOXD3* and *HOXD4*. GenBank IDs of the spinal cord marker gene are shown in Table 5 below.

[Table 5]

| Gene name | GenBank ID |
|-----------|-----------|
| HOXB2 | NM_002145.3<br>XM_005257275.4 |
| HOXA5 | NM_019102.4 |
| HOXC5 | NM_018953.3<br>NR_003084.2 |
| HOXD1 | NM_024501.3 |
| HOXD3 | NM_006898.4<br>XM_005246509.4<br>XM_005246511.4<br>XM_005246513.5<br>XM_011511065.3<br>XM_011511066.3 |
| HOXD4 | NM_014621.3<br>XM_005246514.4 |

The spinal cord marker gene is preferably the gene described in Table 5, though not limited thereto. Other examples of the spinal cord marker gene include a gene group forming the Hox cluster.

Meanwhile, in an embodiment, in the case of using retinoids (examples thereof include retinoic acid, retinal, retinol, all-trans-retinoic acid, and 11-cis-retinoic acid) in a production step, the expression of HOX gene (e.g., *HOXC5, HOXA5* and *HOXB2*) may be found even if a good product of retinal tissue is produced. It is considered that the expression of the HOX gene is regulated by retinoic acid signals, and the HOX gene expression increases to an extent that does not influence differentiation into retinal tissue. This effect of the retinoic acid signals is considered to be ascribable to the promotion of posterior shift along the anteroposterior axis. Thus, in the case of using retinoids in a production step (particularly, in the case of using retinoids at the time or later when differentiation into the retina has started), the HOX gene (e.g., *HOXC5, HOXA5* and *HOXB2*) can be excluded from subject genes of quality evaluation, or the quality of a transplant neural retina can be determined as being good even if the expression of these genes is found.

The optic stalk marker gene means a gene expressed in the optic stalk. The optic stalk marker gene may comprise one or more genes selected from the group consisting of *GREM1, GPR17, ACVR1C, CDH6, Pax2, Pax8, GAD2* and *SEMA5A.* GenBank IDs of the optic stalk marker genes are shown in Table 6 below.

[Table 6]

| Gene name | GenBank ID |
|---|---|
| *GREM1* | NM_013372.7, NM_001191322.1 NM_001191323.1, NM_001368719.1 XM_017022077.1 |
| GPR17 | NM_001161417.1, NM_005291.2 NM_001161415.1, NM_001161416.1 XM_017003833.2 |
| *ACVR1C* | NM_145259.3, NM_001111031.1 NM_001111032.1, NM_001111033.1 |
| CDH6 | NM_004932.4, NM_001362435.1 XM_011513921.3, XM_017008910.2 XR _ 001741972.2 |
| *Pax2* | NM_000278.4, NM_003987.4 NM_003988.4, NM_003989.4 NM_003990.4, NM_001304569.1 |
| *Pax8* | NM_003466.4, NM_013952.3 NM_013953.3, NM_013992.3 |
| GAD2 | NM_001134366.2, NM_000818.2 |
| *SEMA5A* | NM_003966.3, XM_006714506.3 XM_006714507.3, XM_011514155.2 XM_011514156.2, XM_011514157.2 XM_011514158.2, XM_011514159.2 XM_017010016.2 |

The lens marker gene means a gene expressed in the lens. The lens marker gene may comprise one or more genes selected from the group consisting of *CRYAA* and *CRYBA1.* GenBank IDs of the lens marker genes are shown in Table 7 below.

[Table 7]

| Gene name | GenBank ID |
|---|---|
| *CRYAA* | NM_000394.3 NM_001363766.1 |
| *CRYBA1* | NM_005208.4 XM_017024198.1 |

The ciliary body marker gene means a gene expressed in the ciliary body and/or the ciliary marginal zone. The ciliary body marker gene may comprise one or more genes selected from the group consisting of *Zic1, MAL, HNF1beta, FoxQ1, CLDN2, CLDN1, GPR177, AQP1* and *AQP4.* GenBank IDs of the ciliary body marker genes are shown in Table 8 below.

[Table 8]

| Gene name | GenBank ID |
|---|---|
| *Zic1* | NM_003412.4 |

(continued)

| Gene name | GenBank ID |
|---|---|
| MAL | NM_002371.4, NM_022438.2 NM_022439.2, NM_022440.2 |
| HNF1beta | NM_000458.4, NM_001165923.3 NM_001304286.1, XM_011525160.1 XM_011525161.1, XM_011525162.2 XM_011525163.2, XM_011525164.1 |
| FoxQ1 | NM_033260.4 |
| CLDN2 | NM_001171092.1, NM_020384.3 NM_001171095.1 |
| CLDN1 | NM_021101.5 |
| GPR177 | NM_024911.7, NM_001002292.3 NM_001193334.1, XM_011542191.2 XM_011542192.3, XM_017002390.2 |
| AQP1 | NM_198098.3, NM_001329872.1 |
| AQP4 | NM_001650.7, NM_004028.4 NM_001317384.2, NM_001317387.2 NM_001364286.1, NM_001364287.1 NM_001364289.1, XM_011525942.3 |

The retinal pigment epithelium marker gene means a gene expressed in retinal pigment epithelial cells. Examples of the retinal pigment epithelium marker gene include the retinal pigment epithelium markers described above, and may comprise one or more genes selected from the group consisting of *MITF, TTR* and *BEST1*. GenBank IDs of the retinal pigment epithelium marker genes are shown in Table 9 below.

[Table 9]

| Gene name | GenBank ID |
|---|---|
| MITF | NM_000248.3, NM_006722.2 NM_198158.2, NM_198159.2 NM_198177.2, NM_198178.2 NM_001184967.1, NM_001184968.1 NM_001354604.1, NM_001354605.1 NM_001354606.1, NM_001354607.1 NM_001354608.1 |
| BEST1 | NM_004183.4, NM_001139443.1 NM_001300786.1, NM_001300787.1 NM_001363591.1, NM_001363592.1 NM_001363593.1, NR_134580.1 XM_005274210.4, XM_005274215.4 XM_005274216.4, XM_005274219.4 XM_005274221.4, XM_011545229.3 XM_011545230.3, XM_011545233.3 XM_017018230.2, XR_001747952.2 XR_001747953.2, XR_001747954.2 |
| TTR | NM_000371.3 |

In an embodiment, the non-target cell-related gene may further comprise undifferentiated pluripotent stem cell marker gene.

The undifferentiated pluripotent stem cell marker gene may comprise one or more genes selected from the group consisting of *Oct3/4, Nanog* and *lin28.* Preferably, the undifferentiated pluripotent stem cell marker gene is one or more genes selected from the group consisting of *Oct3/4, Nanog* and *lin28.* GenBank IDs of the undifferentiated pluripotent stem cell marker genes are shown in Table 10 below.

[Table 10]

| Gene name | GenBank ID |
|---|---|
| *Oct3/4 (POU5F1)* | NM_002701.6, NM_203289.5 NM_001173531.2, NM_001285986.1 NM_001285987.1 |
| *Nanog* | NM_024865.4, NM_001297698.1 |
| *lin28* | NM_024674.6, XM_011542148.2 |
| *SOX2* | NM_003106.4 |
| *KLF4* | NM_001314052.1, NM_004235.6 |
| *c-Myc* | NM_001354870.1, NM_002467.6 |
| *Glis1* | NM_001367484.1, NM_147193.2 XM_017000409.1, XM_017000411.1 XM_017000408.1, XM_017000410.1 XM_017000412.1 |
| *Sall4* | NM_001318031.1, NM_020436.5 XM_011528921.2, XM_011528922.2 XM_005260467.4 |
| *Esrrb* | NM_004452.3, XM_011536553.2 XM_024449508.1, XM_011536547.2 XM_011536554.2, XM_011536550.2 XM_024449509.1, XM_017021085.1 |

(Detection approach)

In an embodiment, although the detection of the expression of the neural retina-related cell-related gene and the non-neural retina-related non-target cell-related gene is not particularly limited, examples thereof include approaches such as Western blotting, immunostaining, flow cytometry analysis/flow cytometers (FACS(R) manufactured by Becton, Dickinson and Company), etc.), Northern blotting, electrophoresis, PCR (preferably, quantitative PCR (qPCR) and/or real-time PCR), gene chip analysis, and next-generation sequencers. Among them, quantitative PCR is useful from the viewpoint of quantitativeness, detection sensitivity, stability of results and rapidness. Furthermore, by applying an apparatus that is used for performing single-cell quantitative PCR (e.g., Biomark HD (manufactured by Fluidigm Corp.)) to usual quantitative PCR, it is possible to evaluate a plurality of samples for quality evaluation in a short time.

In an embodiment, the respective expression levels of the neural retina-related cell-related gene and the non-neural retina-related cell-related gene in two or more samples for quality evaluation may be simultaneously detected by quantitative PCR. The quantitative PCR may be performed by, for example, a method comprising the following steps (1) to (5). Specific methods are known to those skilled in the art.

(1) providing a flow channel plate having one sample well group consisting of 8 or more and 800 or less independent sample wells, one or more primer well groups consisting of 8 or more and 800 or less independent primer wells, and flow channels connecting the independent sample wells in the sample well group with the independent primer wells in each primer well group, solutions containing nucleic acids obtained from the two or more of the samples for quality evaluation (sample solutions), and a solution containing one or a plurality of primers specific for each of one or more of the neural retina-related cell-related genes or the non-neural retina-related cell-related genes (primer solution);

(2) adding the sample solutions at one sample solution/one sample well for each of the samples for quality evaluation to the sample well group;

(3) adding the primer solution to one or more primer wells in the one or more primer well groups so as to be different primer well groups;

(4) separately mixing the primers with the nucleic acids via the flow channels; and

(5) performing quantitative PCR using the mixture obtained in (4).

Flow cytometry analysis using a flow cytometer capable of detecting the ratios of expressing cells is also useful. In recent years, improvement in detection rate has advanced, and a flow cytometer capable of evaluating multiple samples with high-throughput properties (FACS(R), etc.) is also available. Thus, for the detection of the expression of the neural retina-related cell-related gene and the non-neural retina-related non-target cell-related gene, use of a high-throughput flow cytometer is also useful. It is possible for such a high-throughput flow cytometer to use a commercially available product (e.g., MACSQuant(R) Analyzers: manufactured by Miltenyi Biotec).

<Determination step>

It can be determined that, when the expression of the neural retina-related cell-related gene is found and the expression of the non-neural retina-related cell-related gene is not found, the transplant neural retina sheet dissected from the cell aggregate is applicable to transplantation, i.e., is suitable for transplantation (determination step).

"The expression of the neural retina-related cell-related gene is found" means that, for a detection method for gene expression, the expression of the neural retina-related cell-related gene at a level substantially detectable by the detection method (e.g., detection lower limit value or more) is found. Also, "the expression of the non-neural retina-related cell-related gene is not found" means that, for a detection method for gene expression, the expression of the non-neural retina-related cell-related gene cannot be substantially detected by the detection method (e.g., less than detection lower limit value). The substantial detectability means that the gene is detected beyond an extent that cannot regard the gene as substantially functioning. Those skilled in the art are appropriately capable of setting it according to the genes and the detection method. For example, in the case of a detection method for gene expression with quantitativeness, it can be determined that the range of more than 0% to 10% or less or more than 0% to 5% or less with reference to the detection lower limit value of the gene expression is not the substantially detectable level (i.e., the expression of the gene cannot be detected).

In an embodiment, it is preferable to determine being applicable as the transplant neural retina when the following reference 1 and reference 2 are satisfied in the quantitative PCR:

reference 1: the difference between the threshold cycle (Ct) value of the neural retina-related cell-related gene and the Ct value of an internal standard gene ($\Delta$Ct value) is 10 or less, and

reference 2: the difference between the Ct value of the non-neural retina-related cell-related gene and the Ct value of the internal standard gene ($\Delta$Ct value) is 5 or more.

The threshold cycle (Ct) value means the number of cycles that reaches a predetermined amount of an amplification product in a region where the amplification of a gene by PCR occurs exponentially. The Ct value has an inverse correlation with the initial amount of the gene and as such, is used in the calculation of the initial copy number of the gene. In an embodiment, the "$2^{Ct}$ value (Ct value power of 2)" is inversely proportional to the initial amount of the gene and as such, is used in the calculation of the initial copy number of the gene. Specifically, a sample containing a 2-fold initial amount of a gene has a Ct value more rapid by one cycle than that of a sample containing the gene at only half the copy number before amplification. The predetermined amount of an amplification product can fall within the region where the amplification of a gene by PCR occurs exponentially, and can be set by those skilled in the art.

The internal standard gene means a gene whose difference in expression level is small among samples. As the internal standard gene, the one known to those skilled in the art can be appropriately used, and examples thereof include 18S ribosomal RNA, $\beta$ actin, HPRT, $\alpha$ tubulin, transferrin receptor, ubiquitin, and GAPDH, with GAPDH being preferable.

The Ct value is inversely correlated with the initial amount of a gene and therefore depends on the expression level of the gene within cells. Specifically, when the concentration of a nucleic acid-containing solution is constant, the Ct value differs depending on the internal standard gene used and the difference between the Ct value of a predetermined gene and the Ct value of the internal standard gene ($\Delta$Ct value) is influenced by the internal standard gene used. In the specification, the $\Delta$Ct value is described with reference to a value with GAPDH used as the internal standard gene, unless otherwise specified.

In the case of using an internal standard gene other than GAPDH as the internal standard gene, the $\Delta$Ct values of the reference 1 and the reference 2 can be corrected by comparing the expression levels of GAPDH and the internal standard gene other than GAPDH.

In an embodiment, in the case of using $\beta$ actin as the internal standard gene, the Ct value of GAPDH is lower by about 1 than the Ct value of β actin, i.e., the absolute amount of GAPDH RNA is about twice the absolute amount of β actin RNA, as to GAPDH and β actin in the production method of the present application. Therefore,

reference 1: the difference between the threshold cycle (Ct) value of the neural retina-related cell-related gene and the Ct value of an internal standard gene (ΔCt value) is 9 or less, and
reference 2: the difference between the Ct value of the non-neural retina-related cell-related gene and the Ct value of the internal standard gene (ΔCt value) is 4 or more, can hold.

In an embodiment, in the case of using HPRT as the internal standard gene, the Ct value of GAPDH is lower by about 7 than that of HPRT, i.e., the absolute amount of GAPDH RNA is about $2^7$ (128 times) of the absolute amount of HPRT RNA, as to GAPDH and HPRT in the production method of the present application. Therefore,

reference 1: the difference between the threshold cycle (Ct) value of the neural retina-related cell-related gene and the Ct value of an internal standard gene (ΔCt value) is 3 or less, and
reference 2: the difference between the Ct value of the non-neural retina-related cell-related gene and the Ct value of the internal standard gene (ΔCt value) is -2 or more, can hold.

The neural retina-related cell-related gene can be the gene mentioned above. As the neural retina-related cell-related gene, a plurality of genes are present. Specifically, even in the case of sampling it from the same neural retina-related cells, the Ct value of the reference 1 may differ depending on the type of the neural retina-related cell-related gene. Those skilled in the art can set a ΔCt value from which the expression of the neural retina-related cell-related gene can be determined on a gene basis, from known information such as the expression site or expression level of the neural retina-related cell-related gene.

For example, as for the Chx10 gene, when GAPDH is used as the internal standard, the ΔCt value may be 20 or less, preferably 15 or less, more preferably 10 or less.

For example, as for the recoverin gene, when GAPDH is used as the internal standard, the ΔCt value may be 16 or less, preferably 11 or less, more preferably 6 or less.

In general, the difference between the Ct value of the neural retina-related cell-related gene and the Ct value of the internal standard gene (e.g., GAPDH) (ΔCt value) may be, for example, 25 or less, 20 or less, 15 or less or 10 or less. The difference between the Ct value of the neural retina-related cell-related gene and the Ct value of the internal standard gene may be, for example, -10 or more, -5 or more, 0 or more or 5 or more.

The non-neural retina-related cell-related gene can be the gene mentioned above. As the non-neural retina-related cell-related gene, a plurality of genes are present. Specifically, even in the case of sampling it from the same non-retinal cells, the Ct value differs depending on the non-neural retina-related cell-related gene. Those skilled in the art can set a ΔCt value from which the expression of the non-neural retina-related cell-related gene can be determined on a gene basis, from known information such as the expression site or expression level of the non-neural retina-related cell-related gene. For example, as for the PAX2 gene, when GAPDH is used as the internal standard, the ΔCt value may be 5 or more. As for the HOXB2 gene, when GAPDH is used as the internal standard, the ΔCt value may be 5 or more. In general, the difference between the Ct value of the non-neural retina-related cell-related gene and the Ct value of the internal standard gene may be 30 or less, 25 or less or 20 or less. Also, the difference between the Ct value of the non-neural retina-related cell-related gene and the Ct value of the internal standard gene may be, for example, 0 or more, 3 or more or 5 or more.

In an embodiment, the neural retina according to the present invention is a transplant neural retina sheet having the following features:

(1) being derived from a pluripotent stem cell,
(2) having a three-dimensional structure,
(3) comprising a neural retinal layer having a plurality of layer structures including a photoreceptor layer and an inner layer,
(4) the photoreceptor layer comprising one or more cells selected from the group consisting of a photoreceptor progenitor cell and a photoreceptor cell,
(5) the inner layer comprising one or more cells selected from the group consisting of a retinal progenitor cell, a ganglion cell, an amacrine cell and a bipolar cell,
(6) the surface of the neural retinal layer having an apical surface,
(7) the inner layer being present inside the photoreceptor layer present along the apical surface,
(8) the area of the neural retinal layer being 50% or more with respect to the total area of the surface of the transplant neural retina sheet,
(9) the area of a continuous epithelium structure being 80% or more with respect to the total area of the apical surface of the neural retinal layer, and

(10) the expression of neural retina-related cell-related gene being found and the expression of non-neural retina-related cell-related gene being not found in the transplant neural retina sheet, and the non-neural retina-related cell-related gene comprising one or more genes selected from the group consisting of brain and spinal cord tissue marker gene and eyeball-related tissue marker gene.

In an embodiment, the transplant neural retina sheet is preferably a sheet-shaped cell aggregate dissected as a part containing a neural retina from a sphere-like cell aggregate derived from a pluripotent stem cell. A specific preparation method is as described in, for example, International Application No. PCT/JP2020/011254.

In an embodiment, the neural retina (3) comprises a neural retinal layer having a plurality of layer structures including a photoreceptor layer and an inner layer. As described in (6) and (7), although the photoreceptor layer is present outside (surface) the neural retina, an ectopic photoreceptor layer may be present in the inner layer.

In an embodiment, in the neural retina, (5) the inner layer comprises one or more cells selected from the group consisting of a retinal progenitor cell, a ganglion cell, an amacrine cell and a bipolar cell, but may comprise one or more cells selected from the group consisting of an ectopic photoreceptor progenitor cell and photoreceptor cell. In an embodiment, a neural retina in which the content of a ganglion cell, an amacrine cell and a horizontal cell is 30% or less of the total number of cells, a neural retina in which the content of a ganglion cell, an amacrine cell, a horizontal cell and a bipolar cell is 30% or less of the total number of cells, and/or a neural retina in which the content of a bipolar cell is 10% or less of the total number of cells is also provided.

In an embodiment, in the neural retina, (8) the area of the neural retinal layer is 40% or more, preferably 50% or more, more preferably 60% or more, with respect to the total area of the surface of the neural retina. In the neural retina, (9) the area of a continuous epithelium structure is 60% or more, preferably 70% or more, more preferably 80% or more, with respect to the total area of the apical surface of the neural retinal layer.

The neural retina-related cell-related gene and the non-neural retina-related cell-related gene (brain and spinal cord tissue marker gene and eyeball-related tissue marker gene) are the genes mentioned above.

(10) The expression of neural retina-related cell-related gene being found and the expression of non-neural retina-related cell-related gene being not found in the transplant neural retina sheet can be revealed by isolating a part of the transplant neural retina sheet, and detecting the expression of the genes. For a transplant neural retina sheet isolated from a cell aggregate in which the expression of neural retina-related cell-related gene is substantially found and the expression of non-neural retina-related cell-related gene is not substantially found, the detection of gene expression in the transplant neural retina sheet itself is unnecessary. The expression of the gene being substantially found or the expression being not found is determined depending on whether or not to be a level substantially detectable by the detection method.

The neural retina-related cell-related gene in the transplant neural retina sheet may be, for example, one or more selected from the group consisting of *Rx, Chx10, Pax6* and *Crx.* The ratio of cells expressing the neural retina-related cell-related gene (positive cell) to the total number of cells differs depending on the stage of differentiation into the neural retina.

In an embodiment, the ratio of a Rx-positive cell to the total number of cells in the transplant neural retina sheet may be 30% or more, 40% or more, 50% or more, or 60% or more. In an embodiment, the ratio of a Chx10-positive cell or a Pax6-positive cell to the total number of cells in the transplant neural retina sheet may be 10% or more, 20% or more, 30% or more, 40% or more, or 50% or more. In an embodiment, the ratio of a Crx-positive cell to the total number of cells in the transplant neural retina sheet may be 10% or more, 20% or more, 30% or more, 40% or more, or 50% or more.

In an embodiment, the ratio of the Rx-positive cell to the total number of cells in the transplant neural retina sheet may be 30% or more and 80% or less, 40% or more and 70% or less, 45% or more and 60% or less, or 50% or more and 60% or less. In an embodiment, the ratio of the Chx10-positive cell or the Pax6-positive cell to the total number of cells in the transplant neural retina sheet may be 10% or more and 80% or less, 20% or more and 70% or less, 30% or more and 60% or less, or 40% or more and 50% or less. In an embodiment, the ratio of the Crx-positive cell to the total number of cells in the transplant neural retina sheet is 10% or more and 70% or less, 10% or more and 60% or less, 20% or more and 60% or less, 30% or more and 60% or less, 40% or more and 60% or less, or 50% or more and 60% or less.

In an embodiment, (1) the ratio of a Chx10-positive and Pax6-positive cell (neural retinal progenitor cell) may be 10% or more and 50% or less or 10% or more and 30% or less, (2) the ratio of a Chx10-positive and Pax6-negative cell (progenitor cell biased toward a bipolar cell) may be 10% or more and 25% or less or 15% or more and 25% or less, and (3) the ratio of a Chx10-negative and Pax6-positive cell (ganglion cell and amacrine cell) may be 10% or more and 25% or less or 10% or more and 20% or less, to the total number of cells in the transplant neural retina sheet.

In another embodiment, (1) the ratio of the Chx10-positive and Pax6-positive cell (neural retinal progenitor cell) may be 20% or more and 40% or less, (2) the ratio of the Chx10-positive and Pax6-negative cell (progenitor cell biased toward a bipolar cell) may be 5% or more and 20% or less, and (3) the ratio of the Chx10-negative and Pax6-positive cell (ganglion cell and amacrine cell) may be 5% or more and 20% or less or 5% or more and 15% or less, to the total number of cells in the transplant neural retina sheet.

In an embodiment, the transplant neural retina sheet according to the present invention is a transplant neural retina determined as being applicable as the transplant neural retina by the method for evaluating the quality of a transplant neural retina, and may be an isolated sheet-shaped transplant neural retina.

In an embodiment, the transplant neural retina sheet according to the present invention has been isolated from a cell aggregate containing a neural retina, and may be a transplant neural retina sheet containing a region of the center and/or its neighborhood of continuous epithelial tissue in the cell aggregate.

In an embodiment, the neural retina is a transplant neural retina sheet, and the transplant neural retina sheet

(1) has been isolated from a cell aggregate containing a neural retinal layer,
(2) contains a region of the center and/or its neighborhood of continuous epithelial tissue in the cell aggregate, and
(3) is from 600 $\mu$m to 2500 $\mu$m in major axis, from 200 $\mu$m to 1500 $\mu$m in minor axis, and from 100 $\mu$m to 1000 $\mu$m in thickness.

In an embodiment, the transplant neural retina sheet according to the present invention has been isolated from a cell aggregate containing a neural retina, a retinal pigment epithelial cell and a ciliary marginal zone structure, and may be a transplant neural retina sheet that has the continuity of the slope of a tangent line to a surface different from the continuity of the slope of a tangent line to the surface of a neural retinal epithelial structure, and contains a region on an epithelial structure most distant from a portion containing the retinal pigment epithelial cell.

In an embodiment, the transplant neural retina sheet according to the present invention has been isolated from a cell aggregate containing a neural retina and brain and spinal cord tissue, and may be a transplant neural retina sheet that has the continuity of the slope of a tangent line to a surface different from the continuity of the slope of a tangent line to the surface of a neural retinal epithelial structure, and contains a region on an epithelial structure most distant from a portion containing the brain and spinal cord tissue.

In an embodiment, the transplant neural retina sheet according to the present invention has been isolated from two or more cell aggregates each containing neural retinal epithelium, and may be a transplant neural retina sheet containing the central part of morphologically favorable and/or large-size neural retinal epithelium suitable for isolation thereamong.

In an embodiment, the neural retina is a transplant neural retina sheet, and the transplant neural retina sheet

(1) has been isolated from a cell aggregate containing at least first epithelial tissue and second epithelial tissue, wherein
the first epithelial tissue contains a human neural retina, and the second epithelial tissue has the continuity of the slope of a tangent line to a surface different from the continuity of the slope of a tangent line to the surface of the first epithelial tissue, and contains a non-neural retina-related cell,
(2) contains a region on the first epithelial tissue most distant from the second epithelial tissue, and
(3) is from 600 $\mu$m to 2500 $\mu$m in major axis, from 200 $\mu$m to 1500 $\mu$m in minor axis, and from 100 $\mu$m to 1000 $\mu$m in thickness, wherein
the second epithelial tissue is a tissue selected from the group consisting of eyeball-related tissue, brain and spinal cord tissue and other tissues different from the neural retina of the first epithelial tissue.

In an embodiment, the major axis of the transplant neural retina sheet according to the present invention may be, for example, from 300 $\mu$m to 3300 $\mu$m and is preferably from 600 $\mu$m to 2500 $\mu$m, more preferably from 1100 $\mu$m to 1700 $\mu$m.

In an embodiment, the minor axis of the transplant neural retina sheet according to the present invention may be, for example, from 100 $\mu$m to 2000 $\mu$m and is preferably from 200 $\mu$m to 1500 $\mu$m, more preferably from 400 $\mu$m to 1100 $\mu$m.

In an embodiment, the height of the transplant neural retina sheet according to the present invention may be, for example, from 50 $\mu$m to 1500 $\mu$m and is preferably from 100 $\mu$m to 1000 $\mu$m, more preferably from 200 $\mu$m to 700 $\mu$m.

In an embodiment, the volume of the transplant neural retina sheet according to the present invention may be, for example, from 0.001 $mm^3$ to 4.0 $mm^3$ and is preferably from 0.01 $mm^3$ to 1.5 $mm^3$, more preferably from 0.07 $mm^3$ to 0.57 $mm^3$.

Methods for measuring the major axis, minor axis and height of the transplant neural retina sheet are not particularly limited, and they can be measured, for example, from an image taken under a microscope. For example, a front image taken with a cut surface turned to an objective lens side, and a side image taken with the cut surface inclined so as to be perpendicular to an objective lens are taken under a stereo microscope as to the transplant neural retina sheet dissected from a cell aggregate, and they can be measured from the taken images. In this context, the major axis means the longest line segment among line segments connecting two end points on the sheet cross section in the front image, and the length thereof. The minor axis means the longest line segment among line segments connecting two end points on the sheet cross section in the front image and orthogonal to the major axis, and the length thereof. The height means the longest line segment among line segments orthogonal to the sheet cross section and having a point intersecting the sheet cross section and the apex of the retina sheet as end points, and the length thereof. The volume of the sheet means a volume calculated according to the following calculation expression by approximating a graft as being an ellipsoid halved such that the cross section passes through the major axis.

$$\text{Volume} = 2/3 \times \text{Ratio of the circumference of a circle } (\pi) \times$$

$$(\text{Major axis } / 2) \times (\text{Minor axis } / 2) \times \text{Height}$$

(2) Matrix

The matrix (hereinafter, also referred to as matrix gel) according to the present invention can be a matrix that can embed two or more neural retina-containing cell aggregates alive and is capable of becoming gel-like, preferably hydrogel. Examples thereof include fibrin gel, gelatin, collagen, pectin, hyaluronic acid, and alginic acid. It is preferable to be fibrin gel or gelatin. In the specification, the "hydrogel" is a polymer cross-linked into a three-dimensional network structure, the inside of which contains an incorporated liquid such as water. Such hydrogel has biodegradability and the desired melting point and is therefore easy to handle. In addition, a high graft survival rate can be expected. The biodegradability means being degraded by an enzyme or the like *in vivo*, and absorbed or excreted.

The hydrogel according to the present invention can be hydrogel having a melting point in the range of 20°C to 40°C. Specifically, it is characterized by being melted at a body temperature when transplanted. Hereinafter, its physical properties will be described in detail by taking "gelatin" as an example. For hydrogel, such as fibrin gel, other than gelatin, it is preferable to have physical properties similar to those of gelatin mentioned later.

The matrix gel is preferably fibrin gel. The fibrin gel is gel-like fibrin that is obtained by reacting a fibrinogen solution with a thrombin solution. In the specification, a substance having a property of forming gel through reaction is referred to as a matrix precursor, and, for example, thrombin and fibrinogen which react to form fibrin gel are one example of the matrix precursor. The fibrinogen solution can be prepared by dissolving a fibrinogen powder or the like in a dissolving liquid that contains aprotinin and can dissolve fibrinogen, and its concentration is not particularly limited, but is, for example, from 40 to 480 mg/ml, preferably from 80 mg/mL to 320 mg/mL (e.g., 160 mg/mL). When the activity of blood coagulation factor VIII contained in 1 ml of normal human plasma is defined as 1 unit, it can be from 37.5 units/mL to 225 units/mL (e.g., 75 units/mL). The thrombin solution can be prepared by dissolving a thrombin powder or the like in a thrombin-dissolving liquid containing calcium chloride hydrate, and its concentration is not particularly limited, but is, for example, from 125 units/mL to 750 units/mL (e.g., 75 units/mL). The fibrinogen solution and the thrombin solution are contacted or mixed so that they can be reacted with each other. In this respect, it is preferable that the fibrinogen solution and the thrombin solution should be used in the range of 1:1 to 1:9, preferably 1:3 to 1:4, in terms of an activity ratio.

The matrix gel is preferably gelatin. The gelatin is a solubilized form of water-insoluble collagen, for example, by pretreatment with an acid or an alkali and thermal hydrolysis. In the specification, it means gelatin in the state of hydrogel unless otherwise specified, but is also clearly described as "hydrogel of gelatin". Collagen is divided into three random molecules through the disruption of its triple-helical molecular structure by heating and thereby solubilized. Beef bone and bovine hide, porcine skin, pig bone, fish skin, or the like is typically used as a starting material of gelatin. In order to extract gelatin from these collagen starting materials, the pretreatment of the starting material is performed using an inorganic acid such as hydrochloric acid or sulfuric acid, or lime. Depending on the pretreatment conditions of the starting material, the former is also called acid-treated gelatin (or A-type gelatin), and the latter is also called alkali-treated (limed) gelatin (B-type gelatin). The acid-treated gelatin and the alkali-treated gelatin differ in the properties of gelatin. By performing hydrolysis by heat treatment, the molecular weight of gelatin is decreased, and the solubility thereof is improved. Its jelly strength is reduced under this condition. For example, the heat treatment can be performed with warm water (50°C to 80°C) and may be repeated a plurality of times. It is also possible to obtain commercially available gelatin. Examples thereof include gelatin LS-H (Nitta Gelatin Inc., alkali-treated porcine skin gelatin, no-heat-treated gelatin, high jelly strength) and gelatin LS-W (Nitta Gelatin Inc., alkali-treated porcine skin gelatin, heat-treated gelatin, low jelly strength). Alkali-treated (limed) gelatin (B-type gelatin) is preferable, and heat-treated gelatin is preferable.

Collagen is constituted by three polypeptide chains ($\alpha$ chains) having a molecular weight of about 100,000. A collagen molecule is denatured by heat treatment and thereby divided into three $\alpha$ chains ($\alpha$ components). In addition, a dimer of the $\alpha$ chains ($\beta$ component; a molecular weight of about 200,000) and a trimer thereof ($\gamma$ component; a molecular weight of about 300,000) may be produced. Depending on the treatment step of gelatin, some intermolecular or intramolecular bonds of collagen or gelatin are randomly cleaved. Therefore, gelatin is an aggregate of molecules having various molecular weights. Commercially available gelatin usually has a molecular weight distribution of several tens of thousands to several millions, though differing depending on the presence or absence of heat treatment, etc. In an embodiment, a molecular weight distribution in which 50% or more (preferably 60% or more, 70% or more, 80% or more, or 90% or more) is included in the range of about 100,000 to about 300,000 is preferable for gelatin. Also, a preferable average molecular weight of gelatin is in the range of about 50,000 to about 1,000,000, about 100,000 to about 800,000, about 100,000 to about 600,000, about 200,000 to about 500,000, or about 300,000 to about 500,000.

The molecular weight distribution and the average molecular weight can be measured by methods known to those skilled in the art. For example, the molecular weight distribution can be predicted by determining a chromatogram of an aqueous gelatin solution by a gel filtration method using high-performance liquid chromatography. Further, the average molecular weight can be predicted on the basis of pullulan or the like.

The "isoionic point" means pH that is exhibited by a protein or an aqueous solution of an ampholyte in the absence of other ions (except for a hydrogen ion produced by the ionization of water, a hydroxide ion, and an ion of the ampholyte itself of interest). The isoionic point can be measured by a method known to those skilled in the art. As a specific example, the hydrogen ion concentration of a test solution (aqueous gelatin solution) desalted with an ion-exchange resin can be measured using a pH meter. For example, since alkali treatment performed in a gelatin production step deamidates a great majority, the isoionic point is as low as about pH 5. By contrast, the acid-treated gelatin has a low rate of deamidation and exhibits an isoionic point from pH 7 to 9 which is close to collagen. The aqueous gelatin solution is + (positively) charged in a pH region lower than the isoionic point and - (negatively) charged in a higher pH region. In the specification, it is preferable that the hydrogel (gelatin, etc.) should exhibit an isoionic point from about pH 4 to about pH 7, from about pH 5 to about pH 7, or from about pH 6 to about pH 7.

The hydrogel containing gelatin is heated or cooled so that the solution changes a phase from gel to sol or from sol to gel. The hydrogel of gelatin, etc. is converted to gel (jelly) by losing fluidity through cooling and converted to sol (aqueous solution) by acquiring fluidity through heating. When gelatin is taken as an example, gelatin is converted to jelly because some gelatin molecules take a helical structure similar to that of collagen by cooling and thereby form an intermolecular network. In this context, the "melting point" means a temperature at which solation occurs under a predetermined pressure, and the "freezing point" means a temperature at which gelation occurs under a predetermined pressure. As mentioned above, stronger gel is formed by continuous cooling. In the specification, the melting point of the hydrogel is from 20°C to 40°C (e.g., from 20°C to 35°C, from 25°C to 35°C, from 30°C to 40°C, or from 35°C to 40°C). In general, the melting point of gel is a measure for the strength of a network. The melting point of the hydrogel (e.g., gelatin) is elevated with the elevation of the concentration and molecular weight of the hydrogel. For example, the melting point and the freezing point tend to be elevated as a solid content is increased with a saccharide. Thus, it is possible to vary the melting point and the freezing point within predetermined ranges.

A method for measuring the melting point of the hydrogel is not particularly limited, and it can be measured by, for example, a method prescribed in JIS K6503. Specifically, gel having a height of 45 mm from a position of a 5 mm end is prepared from a test solution (e.g., a gelatin solution having a concentration of 10 w/w%) in a glass tube having 10 mm in diameter, and placed in a water tank such that the 5 mm end is positioned at the bottom. A temperature at which the gel is melted when heated so that the upper end of an air bubble is elevated by 10 mm may be defined as a melting point.

A method for measuring the freezing point of the hydrogel is not particularly limited, and it can be measured by, for example, a method prescribed in JIS K6503. Specifically, a test solution (e.g., a gelatin solution having a concentration of 10 w/w%) set to 35°C is gradually cooled with stirring in a water tank of 15°C having a buffering bath of 35°C. A temperature at which a return phenomenon (phenomenon in which air bubbles or the like resulting from the stirring of a solution do not inertially move in the direction of stirring and are instead brought back to the opposite direction, when the stirring is stopped) is found may be defined as a freezing point. In order to facilitate confirming the return phenomenon, a filter paper strip or the like may be placed in the test solution.

The "jelly strength" of the hydrogel means the mechanical strength of an object that has formed gel. It is usually expressed as force required for deforming gel in a predetermined shape or force required for breaking gel (unit: g, dyne(s)/cm$^2$ or g/cm$^2$), and is typically a measure for the hardness of gel. 1 dyne is defined as force which when acting on a body of mass 1 g produces an acceleration of 1 cm/s2 in that direction. For example, the jelly strength of the hydrogel containing gelatin varies depending on specific properties such as a molecular weight under the same conditions, though varying due to its concentration, temperature, pH and a coexistent substance, etc. The "jelly strength" of gelatin is tested by, for example, a method prescribed in Pharmaceutical and Food Safety Bureau (PFSB) Notification No. 0531(3) of the Japanese Pharmacopoeia or JIS K6503. Specifically, jelly is prepared by cooling a gelatin solution at 10°C for 17 hours, and load required for pushing down its surface by 4 mm using a plunger having a diameter of 1/2 inches (12.7 mm) is defined as jelly strength. Usually, the jelly strength is increased with increase in gelatin concentration. Usually, the jelly strength of gelatin is largely decreased at acidic pH (e.g., pH 4 or less) and alkaline pH (e.g., pH 8 or more). The jelly strength is elevated with the elevation of a molecular weight within a certain range (e.g., a molecular weight of 30,000 to 70,000), whereas the jelly strength becomes constant at a certain level or more (e.g., a molecular weight of 100,000 or more). Usually, gelation requires cooling at 20°C or less. For example, the jelly strength becomes higher at a lower cooling temperature within a cooling temperature range on the order of, for example, 4°C to 20°C. Since reaction for molecular orientation or network formation proceeds at a relatively slow rate, an intermolecular network is formed in the inside even after gel formation. Therefore, the jelly strength is elevated over about 1 to 5 hours after the start of cooling. Also, the jelly strength is elevated by rapid cooling. This is because an intermolecular network is finely formed without much time for molecular orientation.

In the specification, the jelly strength may be in a range that does not influence a cell or tissue to be embedded, for example, at 10°C to 20°C, and to an extent that the hydrogel does not collapse in the usual transplantation operation, etc. of the complex. In hydrogel (gelatin) whose jelly strength is on the order of 5% by weight to 30% by weight, the jelly strength of the hydrogel (gelatin) may be, for example, 50 g or more, 100 g or more, 200 g or more, 500 g or more, 1000 g or more, 1200 g or more, 1300 g or more, 1400 g or more or 1500 g or more in a method prescribed in JIS K6503. The jelly strength of the hydrogel (gelatin) may be 3000 g or less, 2500 g or less or 2000 g or less.

The "viscosity" is an index that represents the strength of stickiness of a fluid. Those skilled in the art can measure the viscosity by a known method. It can be measured by, for example, a method prescribed in JIS K6503. Specifically, a time for which a predetermined amount of a gelatin solution (60°C, 6.67%) flows down in a pipette-based viscometer may be converted to a viscosity value (unit: mPa·s).

The viscosity in a sol state of the hydrogel containing gelatin is influenced by the concentration of gelatin, etc., the temperature of the system, pH, coexistent salts, etc. In general, the viscosity is elevated with the elevation of the concentration of gelatin, etc. or decrease in temperature. For example, the viscosity of B-type gelatin depends on pH, and the viscosity becomes minimal around pH of an isoionic point. On the other hand, A-type gelatin is not found to have a marked relationship between viscosity and pH.

The viscosity of the hydrogel containing gelatin is not particularly limited as long as it is viscosity to an extent that the complex mentioned later can be formed. Since live cells are embedded therein, it is necessary to embed cells using the hydrogel of about 40°C (from about 30°C to about 50°C) in consideration of damage on the cells. Thus, examples thereof include hydrogel that exhibits viscosity required for embedding cells at about 40°C (from about 30°C to about 50°C), for example, viscosity on the order of 2 to 50 mPa·s (from 5 to 30 mPa·s).

The "rate of foaming" means the ratio of the total volume (Vi) including foam to the original volume ($V_0$) of a sample ($V_1/V_0$). A lower rate of foaming is preferable. Specifically, it is 1.2 or less (preferably 1.15 or less, 1.1 or less, or 1.05 or less). The rate of foaming can be measured by, for example, a PAGI method, a method for testing photographic gelatin, the 10th edition (2006 edition). Specifically, 50 mL of a test solution of 50°C placed in a measuring cylinder is vibrated for 1 minute at an amplitude of 300 mm with a frequency of 145 times/min. Then, the total volume including foam may be read 3 minutes after the termination of vibration.

It is preferable that the hydrogel described in the specification should meet the criteria of a purity test prescribed in the Japanese Pharmacopoeia, because it is administered *in vivo* by transplantation. For example, the specifications and testing methods of the quality of gelatin and purified gelatin are prescribed in the Japanese Pharmacopoeia. Specifically, the following criteria are satisfied.

(1) Off-flavor and insoluble matter: 40 mL of water is added to 1.0 g of the product, and when it is melted by heating, the solution has no unpleasant odor. This solution is clear or turbid only slightly, and its color is not darker than that of solution A for color comparison.
(2) Sulfite: 60 ppm or less
(3) Heavy metal: 50 ppm or less (20 ppm or less)
(4) Arsenic: 1 ppm or less
(5) Mercury: 0.1 ppm or less
(6) Loss in weight on drying: 15.0% or less
(7) Ignition residue: 2.0% or less

A higher concentration of the hydrogel is preferable from the viewpoint of preventing collapse ascribable to various operations or dissolution, etc. during operation. The concentration of the hydrogel is the ratio of the hydrogel to a vehicle (solvent) in which the hydrogel is dissolved. In the case of using, for example, gelatin, the concentration of the hydrogel may be from 10% by weight to 50% by weight and is preferably from 25% by weight to 50% by weight, from 30% by weight to 50% by weight, or from 20% by weight to 40% by weight.

The weighed hydrogel may be dissolved in a suitable vehicle such that the concentration of the hydrogel falls within the range mentioned above. The vehicle may be a vehicle that does not influence cells. Examples thereof include buffered salt solutions such as HBSS.

The pH of the hydrogel is preferably around neutrality for the purpose of reducing damage on tissue to be embedded or a living body after transplantation. It may be, for example, from 6 to 8 or from 6.5 to 7.5 and is preferably from 7 to 7.5. The pH of the hydrogel may be measured as pH in a solution or sol state. As mentioned above, jelly strength is not largely reduced within the pH range. A method for measuring the pH of the hydrogel is not particularly limited, and it can be measured using, for example, a commercially available pH meter or a pH test strip.

In an embodiment, it is preferable the hydrogel should be hydrogel having biodegradability. After the complex is transplanted to a human, the transplanted neural retina comes in gradual contact with a retina *in vivo* by the biodegradation of the biodegradable hydrogel so that a high graft survival rate is obtained. Gelatin, which is obtained by treating the main *in vivo* constituent collagen, is hydrogel having biodegradability.

In an embodiment, the hydrogel in the specification is preferably hydrogel that has a melting point in the range of 20°C to 40°C and satisfies one or more of the following physical properties:

(1) preparation method: alkali treatment and/or heat treatment
(2) average molecular weight: from about 100,000 to about 500,000
(3) concentration: from 10% by weight to 50% by weight (preferably from 25% by weight to 50% by weight, from 30% by weight to 50% by weight, or from 20% by weight to 40% by weight)
(4) jelly strength: 50 g or more, 100 g or more, 200 g or more, 500 g or more, 1000 g or more, 1200 g or more, 1300 g or more, 1400 g or more or 1500 g or more (3000 g or less, 2500 g or less or 2000 g or less)
(5) pH :from 6 to 8 (preferably from 6.5 to 7.5 or from 7 to 7.5)
(6) isoionic point: acidic region (from about pH 4 to about pH 7, from about pH 5 to about pH 7, or from about pH 6 to about pH 7)
(7) rate of foaming: 1.2 or less (preferably 1.15 or less, 1.1 or less, or 1.05 or less)
(8) viscosity: from about 5 to 30 mPa·s at about 40°C (from about 30°C to 50°C)

(3) Complex

<Structure of complex>

In an embodiment, the complex comprises two or more neural retina-containing cell aggregates and a matrix, the two or more cell aggregates being arranged in the matrix. In this context, the phrase "arranged in the matrix" means a state where the two or more cell aggregates are buried in the matrix gel, i.e., are present in a state covered with the matrix gel. Since the two or more cell aggregates are covered with the matrix gel, the cell aggregates are not in direct contact with the external environment (preservation solution, etc.) of the complex. Although a transplant neural retina sheet (Cap) dissected from the cell aggregates, particularly, sphere-like cell aggregates, is small and is therefore difficult to handle as it is, there is an advantage that the cell aggregates are arranged in the matrix gel and thereby become easy to handle. Also, it becomes possible to simultaneously transplant a plurality of cell aggregates.

In the complex, the two or more cell aggregates may be in partial contact with each other or may not be in contact with each other. It is preferable that the two or more cell aggregates should be separated via the matrix gel and be not in contact with each other, from the viewpoint of reducing damage on cells due to mechanical contact. The phrase "not in contact with each other" means that the cell aggregates are not in physical contact with each other. The cell aggregates that are not in contact with each other can be confirmed, for example, visually (e.g., under a microscope).

In an embodiment, it is preferable that the two or more neural retina-containing cell aggregates should be arranged in a row in the matrix gel. In this context, the term "in a row" means that the two or more neural retina-containing cell aggregates assume a continuous linear pattern, and the linear pattern may be a straight line or a curve that roughly extends in a predetermined direction. It is preferable that 2 to 20 cell aggregates should be arranged in a row, and it is more preferable that 4 to 16, 4 to 14, 4 to 10, or 6 to 8 cell aggregates should be arranged in a row. Although their respective apical surfaces or basal surfaces may face the same direction or may face different directions, it is preferable that the two or more neural retinas should be aligned side by side in a row such that the respective apical surfaces face a direction roughly perpendicular to the linear direction in the same direction.

In an embodiment, each neural retina may be a transplant neural retina (also referred to as a graft or Cap) dissected from a cell aggregate or a neural retina sheet obtained by dispersing a cell aggregate into single cells which are then prepared into a sheet again. The complex of the present invention contains two or more neural retina-containing cell aggregates and can thereby treat a wider range of damage states of retinal tissue.

[Method for manufacturing complex]

The method for manufacturing a complex in which two or more neural retina-containing cell aggregates are arranged in a matrix according to the present invention comprises: (1) a first step of preparing two or more cell aggregates of neural retinas from pluripotent stem cells; and (2) a second step of contacting the two or more cell aggregates in a predetermined arrangement with the matrix or a precursor of the matrix, followed by the gelation of the matrix.

In the first step (1), it is preferable that the two or more neural retina-containing cell aggregates should be derived from human pluripotent stem cells, and it is also preferable to be transplant tissue.

A method for producing the neural retina-containing cell aggregates (sphere-like cell aggregates, neural retina sheets, transplant neural retina sheets) from pluripotent stem cells is as described above. Detachment with an enzyme (e.g., collagenase) that degrades an extracellular matrix may be performed for the recovery of the neural retina-containing cell aggregates which have been cultured on the extracellular matrix (e.g., collagen) and produced. In this case, it is preferable to perform washing a plurality of times with a culture medium or the like, in order to prevent contamination with the enzyme.

In an embodiment, the second step (2) comprises contacting the two or more cell aggregates in a predetermined arrangement with a matrix precursor, followed by the gelation of the matrix. When the matrix is fibrin gel, the matrix precursor is thrombin and fibrinogen. In general, thrombin is a gelling agent and is not called precursor. However, in the specification, thrombin is also referred to as a matrix precursor. The gelation may be performed by contacting all the two or more cell aggregates with any one of a fibrinogen solution and a thrombin solution as matrix precursors and then further with the other solution of the fibrinogen solution and the thrombin solution so that they are reacted, or the gelation may be performed by contacting some cell aggregates of the two or more cell aggregates with any one of a fibrinogen solution and a thrombin solution, and then contacting the remaining cell aggregates of the two or more cell aggregates with the other solution of the fibrinogen solution and the thrombin solution so that they are reacted. In this context, the contact with a solution means that the cell aggregates are contacted so as to be soaked in the fibrinogen solution or the thrombin solution.

It is preferable that the fibrinogen solution and the thrombin solution should be used in the range of 1:1 to 1:9, preferably 1:3 to 1:4, in terms of an activity ratio.

In an embodiment, the second step (2) comprises contacting the two or more cell aggregates in a predetermined arrangement with the matrix, followed by the gelation of the matrix. Examples of the matrix include gelatin, collagen, pectin, hyaluronic acid, and alginic acid, as described above. It is preferable to be gelatin. A suitable concentration of a gelatin solution and a gelation method are as mentioned above.

Contacting the two or more cell aggregates in a predetermined arrangement with the matrix or a precursor of the matrix means that the two or more cell aggregates are aligned in the desired arrangement (e.g., in a row) and then contacted with the matrix or the precursor of the matrix, or the two or more cell aggregates are aligned in the desired arrangement (e.g., in a row) in the matrix or the precursor of the matrix. It can be carried out, for example, by providing a container having a narrow groove, and aligning the two or more cell aggregates at a predetermined interval and in a predetermined orientation. The shape of the groove may be a V shape or may be a U shape or may be a flat bottom, and the shape is not limited. The diameter of the groove may serve as the diameter of the complex, and for example, the depth may be from 100 $\mu$m to 2000 $\mu$m. Also, the length of the groove may serve as the length of the complex and may be, for example, from 300 $\mu$m to 50000 $\mu$m, though the length is not particularly limited and it is preferable to adjust it to a length that permits transplantation. It is also preferable that an interval on the order of 10 $\mu$m to 50 $\mu$m should be provided between the two or more cell aggregates. It is preferable that the two or more cell aggregates should be aligned side by side in a row such that the apical surfaces of their respective neural retinas face the same direction, particularly, a direction roughly perpendicular to the extending direction of the groove. In an embodiment, the cell aggregates become easy to align side by side in a row by setting the width of the groove to almost the same as the minor axis of the cell aggregates to about twice the minor axis.

In the case of a matrix precursor, the gelation may be performed by adding any one of a fibrinogen solution and a thrombin solution which are precursors of fibrin gel to the groove, arranging therein the two or more cell aggregates at a predetermined interval and in a predetermined orientation, and gently further adding the other solution of the fibrinogen solution and the thrombin solution such that the two or more cell aggregates are not washed away from a predetermined position. In the case of gelatin, the gelation may be performed by adding a gelatin solution to the groove, arranging therein the two or more cell aggregates at a predetermined interval and in a predetermined orientation, and gently further adding the gelatin solution such that the two or more cell aggregates are not washed away from a predetermined position, followed by cooling. Before this operation, operation of blending the cell aggregates with the matrix precursor or the matrix may be performed. Specifically, the final gelatin concentration of interest can be easily attained by staged blending with the matrix precursor or the matrix having a concentration lower than the concentration of gelation.

In an embodiment, the complex obtainable by the manufacturing method of the present invention permits suction into and ejection from an injector without disrupting the matrix gel. More neural retina-containing cell aggregates can thereby be introduced to a transplantation site by single transplantation. Since the matrix gel has biodegradability, the neural retinas to be transplanted are engrafted by coming in gradual contact with a retina *in vivo.*

[Pharmaceutical composition, therapeutic product and treatment method]

An aspect of the present invention includes a pharmaceutical composition containing a complex obtainable in the present invention as an active ingredient. The pharmaceutical composition preferably further contains a pharmaceutically acceptable carrier, in addition to the complex of the present invention.

The pharmaceutical composition can be used in the treatment of a disease caused by the damage of a retinal cell or retinal tissue or the injury of retinal tissue. Examples of the disease caused by the damage of a retinal cell or retinal tissue include ophthalmic diseases such as retinal degenerative diseases, macular degeneration, age-related macular degeneration, retinitis pigmentosa, glaucoma, corneal diseases, retinal detachment, central serous chorioretinopathy, cone dystrophy, and cone rod dystrophy. Examples of the injury state of retinal tissue include a state in which photoreceptor cells, retinal pigment epithelial cells, or the like die of degeneration.

As the pharmaceutically acceptable carrier, a physiological aqueous solvent (physiological saline, buffer, serum-free medium, etc.) can be used. If necessary, the pharmaceutical composition may be blended with a preservative, a stabilizer, a reducing agent, a tonicity agent, and the like which are usually used in a medicine containing tissues or cells to be transplanted in medical transplantation.

An aspect of the present invention includes a therapeutic product for a disease caused by the damage of a retinal cell or retinal tissue or the injury of retinal tissue, comprising a complex obtainable in the present invention.

The therapeutic product of the present invention can treat the damage state of a retinal cell or retinal tissue or the injury state of retinal tissue by transplanting the complex obtainable in the present invention to a patient having the disease caused by the damage of a retinal cell or retinal tissue or the injury of retinal tissue. Examples of the disease caused by the damage of a retinal cell or retinal tissue or the injury of retinal tissue include the diseases mentioned above.

An aspect of the present invention includes a composition for transplantation, comprising a complex obtainable in the present invention. The complex of the present invention can be used for transplanting it to the eye fundus in a patient. Also, the complex of the present invention can be used because it can be transplanted such that the neural retinas of the transplanted complex are engrafted in the recipient patient while facing the neural retinal layer of the patient. The composition for transplantation preferably further contains a pharmaceutically acceptable carrier, in addition to the complex of the present invention. The pharmaceutically acceptable carrier is as mentioned above.

One aspect of the present invention includes a method for treating a disease caused by the damage of a retinal cell or retinal tissue or the injury of retinal tissue, comprising the following steps:

(1) the step of transplanting a complex obtainable in the present invention to the eye fundus in a patient; and
(2) the step in which the neural retinas of the transplanted complex are engrafted *in vivo* in the patient while facing the neural retinal layer of the patient.

One aspect of the present invention includes a method for treating a disease caused by the damage of a retinal cell or retinal tissue or the injury of retinal tissue, comprising transplanting a complex obtainable in the present invention to a subject in need of transplantation (e.g., subretinally to an eye having the ophthalmic disease). As the therapeutic product for a disease caused by the damage of a retinal cell or retinal tissue, or in order to make up for a corresponding injured site in the injury state of the retinal tissue, the complex of the present invention can be used. The disease caused by the damage of retinal tissue or the injury state of retinal tissue can be treated by transplanting the complex of the present invention to a patient having the disease caused by the damage of a retinal cell or retinal tissue, or a patient with the injury state of retinal tissue, in need of transplantation, and making up for the damaged retinal tissue. Examples of a transplantation method include a method of subretinally transplanting the transplant complex of the present invention to an injured site through an incision to an eyeball. Examples of a method for transplantation include a method of performing infusion using a thin tube, and a method of performing transplantation by sandwiching between tweezers, and examples of the thin tube include injection needles.

## Examples

<Example 1 Preparation of complex gel of fibrin gel and plurality of transplant neural retinas>

A study was made on a complex in which a plurality of neural retina-containing cell aggregates (grafts) were encapsulated in fibrin gel. As the grafts, neural retinal tissue pieces (also referred to as Caps) were used. The Caps were prepared as follows: Human ES cells genetically engineered so as to have Rx:: Venus reporter gene (derived from KhES-1 strain (Non Patent Literature 1)) were cultured under feeder-free conditions in accordance with the method described in "Scientific Reports, 4, 3594 (2014)". As a feeder-free medium, StemFit medium (trade name: AK03N, manufactured by Ajinomoto Co., Inc.) was used, and as a scaffold as an alternative to feeder cells, Laminin511-E8 (trade name, manufactured by Nippi, Inc.) was used.

Specific operation of maintenance culture for human ES cells was performed as follows: First, human ES cells that reached sub-confluency (state where about 60% of the culture area was covered by cells) were washed with PBS and separated into single cells by use of TrypLE Select (trade name, manufactured by Life Technologies). Then, the separated human ES single cells were seeded in plastic culture dishes coated with Laminin 511-E8 and cultured under feeder-free conditions in StemFit medium in the presence of Y27632 (ROCK inhibitor, 10 μM). When 6-well plates (for cell culture, culture area: 9.4 cm$^2$, manufactured by AGC TECHNO GLASS., LTD) were used as the plastic culture dishes, the number of separated human ES single cells to be seeded was specified as $1.2 \times 10^4$ cells per well. One day after seeding, the medium was exchanged with StemFit medium not containing Y27632. Thereafter, the medium was exchanged with Y27632-free StemFit medium once every 1 to 2 days. Thereafter, the cells were cultured under feeder-free conditions until 1 day before reaching sub-confluency. The human ES cells the 1 day before the sub-confluency were cultured for 1 day (preconditioning treatment) under feeder-free conditions in the presence of SB431542 (TGFβ signaling pathway inhibitor, 5 μM) and SAG (Shh signaling pathway agonist, 300 nM).

The human ES cells were washed with PBS, then treated for cell dispersions using TrypLE Select, and further separated into single cells by pipetting. Thereafter, the separated human ES single cells were suspended in 100 μL of a serum-free medium such that the density of cells per well of a non-cell adhesive 96-well culture plate (trade name: PrimeSurface, 96-well V-bottom plate, manufactured by Sumitomo Bakelite Co., Ltd.) was $1.2 \times 10^4$ cells, and subjected to suspension culture in the conditions of 37°C and 5% CO$_2$. The serum-free medium (gfCDM + KSR) used herein is a serum-free medium prepared by adding 10% KSR and 450 μM 1-monothioglycerol and 1X Chemically defined lipid concentrate to a mixture of culture fluids containing F-12 medium and IMDM medium in a ratio of 1:1.

At the initiation time of the suspension culture (Day 0 from initiation of the suspension culture), Y27632 (ROCK inhibitor, final concentration: 10 μM) and SAG (Shh signaling pathway agonist, 300 nM, 30 nM or 0 nM) were added to the serum-free medium. Day 3 from initiation of the suspension culture, 50 μL of a medium containing exogenous human recombinant BMP4 at a final concentration of 1.5 nM was added using a medium, which did not contain Y27632 or SAG and contained human recombinant BMP4 (trade name: Recombinant Human BMP-4, manufactured by R&D). Day 6 or later from initiation of the suspension culture, a half of the medium was exchanged with a culture medium, which did not contain Y27632, SAG or human recombinant BMP4, once every 3 days.

Further, the aggregates of Day 14 from initiation of the suspension culture were transferred to a 90-mm low adhesive plate (manufactured by Sumitomo Bakelite Co., Ltd.) and cultured for 3 days in the conditions of 37°C and 5% CO$_2$ in a serum-free medium (DMEM/F12 medium supplemented with 1% N2 Supplement) containing a Wnt signaling pathway agonist (CHIR99021, 3 μM) and a FGF signaling pathway inhibitor (SU5402, 5 μM). Thereafter, culture was performed using a DMEM/F12 medium, which did not contain the Wnt signaling pathway agonist or the FGF signaling pathway inhibitor and contained serum (NucT0 medium), in a 90-mm low adhesive plate (manufactured by Sumitomo Bakelite Co., Ltd.). Day 40 or later from initiation of the suspension culture, culture was performed using a mixture of a NucT0 medium and a NucT2 medium (NucT1 medium). Day 60 or later from initiation of the suspension culture, culture was performed using a neurobasal medium containing thyroid gland hormone signaling pathway agonist T3 (NucT2 medium). A plurality of Caps were dissected from the aggregates of Day 82 from initiation of the suspension culture using microscissors. A study was made on the formation of a complex using the neural retinal tissue pieces (Caps) thus obtained.

As a container for Caps to be aligned and left standing, a container was prepared in which a groove having a width on the order of 1 mm, a depth on the order of 1 mm, and a length on the order of 20 mm was dug in silicone gel using a surgical knife. 8 to 13 dissected Caps were aligned at roughly equal intervals in the groove using tweezers.

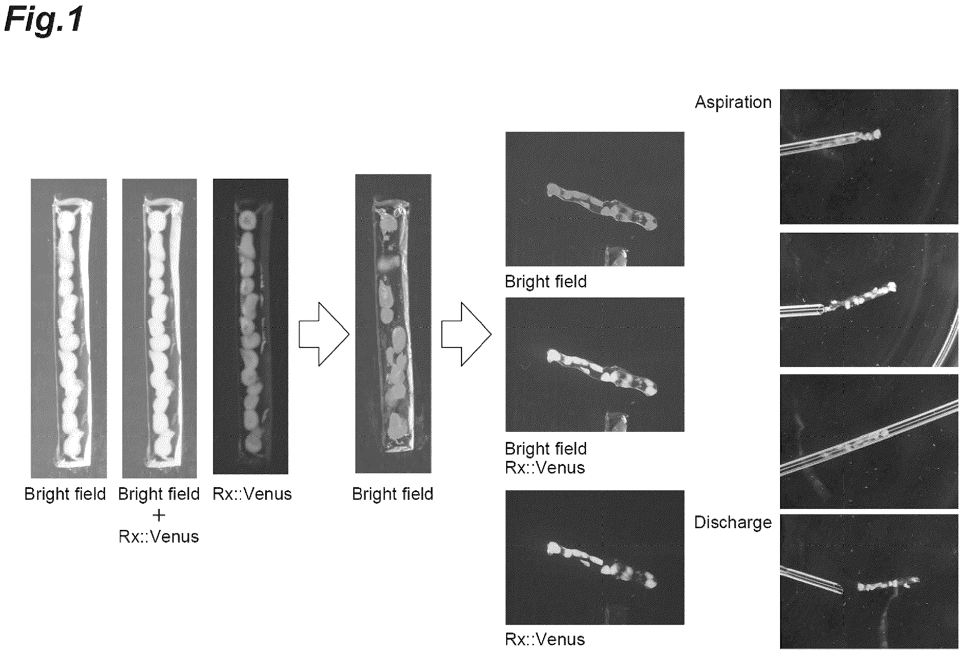

First, 50 μL of fibrinogen was added to the groove in which the Caps were aligned, then blended, and removed. Again, 50 μL of fibrinogen was added and wholly spread. Next, 50 μL of thrombin was added and converted to fibrin by incubation at room temperature for 20 minutes. In this way, a complex (complex gel) in which vertically continuous Caps were disposed in fibrin gel was able to be prepared (Figure 1).

The ease of handling of the complex gel was studied. The complex gel prepared as fibrin gel was recovered from the groove using tweezers and a surgical knife and was consequently able to be recovered in a solid state. This fibrin gel was transferred into HBSS, and whether to endure the operation of aspiration and discharge was confirmed using 20 G Surflo. As a result, it was able to be confirmed that the gel could be unproblematically taken in and out in the state of the complex without collapsing (Figure 1). This complex gel was able to be confirmed to have strength that endured operation at the time of transplantation.

Thus, it was found that complex gel that is easy to handle can be prepared by adding thrombin and fibrinogen to neural retinal tissue pieces (Caps) so that fibrin gel is prepared in which a plurality of neural retina Caps become solid.

<Example 2 Preparation of complex gel of gelatin and plurality of transplant neural retinas>

A study was made on a complex in which a plurality of neural retina-containing cell aggregates (grafts) were encapsulated in gelatin. As the grafts, neural retinal tissue pieces (also referred to as Caps) were used. The Caps were prepared as follows: Human ES cells genetically engineered so as to have Rx:: Venus reporter gene (derived from KhES-1 strain (Non Patent Literature 1)) were cultured under feeder-free conditions in accordance with the method described in "Scientific Reports, 4, 3594 (2014)". As a feeder-free medium, StemFit medium (trade name: AK03N, manufactured by Ajinomoto Co., Inc.) was used, and as a scaffold as an alternative to feeder cells, Laminin511-E8 (trade name, manufactured by Nippi, Inc.) was used.

A plurality of Caps were dissected from aggregates of Day 82 from initiation of the suspension culture, which were prepared in the same manner as in Example 1, using microscissors. A study was made on the formation of a complex using the neural retinal tissue pieces (Caps) thus obtained.

As a container for Caps to be aligned and left standing, a container was prepared in which a groove having a width on the order of 1 mm, a depth of 1 mm, and a length on the order of 20 mm was dug in silicon gel using a surgical knife. 8 to 10 dissected Caps were aligned at roughly equal intervals in the groove using tweezers.

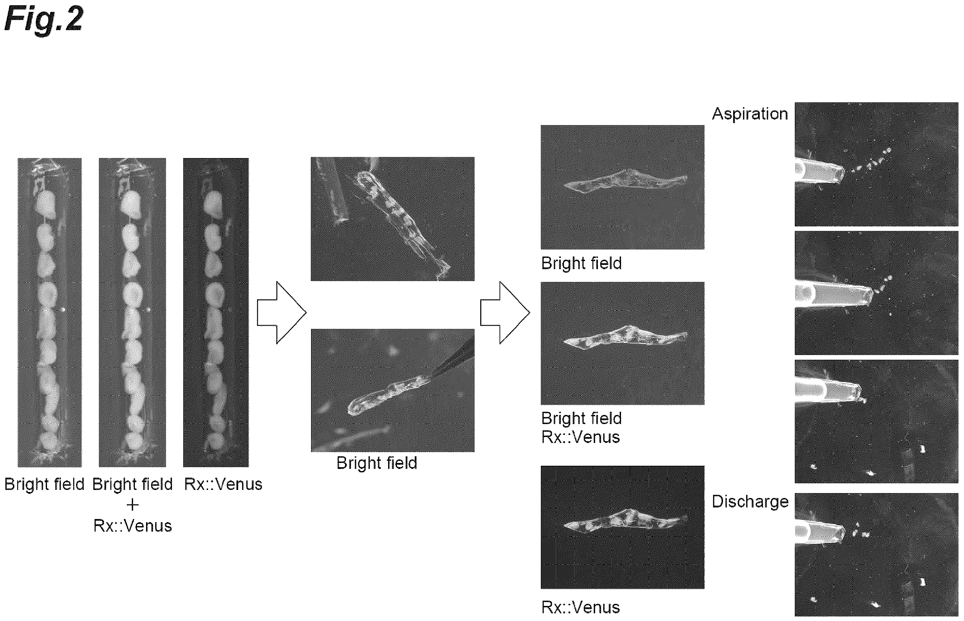

First, 50 µL of 10% gelatin (w/v) (LS-W from Nitta Gelatin Inc.) was added to the groove in which the Caps were aligned, then blended, and removed. Again, 50 µL of 20% gelatin (w/v) was added, then blended, and removed. Next, 50 µL of 30% gelatin was added and incubated at 4°C for 20 minutes. In this way, a complex (complex gel) in which solidified, vertically continuous Caps were disposed was able to be prepared (Figure 2).

The ease of handling of the complex gel was studied. The complex gel of the solidified gelatin was recovered from the groove using tweezers and a surgical knife and was consequently able to be recovered in a solid state. This gelatin was transferred into HBSS, and whether to endure the operation of aspiration and discharge was confirmed using a 1 ml syringe with a catheter tip. As a result, it was able to be confirmed that the gel could be unproblematically taken in and out in the state of the complex without collapsing (Figure 2). This complex gel was able to be confirmed to have strength that endured operation at the time of transplantation.

Thus, it was found that complex gel that is easy to handle can be prepared by adding gelatin to neural retinal tissue pieces (Caps) so that a plurality of neural retina Caps become solid by solidification.

<Reference Example 1 Production of retina sheet containing neural retina>

Human iPS cells (DSP-SQ strain, established by Sumitomo Dainippon Pharma Co., Ltd.) were subjected to feeder-free culture in accordance with the method described in Scientific Reports, 4, 3594 (2014). As a feeder-free medium, StemFit medium (AK03N, manufactured by Ajinomoto Co., Inc.) was used, and as a feeder-free scaffold, Laminin511-E8 (manufactured by Nippi, Inc.) was used.

Operation of differentiation was carried out as follows: Human iPS cells (DSP-SQ strain) were cultured in feeder-free using StemFit medium until 2 days before the cells reached sub-confluency (state where about 30% of the culture area is covered by cells). The human iPS cells the 2 days before the sub-confluency were subjected to feeder-free culture for 2 days (preconditioning treatment) in the presence of SAG (300 nM).

The preconditioned human iPS cells were treated for cell dispersions using TrypLE Select (manufactured by Life Technologies) and further separated into single cells by pipetting. Thereafter, the separated human iPS single cells were suspended in 100 µl of a serum-free medium such that the density of cells per well of a non-cell adhesive 96-well culture plate (PrimeSurface, 96 V-bottom plate, manufactured by Sumitomo Bakelite Co., Ltd.) was $1.3 \times 10^4$ cells, and subjected to suspension culture in the conditions of 37°C and 5% $CO_2$. The serum-free medium (gfCDM + KSR) used herein is a serum-free medium prepared by adding 10% KSR and 450 µM 1-monothioglycerol and 1X Chemically defined lipid concentrate to a mixture of culture fluids containing F-12 medium and IMDM medium in a ratio of 1:1. At the initiation time of the suspension culture (Day 0 from initiation of the suspension culture), Y27632 (final concentration 20 µM) and SAG (final concentration 10 nM) were added to the serum-free medium. Day 2 from initiation of the suspension culture, 50 µl of a fresh serum-free medium (the same one as mentioned above), which did not contain Y27632 or SAG and contained human recombinant BMP4 (manufactured by R&D), was added such that the final concentration of exogenous human recombinant BMP4 became 1.5 nM (55 ng/ml).

Four days later (that is, Day 6 from initiation of the suspension culture), the medium was exchanged with the serum free medium, which did not contain Y27632, SAG or human recombinant BMP4. Operation of medium exchange was carried out as follows: 60 µl of the medium in the incubator was discarded, 90 µl of a fresh serum-free medium (the same one as mentioned above) was added. This operation was carried out to control the total medium volume to be 180 µl. Thereafter, a half of the medium was exchanged with serum-free medium, which did not contain Y27632, SAG or human recombinant BMP4, once every 2 to 4 days. The operation for exchanging a half volume of the medium was as follows. A half volume, i.e., 90 µl, of the medium in the incubator was discarded, 90 µl of a fresh serum-free medium (the same one as mentioned above) was added to control the total medium volume to be 180 µl.

The cell mass obtained on Day 13 from initiation of the suspension culture was cultured in a serum free medium (prepared by adding 1% $N_2$ supplement to DMEM/F12 medium) containing CHIR99021 (3 µM) and SU5402 (5 µM), for 3 days, i.e., up to Day 16 from initiation of the suspension culture.

The resultant cell aggregate on Day 16 from initiation of the suspension culture was cultured in each of the serum media shown in the following [1], [2] and [3] in the condition of 5% $CO_2$ up to Day 75 from initiation of the suspension culture.

[1] Day 16 to day 40 from initiation of the suspension culture:
DMEMIF12 medium containing 10% fetal bovine serum, 1% $N_2$ supplement and 100 $\mu$M taurine (hereinafter referred to as medium A).
[2] Day 40 to day 60 from initiation of the suspension culture:
Mixture of culture fluids containing medium A and a medium, which was Neurobasal medium containing 10% fetal bovine serum, 2% B27 supplement, 2 mM glutamine, 60 nM T3 and 100 $\mu$M taurine (hereinafter referred to as medium B) in a ratio of 1:3.
[3] On and after Day 60 from initiation of the suspension culture: medium B.

The cell mass on Day 75 from initiation of the suspension culture was observed under an inverted microscope to confirm morphology. It was found here that a neuroepithelial structure was formed.

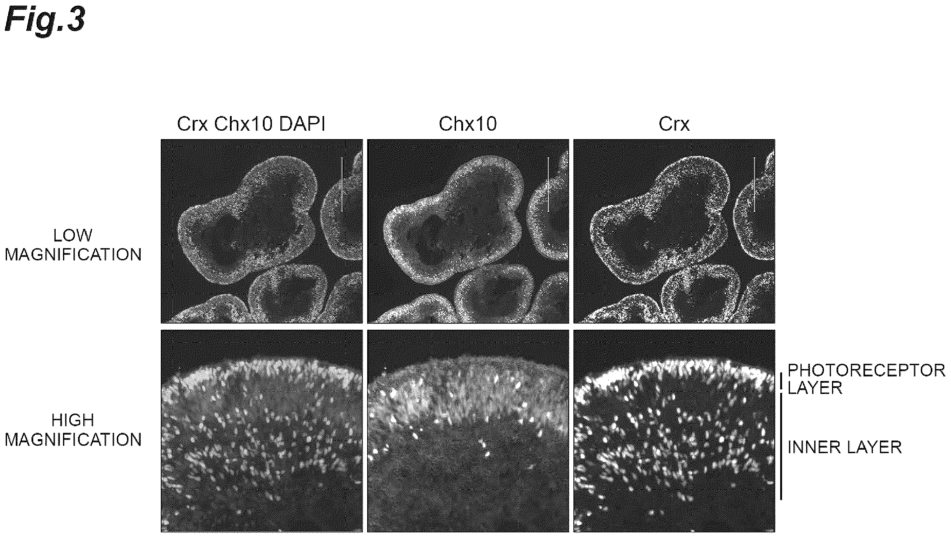

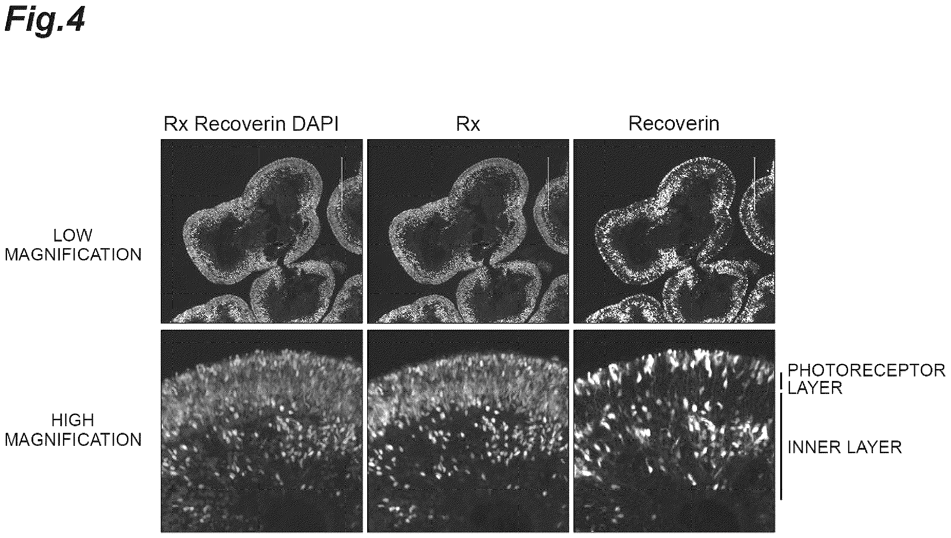

The cell mass on Day 75 from initiation of the suspension culture was fixed with 4% paraformaldehyde, frozen and sectioned. The frozen sections were subjected to immunostaining to stain a neural retina marker, Chx10 (anti-Chx10 antibody, Exalpha Biologicals, sheep) and a photoreceptor progenitor cell marker Crx (anti-Crx antibody, Takara Bio Inc., rabbit) (Figure 3). Other frozen sections were subjected to immunostaining to stain a neural retina marker Rx (anti-Rx antibody, Takara Bio Inc., guinea pig) and a photoreceptor cell marker recoverin (anti-recoverin antibody, Proteintech Group, rabbit) (Figure 4). The nuclei of the cells were stained with DAPI.

These sections stained were observed using a fluorescence microscope (manufactured by Keyence Corp.) to obtain immunostained images. The photographs in which the produced cells were observed under a fluorescence microscope are shown in Figure 3 and Figure 4. The upper boxes of Figure 3 and Figure 4 are images taken with a low-magnification lens, and the lower boxes are images taken with a high-magnification lens.

From the DAPI-stained images of Figure 3 and Figure 4, it was found that neural tissue densely packed with cells was formed on the surface of the cell mass and this neural tissue formed a continuous epithelium structure. As a result of analyzing the image of Figure 3, it was found that in this neural tissue, a Crx-positive layer (photoreceptor layer) with a thickness on the order of 2 to 5 cells was formed on the surface of the cell mass, a Chx10-positive layer with a thickness on the order of 5 to 20 cells was formed inside the Crx-positive layer, and a layer in which Crx-positive cells were sparsely present was further formed inside it (Figure 3). It was found that the surface of this cell mass was morphologically an apical surface. Furthermore, as a result of analyzing the image of Figure 4, it was found that in this neural tissue, a recoverin-positive layer (photoreceptor layer) was formed and a Rx-positive layer was also formed. From these results, it was found that in this neural tissue, a photoreceptor layer containing Crx-positive cells and recoverin-positive cells was formed on the surface, a retinal progenitor cell layer containing Chx10-positive cells was formed inside the photoreceptor layer, and a cell layer was also formed inside the retinal progenitor cell. In short, it was found that by this production method, a neural retina containing a photoreceptor layer and a retinal progenitor cell layer can be prepared from human iPS cells and this neural retina has a continuous epithelium structure.

<Reference Example 2 Preparation and evaluation of graft>

A three-dimensional retina prepared from human iPS cells consists of a neural retina having a neuroepithelial structure with the continuity of the composition or distribution of cells. This neural retina having the neuroepithelial structure has a layer structure constituted by a photoreceptor layer and an inner layer and has a characteristic appearance and morphology (Figure 3).

Individual human three-dimensional retinas differ in shape with a size on the order of 1 to 2 mm. Although a neural retina that is used in transplantation is a main product owing to the characteristics of a production method using self-organization culture, eyeball-related tissue (RPE, ciliary body, etc.) and brain and spinal cord tissue (telencephalon, spinal cord, etc.) which are non-neural retinas are produced as by-products. Therefore, the central part of a neural retina that did not contain a non-neural retina was dissected to obtain a retina piece (graft, cap) (Figure 5 and Figure 6). Specifically, a neighboring part of the retina piece (Cap) was used as a sample for quality evaluation (Ring), and only a Cap corresponding to a Ring adapted for references was used as a transplant neural retina by conducting analysis (preferably, quantitative PCR).

Figure 5 and Figure 6 are conceptual views of typical cell aggregates. A site at which the neuroepithelial structure (preferably continuous epithelium structure) intrinsic to the neural retina where a photoreceptor layer and an inner layer appeared to be divided as two layers was found, was regarded as a graft (cap). A site that is a neighboring site of the Cap and exhibits a neuroepithelial structure (preferably continuous epithelium structure) similar to that of the Cap was regarded as a sample for quality evaluation (ring). A site other than the Cap and the Ring was referred to as a root.

An approach of isolating a Cap and a Ring from a neuroepithelial structure contained in one cell aggregate is as mentioned below.

<Reference Example 3 Shape of graft (cap)>

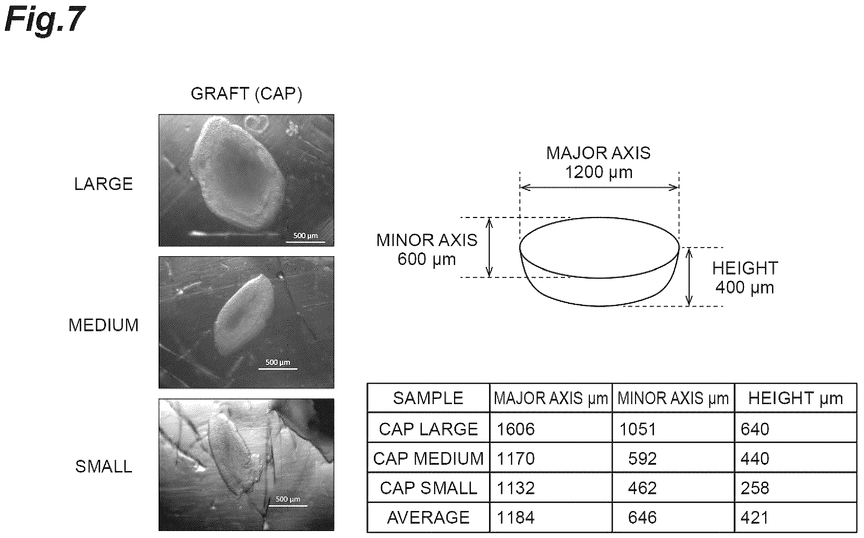

Grafts (caps) were prepared by the following method (Figure 7). First, a bright-field image (phase contrast image) of a cell aggregate on Day 99 from initiation of suspension culture prepared from human iPS cells (DSP-SQ strain) in accordance with the method described in Reference Example 1 was taken under an inverted microscope (manufactured by Olympus Corp.). After confirming that a neural retina was present on the cell aggregate, the cell aggregate was transferred to under a stereo microscope, and various sizes of the neural retina were dissected as grafts by use of the method given below. Also, study was made on the influence of a graft size on the operation of transplantation with a device for transplantation.

(Dissection of retina sheet of neural retina)

The cell aggregate was subjected to observation by an inverted microscope (ECLIPSE Ti, manufactured by Nikon Corp.) as a bright-field image (phase contrast image). The observation was performed, particularly, focusing on features of the morphology of individual cells and the mutual adhesion state between the cells. In the cell aggregate, a site having a continuous epithelium structure where an outer neuroblastic layer (containing photoreceptor layer and neural retinal progenitor cell layer) and an inner neuroblastic layer appeared to be divided as two layers was determined as the neural retina. Also, a tissue in which a continuous epithelium structure was not found, and a site having a continuous epithelium structure where, however, an outer neuroblastic layer and an inner neuroblastic layer were not able to be distinguished from each other and appeared to be one layer, were determined as by-products. Thereafter, while observed under a stereo microscope, tissue pieces were prepared by dissecting the neural retina from the cell aggregate under the stereo microscope using tapered tweezers and scissors.

(Influence of dissected retina sheet on transplantation operation)

A front image taken with a cut surface turned to an objective lens side, and a side image taken with the cut surface inclined so as to be perpendicular to an objective lens were taken under a stereo microscope as to the dissected grafts. Thereafter, the major axes, minor axes, and heights of the grafts were measured from the taken images. For the measurement, the major axis was defined as the longest line segment among line segments connecting two end points on the retina sheet cross section in the front image, and the length thereof. The minor axis was defined as the longest line segment among line segments connecting two end points on the retina sheet cross section in the front image and orthogonal to the major axis, and the length thereof. The height was defined as the longest line segment among line segments orthogonal to the retina sheet cross section in the side image and having a point intersecting the retina sheet cross section and the surface of the retina sheet as end points, and the length thereof. The volume of the graft was calculated according to the following calculation expression by approximating the graft as being an ellipsoid halved such that the cross section passed through the major axis.

$$\text{Volume} = 2/3 \times \text{Ratio of the circumference of a circle } (\pi) \times$$

$$(\text{Major axis} / 2) \times (\text{Minor axis} / 2) \times \text{Height}$$

As a result, it was found that the loading of a graft in a device for transplantation, the stability of the graft in the device for transplantation and the discharge of the graft from the device for transplantation were influenced by the size of the graft. It was also suggested that, particularly, the minor axis was a useful parameter. The major axis, the minor axis, the height and the volume were calculated as to each of 11 grafts for which the operation of transplantation with the device for transplantation was favorable. Results of determining an average value, the maximum value and the minimum value as to each parameter were summarized in Table 11. From this result, it was found that the graft (cap) was at least from 0.8 to 1.7 mm in major axis, from 0.4 to 1.1 mm in minor axis, from 0.2 to 0.7 mm in height, from about 0.07 to about 0.57 mm$^3$ in apparent volume.

[Table 11]

|  | Major axis [mm] | Minor axis [mm] | Height [mm] | Volume [mm$^3$] |
|---|---|---|---|---|
| Average value | 1.184 | 0.646 | 0.421 | 0.184 |
| Maximum value | 1.606 | 1.051 | 0.640 | 0.565 |
| Minimum value | 0.870 | 0.462 | 0.258 | 0.070 |

<Reference Example 4 Composition of cell in graft (cap)>

Grafts (caps) were prepared by the following method (Nos: 18001MF, d89, H5). First, grafts (caps) were isolated by the methods described in Reference Examples 2 and 3 from a cell aggregate on Day 89 from initiation of suspension culture prepared from human iPS cells (DSP-SQ strain) in accordance with the method described in Reference Example 1.

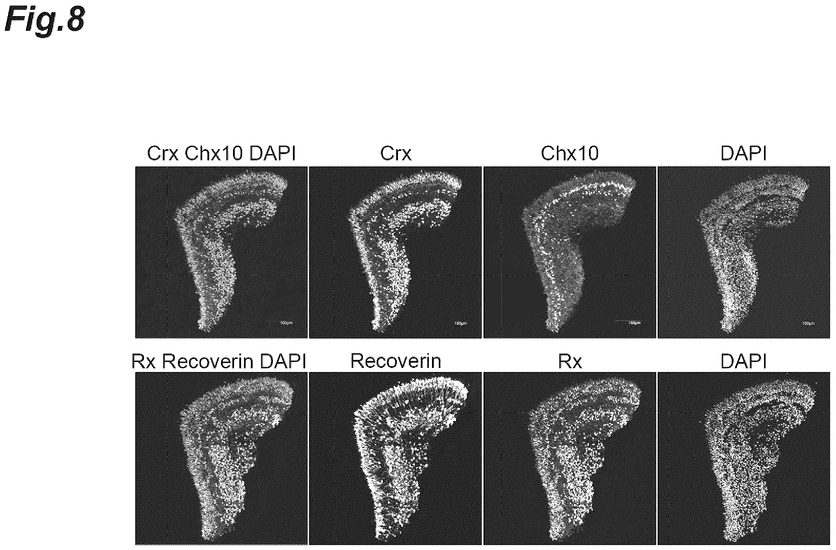

The graft was fixed with 4% paraformaldehyde, frozen and sectioned. The frozen sections were subjected to immunostaining to stain a neural retina marker, Chx10 (anti-Chx10 antibody, Exalpha Biologicals, sheep) and a photoreceptor progenitor cell marker Crx (anti-Crx antibody, Takara Bio Inc., rabbit) (Figure 8). Other frozen sections were subjected to immunostaining to stain a neural retina marker Rx (anti-Rx antibody, Takara Bio Inc., guinea pig) and a photoreceptor cell marker recoverin (anti-recoverin antibody, Proteintech Group, rabbit) (Figure 8). The nuclei of the cells were stained with DAPI. These sections stained were observed using a confocal laser microscope (manufactured by Olympus Corp.) to obtain immunostained images.

From the stained images, it was found that neural tissue densely packed with cells was formed on the surface (left side in the drawing) of the graft (cap) and this neural tissue formed a neuroepithelial structure (particularly, continuous epithelium structure) (Figure 8). It was further found that in this neural tissue, a Crx-positive layer (photoreceptor layer, Figure 8) with a thickness on the order of 2 to 10 cells was formed on the surface of the cell mass, a Chx10-positive layer with a thickness on the order of 5 to 20 cells was formed inside the Crx-positive layer, and a layer in which Crx-positive cells were present was further formed inside it (Figure 8). It was found that the surface of this graft (cap) was morphologically an apical surface. Furthermore, it was found that in this neural tissue, a recoverin-positive layer (photoreceptor layer, Figure 8, arrow) was formed and a Rx-positive layer was also formed. From these results, it was found that in this neural tissue, a photoreceptor layer containing Crx-positive cells and recoverin-positive cells was formed on the surface, a retinal progenitor cell layer containing Chx10-positive cells was formed inside the photoreceptor layer, and a cell layer was also formed inside the retinal progenitor cell. In short, it was found that the graft (cap) can prepare a neural retina containing a photoreceptor layer and a retinal progenitor cell layer and this neural retina has a continuous epithelium structure.

<Reference Example 5 Verification of equivalent state between Cap and ring>

Gene expression between a Cap and a Ring was compared by the following method. First, a cell aggregate on Day 99 from initiation of suspension culture was prepared from human iPS cells (DSP-SQ strain) in accordance with the method described in Reference Example 1, and used as lot 1. Further, a cell aggregate on Day 82 from initiation of suspension culture was prepared from human iPS cells (DSP-SQ strain) in accordance with the method described in Reference Example 1, and used as lot 2. The main product neural retina and by-products were determined as to these two lots using a microscope by the methods described in Reference Example 3 to isolate a Cap of the neural retina and caps of the by-products. Rings were isolated by dissection under a stereo microscope using tapered tweezers and scissors, as in the grafts. From the caps and the rings isolated from the neural retina and the by-products, total RNA was extracted using a spin column (manufactured by Qiagen N.V, RNeasy Micro kit) by the method described in the manual attached to the kit.

The concentration of the total RNA was measured in measurement equipment (Nanodrop, manufactured by Thermo Fisher Scientific Inc.), and then, it was reversely transcribed into cDNA using reverse transcriptase and primers (Reverse Transcription Master Mix Kit, manufactured by Fluidigm Corp.). The cDNA was subjected to multiplex-PCR reaction (Pre-Run) using all the probes used in the test and using a PCR apparatus (Veriti 96 well thermal cycler, manufactured by Applied Biosystems). Thereafter, the Pre-Run reaction solution was injected to multi-wells with flow channels (96.96 Dynamic Array IFC, manufactured by Fluidigm Corp.) using IFC Controller HX (manufactured by Fluidigm Corp.), and the expression level of marker gene in the neural retina and the by-products other than the neural retina was measured by real-time PCR using a multi-sample real-time PCR system (Biomark HD, manufactured by Fluidigm Corp.). The probes for PCR used in the test are shown in Table 12.

[Table 12]

| Classification | Gene name | Probe ID | GenBank ID |
|---|---|---|---|
| Internal standard | GAPDH | Hs02758991_g1 | NM_001256799.2, NM_001289745.2 NM_001289746.1, NM_001357943.1 NM_002046.7 |
| | ActinB | Hs01060665_g1 | NM_001101.5 |

(continued)

| Classification | Gene name | Probe ID | GenBank ID |
|---|---|---|---|
| Neural retina | *RAX* | Hs00429459_m1 | NM_013435.2 |
| | *Chx10* | Hs01584047_m1 | NM_182894.2 |
| | *SIX3* | Hs00193667_m1 | NM_005413.4 |
| | *SIX6* | Hs00201310_m1 | NM_007374.2 |
| | *RCVRN* | Hs00610056_m1 | NM_002903.2 |
| | *CRX* | Hs00230899_m1 | NM_000554.6 |
| | *NRL* | Hs00172997_m1 | NM_006177.4 |
| | *NESTIN* | Hs04187831_g1 | NM_006617.2 |
| Cerebrospinal | *FOXG1* | Hs01850784_s1 | NM_005249.4 |
| | *Emx2* | Hs00244574_m1 | NM_004098.4, NM_001165924.1 |
| | *Nkx2.1* | Hs00968940_m1 | NM_003317.3, NM_001079668.2 |
| | *Dmbx1* | Hs00542612_m1 | NM_172225.1, NM_147192.2 XM_011540668.2, XM_017000289.1 |
| | *HOXB2* | Hs01911167_s1 | NM_002145.3, XM_005257275.4 |
| | *HoxAS* | Hs00430330_m1 | NM_019102.4 |
| Eyeball | *MITF* | Hs01117294_m1 | NM_000248.3, NM_006722.2 NM_198158.2, NM_198159.2 NM_198177.2, NM_198178.2 NM_001184967.1, NM_001184968.1 NM_001354604.1, NM_001354605.1 NM_001354606.1, NM_001354607.1 NM_001354608.1 |
| | *aqp1* | Hs01028916_m1 | NM_198098.3, NM_001329872.1 |
| | *ZIC1* | Hs00602749_m1 | NM_003412.4 |
| | *PAX2* | Hs01057416_m1 | NM_000278.4, NM_003987.4 NM_003988.4, NM_003989.4 NM_003990.4, NM_001304569.1 |
| Undifferentiated iPSC | *POU5F1* | Hs00999632_g1 | NM_002701.6, NM_203289.5 NM_001173531.2, NM_001285986.1 NM_001285987.1 |
| | *Nanog* | Hs04260366_g1 | NM_024865.4, NM_001297698.1 |

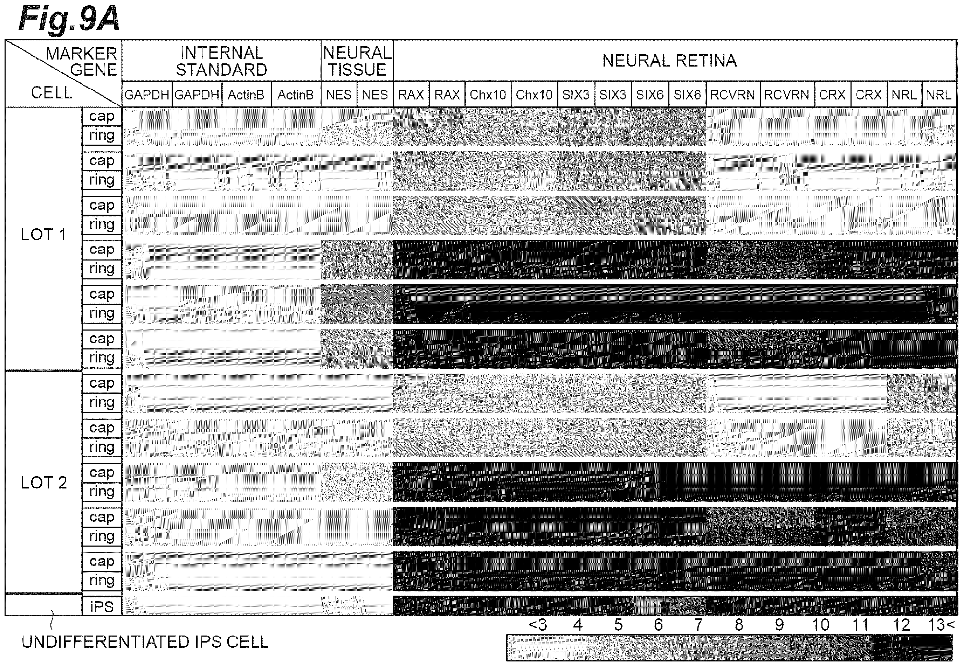

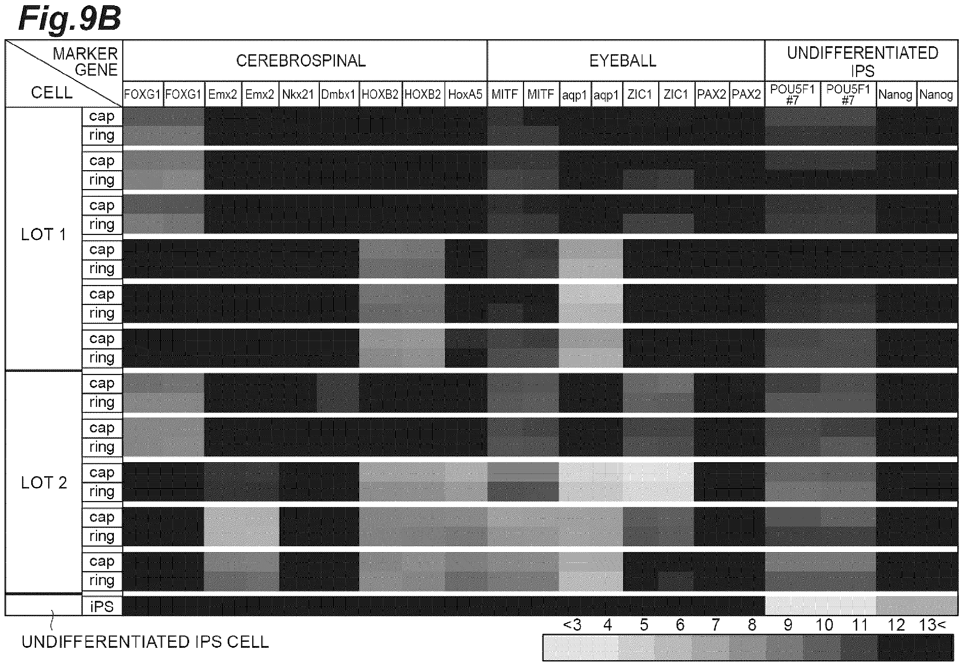

The results are shown in a heatmap in Figures 9A and 9B. The gene expression levels were evaluated from ΔCt values calculated from the difference between the Ct value of the target gene and the Ct value of the GAPDH gene used as an internal standard. A lower ΔCt value represents a higher gene expression level, and a higher ΔCt value represents a lower gene expression level. The gray color corresponds to a high gene expression level, and the black color corresponds to a low gene expression level (a lighter color corresponds to a higher level of gene expression). As a result of examining gene expression in each Cap and ring, the neural retina marker gene group was expressed in the Cap and the Ring isolated from the neural retina in both the lot 1 and the lot 2. On the other hand, as a result of examining gene expression in the caps and the rings isolated from the by-products, the expression level of the neural retina marker gene group was low and the expression levels of the by-product marker gene groups were high, on the contrary to the neural retina, in both the lots. Moreover, as a result of comparing gene expression between the Cap and the Ring isolated from the same cell aggregate, it was found that the expression level of the neural retina marker gene or the expression level of the by-product marker gene was equivalent between the Cap and the Ring isolated from any of the neural retina and the by-products.

From these results, it was able to be demonstrated that, provided that the Ring is the neural retina, the Cap is also the neural retina. It was also able to be demonstrated that gene expression is equivalent between the Cap and the ring.

<Reference Example 6 Transplantation results of graft>

The gene expression of a Ring was analyzed by the method described in Reference Example 5, and then, the corresponding Cap (neural retina sheet) was used as a retina sheet. The retina sheet was dipped in "Viscoat diluted 4-fold (Viscoat:Opeguard = 1:3)" as a vehicle for transplantation to prepare a composition for transplantation. This was transplanted to a retinal degenerative nude rat to evaluate images of post-transplant engraftment.

First, a cell aggregate was prepared from human iPS cells (DSP-SQ strain) in accordance with the method described in Reference Example 1. Thereafter, a Cap and a Ring were isolated by the method described in Reference Example 5 from the cell aggregate on Day 75 or later from initiation of suspension culture. The isolated Cap was preserved using a commercially available preservation solution while the gene analysis of the Ring was carried out. The isolated Ring was subjected to gene expression analysis by real-time PCR using Biomark HD (manufactured by Fluidigm Corp.) in accordance with the method described in Reference Example 5. From the results of the gene expression analysis, a Ring that expressed the neural retina marker gene and did not express the by-product marker gene was selected, and a Cap corresponding to this Ring was selected as a graft (Retina sheet to be transplanted). The graft was washed with a buffer (manufactured by Thermo Fisher Scientific Inc.) and then dipped in a vehicle for transplantation to prepare a composition for transplantation. This was subretinally transplanted to a retinal degenerative nude rat (photoreceptor cell degenerative model, SD-Foxn1 Tg(S334ter)3LavRrrc nude rat) using the injector described in the known literature (Shirai et al., PNAS 113, E81-E90).

The eye tissue obtained on Days 230 to 240 from initiation of the suspension culture was fixed with paraformaldehyde (PFA fixed) and subjected to sucrose replacement. The eye tissue fixed was frozen and sectioned by use of a cryostat. These frozen sections were subjected to immunostaining to stain a human nucleus (anti-HuNu antibody, Merck Millipore, mouse, or anti-HNA antibody), a photoreceptor cell marker recoverin (anti-recoverin antibody, Proteintech Group, rabbit) and a bipolar cell marker PKCα (anti-PKCa antibody, R&D systems, Inc., goat).

Results of summarizing quality evaluation results by the gene expression analysis of rings and transplantation results of grafts are shown in Table 13. A method for calculating ΔCt values employed the method described in Reference Example 5. The gene expression analysis of rings passed them on the quality evaluation test (ring-PCR test) when the ΔCt value of a neural retina marker gene, recoverin, was 10 or less and each of the ΔCt values of by-product marker genes *FOXG1*, *HOXB2*, *ZIC1* and *OCT3/4* was 5 or more. As for the transplantation results, engraftment was evaluated as being favorable when human nucleus-positive and recoverin-positive photoreceptor cells were able to be subretinally detected. It was determined that swelling was not detected unless the transplantation site was much thicker than the proper size of engraftment.

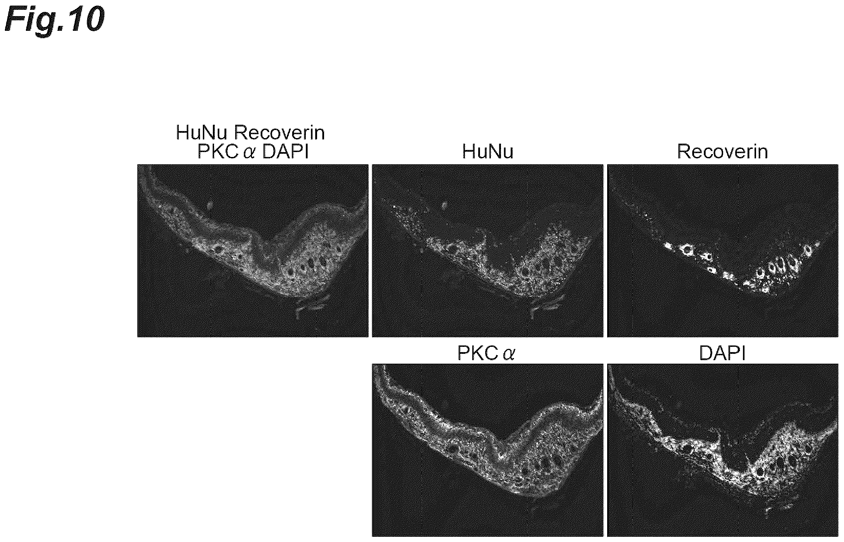

A typical image of engraftment is shown in Figure 10. As a result of evaluating images of post-transplant engraftment as to 14 eyes passed on the quality evaluation test before transplantation, recoverin-positive photoreceptor cells were detected in all the 14 eyes. Thus, favorable engraftment was found. Since these cells were HuNu-positive, it was found that the recoverin-positive photoreceptor cells were derived from the transplanted caps. Swelling was not detected in any of the 14 eyes.

From the results described above, a graft that was subretinally favorably engrafted, i.e., in which photoreceptor cells were engrafted without causing swelling, was able to be selected by examining the expression levels of the neural retina and by-products marker genes before transplantation by the gene expression analysis of the ring.

The safety of "Viscoat diluted 4-fold (Viscoat:Opeguard = 1:3)", a vehicle for transplantation, in subretinal administration was able to be demonstrated. Specifically, although hyaluronic acid and chondroitin sulfate are contained in Viscoat diluted 4-fold, the safety of hyaluronic acid and chondroitin sulfate at the concentrations used in this test in subretinal administration was able to be demonstrated.

[Table 13]

| Graft | | Transplantation results | |
|---|---|---|---|
| Strain | Quality evaluation test | Engraftment of photoreceptor cell | Swelling |
| DSP-SQ | Pass on ring-PCR test | All cases of 14 eyes Favorable engraftment | All cases of 14 eyes Not observed |

<Reference Example 7 Expression of marker in Cap and Ring>

In Reference Example 5, it was demonstrated by PCR that the composition of cells was equivalent between a Cap and a Ring. Accordingly, whether the composition of cells and a tissue structure were equivalent between the Cap and the Ring was then examined by immunostaining.

First, human iPS cells (DSP-SQ strain) were differentiated into retinas by the production method described in Reference Example 1. Thereafter, a Cap and a Ring of the neural retina were isolated by the method described in Reference Example 5 from a cell aggregate on Day 120 from initiation of suspension culture. Thereafter, the Cap and the Ring were washed, then fixed with 4% paraformaldehyde (PFA fixed) and subjected to sucrose replacement. The Cap and the Ring fixed were frozen and sectioned by use of a cryostat. These frozen sections were subjected to immunostaining to stain a nucleus with DAPI, a photoreceptor progenitor cell marker Crx (anti-Crx antibody, Takara Bio Inc., rabbit), a neural retina marker Chx10 (anti-Chx10 antibody, Exalpha Biologicals, sheep), a rod photoreceptor progenitor cell marker NRL (anti-NRL antibody, Bio-Techne Corp., goat), a telencephalon marker FOXG1 (anti-FOXG1 antibody, Takara Bio Inc., rabbit), an optic stalk marker PAX2 (anti-PAX2 antibody, Thermo Fisher Scientific Inc., rabbit), and an undifferentiated pluripotent stem cell marker NANOG (anti-NANOG antibody, Merck, mouse).

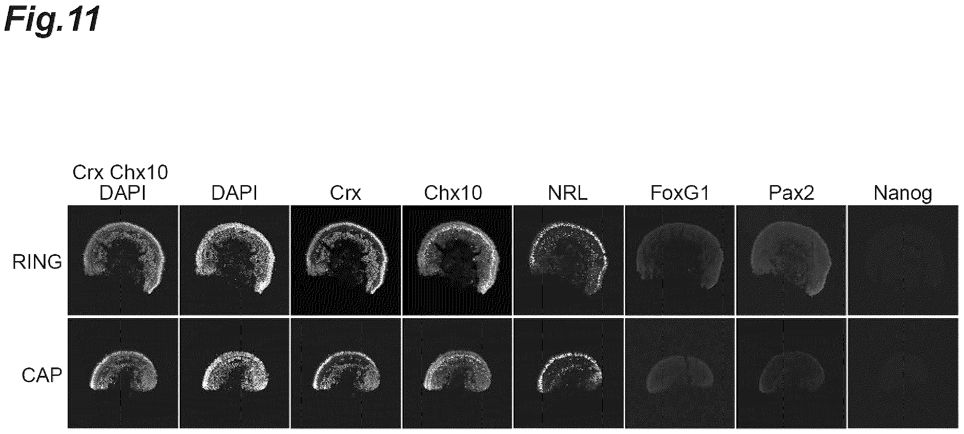

The results of the immunostaining are shown in Figure 11. As for the Cap and the Ring dissected from the same cell aggregate, the results of immunostaining the Ring are shown in the upper boxes, and the results of immunostaining the Cap are shown in the lower boxes. From these results, it was found that Crx-positive photoreceptor progenitor cells, Chx10-positive neural retina, and NRL-positive rod photoreceptor progenitor cells were expressed in a continuous layer pattern in both the Cap and the ring. FOXG1-positive telencephalon, PAX2-positive optic stalk, or NANOG-positive pluripotent stem cells were not detected in any of the Cap and the Ring. When Crx-, Chx10- and NRL-stained images were compared, it was confirmed that these neural retina markers exhibited almost equivalent distribution between the Cap and the ring.

<Reference Example 8 Ratios of photoreceptor progenitor cell and neural retinal progenitor cell constituting transplant neural retina sheet>

The ratios of photoreceptor progenitor cells and neural retinal progenitor cells to cells constituting a neural retina sheet prepared from a cell aggregate differentiated from pluripotent stem cells were analyzed and quantified by an immunostaining method, immunohistochemistry (IHC).

Human iPS cells (DSP-SQ strain) were differentiated into retinas by the production method described in Reference Example 1. Thereafter, caps and rings were isolated by the method described in Reference Example 3 from the cell aggregates on Days 84, 92 and 93 from initiation of suspension culture. The gene expression analysis of the isolated rings was carried out by the method described in Reference Example 6. A Ring that expressed the neural retina marker gene and did not express the by-product marker gene was selected by the method described in Reference Example 6, and a Cap corresponding to this Ring was used as a transplant neural retina sheet. In this way, one transplant neural retina sheet from the cell aggregate on Day 84 from initiation of suspension culture, two transplant neural retina sheets from the cell aggregate on Day 92 from initiation of suspension culture, and one transplant neural retina sheet from the cell aggregate on Day 93 from initiation of suspension culture, were prepared. In other words, a total of four transplant neural retina sheets were prepared.

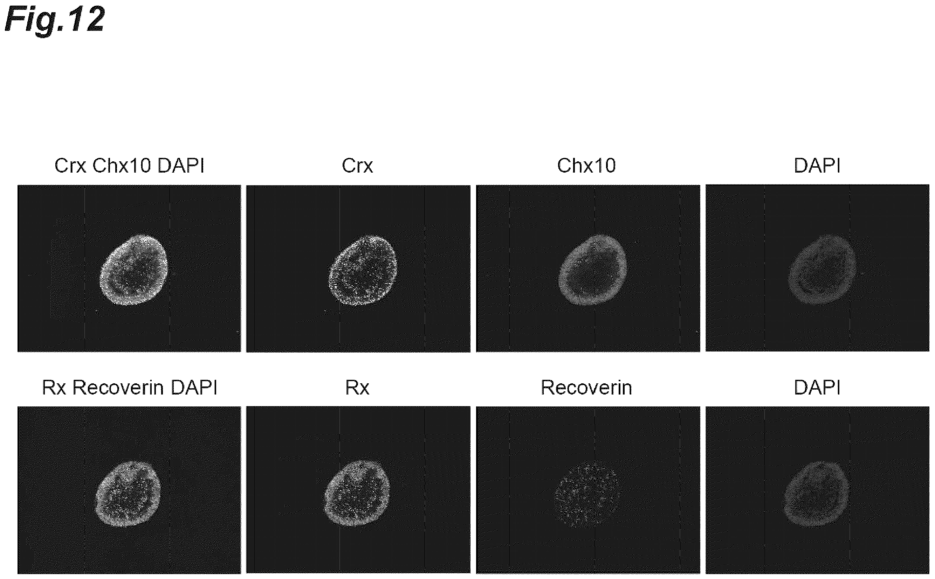

The obtained transplant neural retina sheets were cultured for 7 days in B medium for analysis. The cultured transplant neural retina sheets were fixed with 4% paraformaldehyde, frozen and sectioned. The frozen sections were subjected to immunostaining to stain a neural retinal progenitor cell marker, Chx10 (anti-Chx10 antibody, Exalpha Biologicals, sheep) and a photoreceptor progenitor cell marker Crx (anti-Crx antibody, Takara Bio Inc., rabbit). Other frozen sections were subjected to immunostaining to stain a neural retina marker Rx (anti-Rx antibody, Takara Bio Inc., guinea pig) and a photoreceptor cell marker recoverin (anti-recoverin antibody, Proteintech Group, rabbit). The nuclei of the cells were stained with DAPI. These sections stained were observed using a fluorescence microscope (manufactured by Keyence Corp.) to obtain immunostained images. One example thereof (D3) is shown in Figure 12.

The immunostained images were analyzed in ImageJ (version 1.52a, manufactured by National Institutes of Health (NIH)) to analyze the number of DAPI-positive cells, the number of DAPI-positive and Chx10-positive cells, and the number of DAPI-positive and Crx-positive cells as to each of the four transplant neural retina sheets. The immunostained images were analyzed in the same manner to analyze the number of DAPI-positive cells and the number of DAPI-positive and Rx-positive cells. From these numerical values, the ratio of Chx10-positive cells, the ratio of Crx-positive cells, and the ratio of Rx-positive cells were calculated. The obtained results are shown in Table 14.

[Table 14]

| Cap ID No | The number of days from initiation of suspension culture (d) | Ratio of positive cell (%) | | |
|---|---|---|---|---|
| | | Chx10 | Crx | Rx |
| B3 | 84+7 | 44.6 | 29.7 | 39.5 |
| D2 | 92+7 | 38.9 | 41.5 | 44.3 |
| D3 | 92+7 | 22.7 | 39.0 | 53.5 |
| G3 | 93+7 | 36.6 | 55.5 | 53.5 |

From the results described above, it was found that the ratio of Chx10-positive cells contained in the transplant neural retina sheet dissected from the cell aggregate was on the order of 23 to 45%, the ratio of Crx-positive cells was on the order of 30 to 56%, and the ratio of Rx-positive cells was on the order of 40 to 54%.

In short, it was suggested that about 34% (about 23 to 45%) Chx10-positive neural retinal progenitor cells, about 40% (30 to 56%) Crx-positive photoreceptor progenitor cells, and about 47% (40 to 54%) Rx-positive cells were contained in the transplant neural retina sheet.

<Reference Example 9 Ratios of photoreceptor progenitor cell and neural retinal progenitor cell constituting transplant neural retina sheet>

The composition of cells constituting transplant neural retina sheets prepared from cell aggregates differentiated from various pluripotent stem cells was examined by an immunostaining method, flow cytometry (also referred to as FACS).

Human iPS cells (QHJI01s04 strain) were differentiated into retinas by the production method described in Reference Example 1. Thereafter, a Cap and a Ring were isolated by the method described in Reference Example 3 from the cell aggregate on Day 88 from initiation of suspension culture. The Cap was used as a transplant neural retina sheet. The transplant neural retina sheet was preserved at a low temperature of 17°C for 2 days. Five transplant neural retina sheets obtained were combined as one sample, washed with PBS, enzymatically treated at 37°C for 30 minutes using a neuronal cell dispersion solution (manufactured by FUJIFILM Wako Pure Chemical Corp, containing papain), and dispersed into single cells by pipetting to obtain a single-cell suspension. The obtained single-cell suspension was fixed using a fixative solution (manufactured by Becton, Dickinson and Company, CytoFix) to obtain a sample for FACS. The sample for FACS was subjected to blocking and permeation treatment (cell membrane perforation) using Perm/Wash buffer (manufactured by Becton, Dickinson and Company) containing serum. Then, immunostaining was performed with the following antibodies fluorescently labeled: anti-Chx10 antibody (manufactured by Santa Cruz Biotechnology, Inc.), anti-Pax6 antibody (manufactured by Becton, Dickinson and Company), and anti-Crx antibody (manufactured by Santa Cruz Biotechnology, Inc.). Then, analysis was conducted by flow cytometry using an analyzer (manufactured by Becton, Dickinson and Company).

As a result, it was found that a Chx10-positive and Pax6-positive fraction (neural retinal progenitor cell fraction) occupied 11.5%, a Chx10-positive and Pax6-negative fraction (progenitor cell fraction biased toward bipolar cells) occupied 23.4%, a Chx10-negative and Pax6-positive fraction (ganglion cell and amacrine cell fraction) occupied 10.7%, and a Crx-positive cell fraction (photoreceptor progenitor cell fraction) occupied 17.4%.

Further, human iPS cells (DSP-SQ strain) were differentiated into retinas by the production method described in Reference Example 1. Thereafter, 11 cell aggregates on Day 88 from initiation of suspension culture were prepared, and 11 each of caps and rings were isolated by the method described in Reference Example 3 from each of the cell aggregates. The 11 caps were combined as one sample. Likewise, the 11 rings were combined as one sample. The Cap sample and the Ring sample were each washed with PBS and enzymatically treated at 37°C for 30 minutes using a neuronal cell dispersion solution (manufactured by FUJIFILM Wako Pure Chemical Corp, containing papain) to obtain respective single-cell suspensions of the Cap and the ring. The obtained respective single-cell suspensions of the Cap and the Ring were fixed using a fixative solution (manufactured by Becton, Dickinson and Company, CytoFix) to obtain samples for FACS. The samples for FACS were subjected to blocking and perforation using Perm/Wash buffer (manufactured by Becton, Dickinson and Company) containing serum and subjected to immunostaining with the following antibodies fluorescently labeled: anti-Chx10 antibody (manufactured by Santa Cruz Biotechnology, Inc.), anti-Crx antibody (manufactured by Santa Cruz Biotechnology, Inc.), and anti-SSEA-4 antibody. Isotype controls were used as negative controls for immunostaining. Then, analysis was conducted by flow cytometry using an analyzer (manufactured by Becton, Dickinson and Company). The ratio of Chx10-positive cells, the ratio of Crx-positive cells, and the ratio of SSEA-4-positive cells were calculated from delta from their respective isotype controls. The results are described in Table 15.

[Table 15]

| Sample | The number of days from initiation of suspension culture (d) | Ratio of positive cell (%) | | |
|---|---|---|---|---|
| | | Chx10 | Crx | SSEA-4 |
| Cap | 88 | 29.4 | 15.8 | <1 |
| Ring | 88 | 28.1 | 21.7 | <1 |

In the Cap sample, the ratio of neural retinal progenitor cell marker Chx10-positive cells was 29.4%, the ratio of photoreceptor progenitor cell marker Crx-positive cells was 15.8%, and the ratio of pluripotent stem cell marker SSEA-4-positive cells (non-target cells) was less than 1%. In the Ring sample, the ratio of neural retinal progenitor cell marker Chx10-positive cells was 28.1%, the ratio of photoreceptor progenitor cell marker Crx-positive cells was 21.7%, and the ratio of pluripotent stem cell marker SSEA-4-positive cells (non-target cells) was less than 1%.

From these results, first, it was found that the Cap sample and the Ring sample were neural retinas containing Chx10-positive cells and Crx-positive cells and did not substantially contain undifferentiated iPS cells. Furthermore, it was able to be demonstrated that the ratios of Chx10-positive cells and Crx-positive cells contained in the Cap sample were equivalent to the ratios of Chx10-positive cells and Crx-positive cells contained in the Ring sample. Moreover, it was able to be demonstrated that, provided that the Ring sample was the neural retina, the Cap was also the neural retina.

Besides, it was found that, in the case of using such a Cap or a Ring (preferably cap) as a transplant neural retina sheet, the Chx10-positive cell fraction (neural retinal progenitor cell fraction) contained in this transplant neural retina sheet occupied about 30% (about 20 to 40%), and the Crx-positive cell fraction (photoreceptor progenitor cell fraction) occupied about 17% (about 10 to 30%).

**Claims**

1.  A complex comprising:

    two or more cell aggregates each containing a neural retina derived from a pluripotent stem cell; and
    a matrix,
    the two or more cell aggregates being arranged in the matrix, wherein

    the neural retina is a transplant neural retina sheet having the following features (1) to (10):

    (1) being derived from a pluripotent stem cell,
    (2) having a three-dimensional structure,
    (3) comprising a neural retinal layer having a plurality of layer structures including a photoreceptor layer and an inner layer,
    (4) the photoreceptor layer comprising one or more cells selected from the group consisting of a photoreceptor progenitor cell and a photoreceptor cell,
    (5) the inner layer comprising one or more cells selected from the group consisting of a retinal progenitor cell, a ganglion cell, an amacrine cell and a bipolar cell,
    (6) the surface of the neural retinal layer having an apical surface,
    (7) the inner layer being present inside the photoreceptor layer present along the apical surface,
    (8) the area of the neural retinal layer being 50% or more with respect to the total area of the surface of the transplant neural retina sheet,
    (9) the area of a continuous epithelium structure being 80% or more with respect to the total area of the apical surface of the neural retinal layer, and
    (10) the expression of neural retina-related cell-related gene being found and the expression of non-neural retina-related cell-related gene being not found in the transplant neural retina sheet, wherein the non-neural retina-related cell-related gene comprising one or more genes selected from the group consisting of brain and spinal cord tissue marker gene and eyeball-related tissue marker gene.

2.  The complex according to claim 1, wherein the cell aggregates have a major axis of 600 $\mu$m to 2500 $\mu$m, and the two or more cell aggregates are arranged in a row in the matrix.

3. The complex according to claim 1 or 2, wherein the matrix is fibrin gel or gelatin.

4. A method for manufacturing a complex in which two or more neural retina-containing cell aggregates are arranged in a matrix, comprising:

   (1) a first step of preparing two or more cell aggregates of neural retinas from pluripotent stem cells; and
   (2) a second step of contacting the two or more cell aggregates in a predetermined arrangement with the matrix or a precursor of the matrix, followed by the gelation of the matrix.

5. The manufacturing method according to claim 4, comprising, in the second step, arranging 2 to 20 cell aggregates in a row.

6. The manufacturing method according to claim 4 or 5, wherein the matrix is fibrin gel, and in the second step, the gelation is performed by arranging the two or more cell aggregates in a row, and contacting the two or more cell aggregates arranged with any one of a fibrinogen solution and a thrombin solution and further with the other solution of the fibrinogen solution and the thrombin solution so that the fibrinogen and the thrombin are reacted.

7. The manufacturing method according to claim 4 or 5, wherein the matrix is gelatin.

*Fig.1*

Bright field   Bright field   Rx::Venus        Bright field
                    +
               Rx::Venus

Bright field

Bright field
Rx::Venus

Rx::Venus

Aspiration

Discharge

EP 4 206 321 A1

Fig.2

Aspiration

Discharge

Bright field

Bright field
Rx::Venus

Rx::Venus

Bright field

Bright field  Bright field  Rx::Venus
+
Rx::Venus

# Fig.3

Crx Chx10 DAPI     Chx10     Crx

LOW MAGNIFICATION

HIGH MAGNIFICATION

PHOTORECEPTOR LAYER

INNER LAYER

Fig.4

# Fig.5

FIRST EPITHELIAL TISSUE

SECOND EPITHELIAL
TISSUE

ISOLATION

Cap

Ring

Root

GRAFT (CAP)

SAMPLE FOR QUALITY
EVALUATION (RING)

DIAGRAM VIEWED
FROM SIDE

DIAGRAM VIEWED
FROM ABOVE

cap

ring

cap

ring

EP 4 206 321 A1

*Fig.6*

(1)

(2)

(3)

(4)

(5)

Cap

Ring

GRAFT (CAP)

SAMPLE FOR QUALITY
EVALUATION (RING)

# Fig.7

GRAFT (CAP)

LARGE

MEDIUM

SMALL

MAJOR AXIS
1200 µm

MINOR AXIS
600 µm

HEIGHT
400 µm

| SAMPLE | MAJOR AXIS µm | MINOR AXIS µm | HEIGHT µm |
| --- | --- | --- | --- |
| CAP LARGE | 1606 | 1051 | 640 |
| CAP MEDIUM | 1170 | 592 | 440 |
| CAP SMALL | 1132 | 462 | 258 |
| AVERAGE | 1184 | 646 | 421 |

## Fig.8

EP 4 206 321 A1

Fig.9A

UNDIFFERENTIATED IPS CELL

# Fig.9B

EP 4 206 321 A1

*Fig.10*

Fig.11

Fig.12

## INTERNATIONAL SEARCH REPORT

| International application No. |
| --- |
| **PCT/JP2021/033387** |

**A.    CLASSIFICATION OF SUBJECT MATTER**

*C12N 5/0793*(2010.01)i; *C12N 5/0797*(2010.01)i; *A61L 27/22*(2006.01)i; *A61L 27/36*(2006.01)i; *A61L 27/38*(2006.01)i; *A61L 27/44*(2006.01)i; *A61L 27/52*(2006.01)i; *A61L 27/58*(2006.01)i

FI:    C12N5/0793; A61L27/38 100; A61L27/44; A61L27/58; A61L27/36 400; A61L27/36 300; A61L27/52; A61L27/22; A61L27/38 200; A61L27/36 130; C12N5/0797

According to International Patent Classification (IPC) or to both national classification and IPC

**B.    FIELDS SEARCHED**

Minimum documentation searched (classification system followed by classification symbols)

C12N5/0793; C12N5/0797; A61L27/22; A61L27/36; A61L27/38; A61L27/44; A61L27/52; A61L27/58

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C.    DOCUMENTS CONSIDERED TO BE RELEVANT**

| Category* | Citation of document, with indication, where appropriate, of the relevant passages | Relevant to claim No. |
| --- | --- | --- |
| X | WO 2019/028088 A1 (BIOTIME, INC.) 07 February 2019 (2019-02-07) claims, abstract, page 4, lines 1-9, page 19, lines 6-19, page 21, lines 24-30 | 4, 5 |
| Y | claims, abstract, page 4, lines 1-9, page 19, lines 6-19, page 21, lines 24-30 | 1-3, 6, 7 |
| Y | AHMED, Tamer A. E. et al. Autologous fibrin glue as an encapsulating scaffold for delivery of retinal progenitor cells. Frontiers in Bioengineering and Biotechnology, 2015, vol. 2, Article 85, pp. 1-11 abstract, method and materials | 3, 6 |
| A | abstract, method and materials | 1, 2, 4, 5, 7 |
| Y | M'BAREK, Karim Ben et al. Human ESC-derived retinal epithelial cell sheets potentiate rescue of photoreceptor cell loss in rats with retinal degeneration. SCIENCE TRANSLATIONAL MEDICINE, 2017, vol. 9, eaai7471, pp. 1-12 abstract, fig. 3 | 3, 7 |
| A | abstract, fig. 3 | 1, 2, 4-6 |

☑ Further documents are listed in the continuation of Box C.          ☑ See patent family annex.

| | | |
| --- | --- | --- |
| * | Special categories of cited documents: | "T"  later document published after the international filing date or priority date and not in conflict with the application but cited to understand the principle or theory underlying the invention |
| "A" | document defining the general state of the art which is not considered to be of particular relevance | |
| "E" | earlier application or patent but published on or after the international filing date | "X"  document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y"  document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&"  document member of the same patent family |
| "P" | document published prior to the international filing date but later than the priority date claimed | |

| Date of the actual completion of the international search | Date of mailing of the international search report |
| --- | --- |
| **19 November 2021** | **30 November 2021** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**

International application No.

**PCT/JP2021/033387**

C.    DOCUMENTS CONSIDERED TO BE RELEVANT

| Category* | Citation of document, with indication, where appropriate, of the relevant passages | Relevant to claim No. |
|---|---|---|
| Y | KUWAHARA, Atsushi et al. Generation of a ciliary margin-like stem cell niche from self-organizing human retinal tissue. Nat. Commun., 2015, vol. 6, no. 6286, pp. 1-15<br>page 2, right column, line 23 to page 4, right column, line 22, page 4, right column, line 23 to line 15 from the bottom, page 4, right column, lines 14-1 from the bottom, page 12, left column, line 23 to page 13, line 1 from the bottom, fig. 1-3 | 1-3 |
| A | page 2, right column, line 23 to page 4, right column, line 22, page 4, right column, line 23 to line 15 from the bottom, page 4, right column, lines 14-1 from the bottom, page 12, left column, line 23 to page 13, line 1 from the bottom, fig. 1-3 | 4-7 |

Form PCT/ISA/210 (second sheet) (January 2015)

**INTERNATIONAL SEARCH REPORT**
Information on patent family members

International application No.

**PCT/JP2021/033387**

| Patent document cited in search report | | | Publication date (day/month/year) | Patent family member(s) | | | Publication date (day/month/year) |
|---|---|---|---|---|---|---|---|
| WO | 2019/028088 | A1 | 07 February 2019 | JP | 2020-530839 | A | |
| | | | | US | 2020/0368394 | A1 | |
| | | | | EP | 3661529 | A1 | |
| | | | | CN | 111511377 | A | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2015025967 A
- WO 2016063986 A
- JP 2016052271 A
- WO 9622362 A
- WO 02101057 A
- US 5843780 A
- US 6200806 B
- US 6280718 B
- WO 2011055855 A
- WO 2013077425 A
- WO 2016063985 A
- WO 2017183732 A
- WO 2019017492 A
- WO 2019054514 A
- WO 2019054515 A
- WO 9721993 A
- WO 2004066929 A
- WO 2004093799 A
- WO 2000039077 A
- WO 2001098256 A
- WO 2003018515 A
- WO 2003084915 A
- WO 2002094319 A
- WO 2003064369 A
- JP 2002053564 A
- JP 2002370978 A
- JP 2000256190 A
- WO 2007132475 A
- WO 2007009913 A
- WO 2003094845 A
- WO 2002051805 A
- WO 2010122980 A
- JP 2020011254 W

**Non-patent literature cited in the description**

- **ATSUSHI KUWAHARA et al.** Generation of a ciliary margin-like stem cell niche from self-organizing human retinal tissue. *Nature Communications,* 2015, vol. 6
- **TAYLOR et al.** *IOVS,* August 1989, vol. 30 (8
- **M' BAREK et al.** *Sci. Transl. Med.,* December 2017, vol. 9 (421
- **SEILER et al.** *Prog Retin Eye Res.,* November 2012, vol. 31 (6), 661-687
- **TAMER A.E. AHMED et al.** *Frontiersin Bioengineering and Biotechnology, Biomaterials,* February 2015, vol. 2, 1-11
- **YAMANAKA et al.** *Cell,* 2006, vol. 126 (4), 663-676
- *Cell,* 2007, vol. 131 (5), 861-872
- *Science,* 2007, vol. 318 (5858), 1917-1920
- *Nat. Biotechnol.,* 2008, vol. 26 (1), 101-106
- *Stem Cells,* 2013, vol. 31, 458-466
- *Science,* 2013, vol. 341, 651-654
- *PLoS One.,* 20 January 2010, vol. 5 (1), e8763
- *Stem Cells.,* August 2011, vol. 29 (8), 1206-18
- *Proc Natl Acad Sci USA.,* 10 June 2014, vol. 111 (23), 8518-23
- *Nat Commun.,* 10 June 2014, vol. 5, 4047
- **BRYCE T. MCLELLAND et al.** *IOVS,* May 2018, vol. 59 (6), 2586
- **NAKANO, T. et al.** *Cell Stem Cell,* 2012, vol. 10, 771-785
- **KAWAHARA A. et al.** *Nature Communications,* 2015, vol. 6, 6286
- **KUWAHARA A ; YAMASAKI S et al.** *Sci Rep.,* 12 December 2019, vol. 9 (1), 18936
- **LAMBA, D.A. ; GUST, J. ; REH, T.A.** *Cell Stem Cell,* 2009, vol. 4, 73-79
- **ZHU, J. ; CIFUENTES, H. ; REYNOLDS, J. ; LAMBA, D.A.** *Cell Stem Cell,* 2017, vol. 20, 374-384.e375
- **MEYER, J.S. et al.** *Stem Cells,* 2011, vol. 29, 1206-1218
- **ZHONG, X. et al.** *Nat Commun,* 2014, vol. 5
- **BOUCHERIE, C. et al.** *Stem Cells,* 2013, vol. 31, 408-414
- **GONZALEZ-CORDERO, A. et al.** *Nat Biotechnol,* 2013, vol. 31, 741-747
- **MELLOUGH, C.B. et al.** *Stem Cells,* 2015, vol. 33, 2416-2430
- **HALLAM, D. et al.** *Stem Cells,* 2018
- **REICHMAN, S. et al.** *PNAS,* 2014, vol. 111, 8518-8523
- **GAGLIARDI, G. et al.** *Stem Cell Reports,* 2018, vol. 11, 665-680
- **TUCKER, B.A. et al.** *Stem Cells Transl Med,* 2013, vol. 2, 16-24
- **WAHLIN, K.J. et al.** *Sci Rep,* 2017, vol. 7, 766
- **DISTEFANO, T. et al.** *Stem Cell Reports,* 2018, vol. 10, 300-313

- *Nature Methods,* 2011, vol. 8, 424-429
- *Proc. Natl. Acad. Sci. USA.,* 09 September 2008, vol. 105 (36), 13409-14
- *Nat Biotechnol,* 2010, vol. 28, 611-615
- *Nat Commun,* 2012, vol. 3, 1236
- *Nat Biotechnol,* 2001, vol. 19, 971-974
- *Scientific Reports,* 2014, vol. 4, 3594
- **SHIRAI et al.** *PNAS,* vol. 113, E81-E90